# EUROPEAN PATENT APPLICATION

(11) **EP 4 406 971 A1**
(43) Date of publication of application: **31.07.2024**
(21) Application number: 22872081.9
(22) Date of filing: 23.09.2022
(51) Int. Cl.: C07K 16/28, C12N 15/13, C12N 15/63, A61K 39/395, A61P 35/00, A61P 29/00

(54) **ANTI-CD40 ANTIBODY AND USE THEREOF**

(30) Priority: 24.09.2021 CN 202111123163
(71) Applicant: CHIA TAI TIANQING PHARMACEUTICAL GROUP CO., LTD., Lianyungang, Jiangsu 222062 (CN)
(72) Inventor: LU, Zhenzhen, Nanjing, Jiangsu 211100 (CN); WANG, Yujie, Nanjing, Jiangsu 211100 (CN); ZHANG, Zhengping, Nanjing, Jiangsu 211100 (CN); XU, Hongjiang, Nanjing, Jiangsu 211100 (CN); ZHAO, Wei, Nanjing, Jiangsu 211100 (CN)
(74) Representative: WSL Patentanwälte Partnerschaft mbB
(86) International application number: PCT/CN2022/120689
(87) International publication number: WO 2023/046037

(57) **Abstract**

Provided are an anti-CD40 antibody and a use thereof, such as a mouse, human, chimeric, or humanized antibody specifically binding to CD40, and an antigen-binding fragment thereof, and further provided are a nucleic acid molecule encoding the antibody or the antigen-binding fragment thereof, and an expression vector and a host cell for expressing the antibody or the antigen-binding fragment thereof. Further provided are a preparation method and a use method for the antibody or the antigen-binding fragment thereof, comprising treating and preventing immune diseases.

## Description

### TECHNICAL FIELD

The present disclosure relates to the field of biotechnology, and particularly, to an antibody and an antigen-binding fragment thereof specifically binding to CD40, and a method for using the antibody and the antigen-binding fragment thereof.

### BACKGROUND

CD40 is a type I transmembrane glycoprotein, and a member of the tumor necrosis factor receptor (TNFR) superfamily. CD40 is constitutively expressed in antigen-presenting cells (including dendritic cells, B cells, and macrophages), and to a lesser extent in nonhematopoietic cells such as epithelial cells, endothelial cells, smooth muscle cells, fibroblasts, keratinocytes, and the like. In addition, CD40 is also expressed in a variety of tumor cells, such as B lymphoma cells. CD40L (CD 154), the major ligand for CD40, is a type II transmembrane glycoprotein, and is expressed mainly in activated CD4⁺ T lymphocytes, activated B cells, memory T cells, activated NK cells, and activated platelets.

The CD40/CD40L signaling pathway is involved in humoral immune responses and cellular immune responses of the body, and plays a key regulatory role in T cell-dependent antibody immune responses such as the activation, proliferation and differentiation of B cells, production of antibodies, class switching of antibodies, and the like, and inflammatory responses. Studies have shown that tissues and organs of patients with an autoimmune disease are infiltrated with a large number of T cells and B cells targeting autoantigens, which is manifested by the persistent overexpression of CD40 and/or CD40L. IHC staining results of patients with primary Sjogren's syndrome show that CD40 is enriched in salivary gland epithelial cells accompanied by increased autoantibodies in serum, such as anti-SSA antibodies. In addition, the key role of the CD40/CD40L signaling pathway in inflammatory bowel diseases has been demonstrated in animal models such as mice.

Therefore, an antibody that specifically binds to CD40 and inhibits the CD40/CD40L signaling pathway has potential clinical value for the treatment of immune diseases such as inflammatory diseases and autoimmune diseases. For example, iscalimab (CFZ533), an anti-CD40 antibody developed by Novartis, is currently in clinical trials for the treatment of CD40-mediated immune diseases. The results show that when iscalimab was administered intravenously at a dose of 10 mg/kg to patients with primary Sjogren's syndrome (with positive autoantibodies in serum), 62% (13/21) patients had an ESSDAI disease activity score of less than 5, indicating a therapeutic effect significantly better than that of the placebo group (36%, 4/11). The current large number of patients with immune diseases worldwide creates an urgent need for the development of more anti-CD40 antibodies with better pharmaceutical properties.

### SUMMARY

In one aspect, the present disclosure provides an isolated antibody or an antigen-binding fragment thereof that binds to CD40. In some embodiments, the antibody or the antigen-binding fragment thereof is a murine antibody, a chimeric antibody, a humanized antibody, or a human antibody. In some embodiments, the antibody or the antigen-binding fragment thereof is a monoclonal antibody, a monospecific antibody, a bispecific antibody, a trispecific antibody, a multispecific antibody, an Fab fragment, an F(ab')₂ fragment, an Fd fragment, an Fv fragment, a dAb, an isolated CDR, a single-chain Fv molecule, a recombinant polypeptide, a fusion protein, a bispecific molecule, or a combination thereof. In some embodiments, the antibody or the antigen-binding fragment thereof of the present disclosure binds to human CD40. In some other embodiments, the antibody or the antigen-binding fragment thereof of the present disclosure binds to CD40 (e.g., human CD40 and cynomolgous CD40). In some embodiments, the antibody or the antigen-binding fragment thereof of the present disclosure blocks the interaction between CD40 and CD40L. In some embodiments, the antibody or the antigen-binding fragment thereof of the present disclosure inhibits the activity of CD40.

In some embodiments, the anti-CD40 antibody or the antigen-binding fragment thereof provided in the present disclosure comprises a heavy chain CDR1 (HCDR1), a heavy chain CDR2 (HCDR2), and a heavy chain CDR3 (HCDR3), wherein (1) the heavy chain CDR1 comprises the heavy chain CDR1 amino acid sequence in SEQ ID NO: 9, the heavy chain CDR2 comprises the heavy chain CDR2 amino acid sequence in SEQ ID NO: 9, and the heavy chain CDR3 comprises the heavy chain CDR3 amino acid sequence in SEQ ID NO: 9; (2) the heavy chain CDR1 comprises the heavy chain CDR1 amino acid sequence in SEQ ID NO: 10, the heavy chain CDR2 comprises the heavy chain CDR2 amino acid sequence in SEQ ID NO: 10, and the heavy chain CDR3 comprises the heavy chain CDR3 amino acid sequence in SEQ ID NO: 10; (3) the heavy chain CDR1 comprises the heavy chain CDR1 amino acid sequence in SEQ ID NO: 11, the heavy chain CDR2 comprises the heavy chain CDR2 amino acid sequence in SEQ ID NO: 11, and the heavy chain CDR3 comprises the heavy chain CDR3 amino acid sequence in SEQ ID NO: 11; (4) the heavy chain CDR1 comprises the heavy chain CDR1 amino acid sequence in SEQ ID NO: 12, the heavy chain CDR2 comprises the heavy chain CDR2 amino acid sequence in SEQ ID NO: 12, and the heavy chain CDR3 comprises the heavy chain CDR3 amino acid sequence in SEQ ID NO: 12; (5) the heavy chain CDR1 comprises the heavy chain CDR1 amino acid sequence in SEQ ID NO: 13, the heavy chain CDR2 comprises the heavy chain CDR2 amino acid sequence in SEQ ID NO: 13, and the heavy chain CDR3 comprises the heavy chain CDR3 amino acid sequence in SEQ ID NO: 13; (6) the heavy chain CDR1 comprises the heavy chain CDR1 amino acid sequence in SEQ ID NO: 22, the heavy chain CDR2 comprises the heavy chain CDR2 amino acid sequence in SEQ ID NO: 22, and the heavy chain CDR3 comprises the heavy chain CDR3 amino acid sequence in SEQ ID NO: 22; (7) the heavy chain CDR1 comprises the heavy chain CDR1 amino acid sequence in SEQ ID NO: 30, the heavy chain CDR2 comprises the heavy chain CDR2 amino acid sequence in SEQ ID NO: 30, and the heavy chain CDR3 comprises the heavy chain CDR3 amino acid sequence in SEQ ID NO: 30; (8) the heavy chain CDR1 comprises the heavy chain CDR1 amino acid sequence in SEQ ID NO: 38, the heavy chain CDR2 comprises the heavy chain CDR2 amino acid sequence in SEQ ID NO: 38, and the heavy chain CDR3 comprises the heavy chain CDR3 amino acid sequence in SEQ ID NO: 38; (9) the heavy chain CDR1 comprises the heavy chain CDR1 amino acid sequence in SEQ ID NO: 44, the heavy chain CDR2 comprises the heavy chain CDR2 amino acid sequence in SEQ ID NO: 44, and the heavy chain CDR3 comprises the heavy chain CDR3 amino acid sequence in SEQ ID NO: 44; or (10) the heavy chain CDR1 comprises the heavy chain CDR1 amino acid sequence in SEQ ID NO: 52, the heavy chain CDR2 comprises the heavy chain CDR2 amino acid sequence in SEQ ID NO: 52, and the heavy chain CDR3 comprises the heavy chain CDR3 amino acid sequence in SEQ ID NO: 52.

In some embodiments, the anti-CD40 antibody or the antigen-binding fragment thereof provided in the present disclosure comprises a heavy chain CDR1, a heavy chain CDR2, and a heavy chain CDR3, wherein (1) the heavy chain CDR1, the heavy chain CDR2, and the heavy chain CDR3 comprise the amino acid sequences set forth in SEQ ID NOs: 1, 2, and 5, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 1, 2, and 5, respectively; (2) the heavy chain CDR1, the heavy chain CDR2, and the heavy chain CDR3 comprise the amino acid sequences set forth in SEQ ID NOs: 1, 3, and 5, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 1, 3, and 5, respectively; (3) the heavy chain CDR1, the heavy chain CDR2, and the heavy chain CDR3 comprise the amino acid sequences set forth in SEQ ID NOs: 1, 4, and 5, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 1, 4, and 5, respectively; (4) the heavy chain CDR1, the heavy chain CDR2, and the heavy chain CDR3 comprise the amino acid sequences set forth in SEQ ID NOs: 18, 19, and 20, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 18, 19, and 20, respectively; (5) the heavy chain CDR1, the heavy chain CDR2, and the heavy chain CDR3 comprise the amino acid sequences set forth in SEQ ID NOs: 24, 25, and 26, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 24, 25, and 26, respectively; (6) the heavy chain CDR1, the heavy chain CDR2, and the heavy chain CDR3 comprise the amino acid sequences set forth in SEQ ID NOs: 32, 33, and 34, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 32, 33, and 34, respectively; (7) the heavy chain CDR1, the heavy chain CDR2, and the heavy chain CDR3 comprise the amino acid sequences set forth in SEQ ID NOs: 40, 41, and 42, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 40, 41, and 42, respectively; or (8) the heavy chain CDR1, the heavy chain CDR2, and the heavy chain CDR3 comprise the amino acid sequences set forth in SEQ ID NOs: 46, 47, and 48, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 46, 47, and 48, respectively.

In one embodiment, the anti-CD40 antibody or the antigen-binding fragment thereof provided in the present disclosure comprises a light chain CDR1 (LCDR1), a light chain CDR2 (LCDR2), and a light chain CDR3 (LCDR3), wherein (1) the light chain CDR1 comprises the light chain CDR1 amino acid sequence in SEQ ID NO: 14, the light chain CDR2 comprises the light chain CDR2 amino acid sequence in SEQ ID NO: 14, and the light chain CDR3 comprises the light chain CDR3 amino acid sequence in SEQ ID NO: 14; (2) the light chain CDR1 comprises the light chain CDR1 amino acid sequence in SEQ ID NO: 15, the light chain CDR2 comprises the light chain CDR2 amino acid sequence in SEQ ID NO: 15, and the light chain CDR3 comprises the light chain CDR3 amino acid sequence in SEQ ID NO: 15; (3) the light chain CDR1 comprises the light chain CDR1 amino acid sequence in SEQ ID NO: 16, the light chain CDR2 comprises the light chain CDR2 amino acid sequence in SEQ ID NO: 16, and the light chain CDR3 comprises the light chain CDR3 amino acid sequence in SEQ ID NO: 16; (4) the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17; (5) the light chain CDR1 comprises the light chain CDR1 amino acid sequence in SEQ ID NO: 23, the light chain CDR2 comprises the light chain CDR2 amino acid sequence in SEQ ID NO: 23, and the light chain CDR3 comprises the light chain CDR3 amino acid sequence in SEQ ID NO: 23; (6) the light chain CDR1 comprises the light chain CDR1 amino acid sequence in SEQ ID NO: 31, the light chain CDR2 comprises the light chain CDR2 amino acid sequence in SEQ ID NO: 31, and the light chain CDR3 comprises the light chain CDR3 amino acid sequence in SEQ ID NO: 31; (7) the light chain CDR1 comprises the light chain CDR1 amino acid sequence in SEQ ID NO: 39, the light chain CDR2 comprises the light chain CDR2 amino acid sequence in SEQ ID NO: 39, and the light chain CDR3 comprises the light chain CDR3 amino acid sequence in SEQ ID NO: 39; (8) the light chain CDR1 comprises the light chain CDR1 amino acid sequence in SEQ ID NO: 45, the light chain CDR2 comprises the light chain CDR2 amino acid sequence in SEQ ID NO: 45, and the light chain CDR3 comprises the light chain CDR3 amino acid sequence in SEQ ID NO: 45; or (9) the light chain CDR1 comprises the light chain CDR1 amino acid sequence in SEQ ID NO: 53, the light chain CDR2 comprises the light chain CDR2 amino acid sequence in SEQ ID NO: 53, and the light chain CDR3 comprises the light chain CDR3 amino acid sequence in SEQ ID NO: 53.

In some embodiments, the anti-CD40 antibody or the antigen-binding fragment thereof provided in the present disclosure comprises a light chain CDR1, a light chain CDR2, and a light chain CDR3, wherein (1) the light chain CDR1, the light chain CDR2, and the light chain CDR3 comprise the amino acid sequences set forth in SEQ ID NOs: 6, 7, and 8, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 6, 7, and 8, respectively; (2) the light chain CDR1, the light chain CDR2, and the light chain CDR3 comprise the amino acid sequences set forth in SEQ ID NOs: 21, 7, and 8, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 21, 7, and 8, respectively; (3) the light chain CDR1, the light chain CDR2, and the light chain CDR3 comprise the amino acid sequences set forth in SEQ ID NOs: 27, 28, and 29, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 27, 28, and 29, respectively; (4) the light chain CDR1, the light chain CDR2, and the light chain CDR3 comprise the amino acid sequences set forth in SEQ ID NOs: 35, 36, and 37, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 35, 36, and 37, respectively; (5) the light chain CDR1, the light chain CDR2, and the light chain CDR3 comprise the amino acid sequences set forth in SEQ ID NOs: 43, 36, and 37, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 43, 36, and 37, respectively; or (6) the light chain CDR1, the light chain CDR2, and the light chain CDR3 comprise the amino acid sequences set forth in SEQ ID NOs: 49, 50, and 51, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 49, 50, and 51, respectively.

In some embodiments, the anti-CD40 antibody or the antigen-binding fragment thereof provided in the present disclosure comprises a heavy chain CDR1, a heavy chain CDR2, a heavy chain CDR3, a light chain CDR1, a light chain CDR2, and a light chain CDR3, wherein (1) the heavy chain CDR1 comprises the heavy chain CDR1 amino acid sequence in SEQ ID NO: 9, the heavy chain CDR2 comprises the heavy chain CDR2 amino acid sequence in SEQ ID NO: 9, the heavy chain CDR3 comprises the heavy chain CDR3 amino acid sequence in SEQ ID NO: 9, the light chain CDR1 comprises the light chain CDR1 amino acid sequence in SEQ ID NO: 14, the light chain CDR2 comprises the light chain CDR2 amino acid sequence in SEQ ID NO: 14, and the light chain CDR3 comprises the light chain CDR3 amino acid sequence in SEQ ID NO: 14; (2) the heavy chain CDR1 comprises the heavy chain CDR1 amino acid sequence in SEQ ID NO: 10, the heavy chain CDR2 comprises the heavy chain CDR2 amino acid sequence in SEQ ID NO: 10, the heavy chain CDR3 comprises the heavy chain CDR3 amino acid sequence in SEQ ID NO: 10, the light chain CDR1 comprises the light chain CDR1 amino acid sequence in SEQ ID NO: 15, the light chain CDR2 comprises the light chain CDR2 amino acid sequence in SEQ ID NO: 15, and the light chain CDR3 comprises the light chain CDR3 amino acid sequence in SEQ ID NO: 15; (3) the heavy chain CDR1 comprises the heavy chain CDR1 amino acid sequence in SEQ ID NO: 11, the heavy chain CDR2 comprises the heavy chain CDR2 amino acid sequence in SEQ ID NO: 11, the heavy chain CDR3 comprises the heavy chain CDR3 amino acid sequence in SEQ ID NO: 11, the light chain CDR1 comprises the light chain CDR1 amino acid sequence in SEQ ID NO: 15, the light chain CDR2 comprises the light chain CDR2 amino acid sequence in SEQ ID NO: 15, and the light chain CDR3 comprises the light chain CDR3 amino acid sequence in SEQ ID NO: 15; (4) the heavy chain CDR1 comprises the heavy chain CDR1 amino acid sequence in SEQ ID NO: 10, the heavy chain CDR2 comprises the heavy chain CDR2 amino acid sequence in SEQ ID NO: 10, the heavy chain CDR3 comprises the heavy chain CDR3 amino acid sequence in SEQ ID NO: 10, the light chain CDR1 comprises the light chain CDR1 amino acid sequence in SEQ ID NO: 16, the light chain CDR2 comprises the light chain CDR2 amino acid sequence in SEQ ID NO: 16, and the light chain CDR3 comprises the light chain CDR3 amino acid sequence in SEQ ID NO: 16; (5) the heavy chain CDR1 comprises the heavy chain CDR1 amino acid sequence in SEQ ID NO: 11, the heavy chain CDR2 comprises the heavy chain CDR2 amino acid sequence in SEQ ID NO: 11, the heavy chain CDR3 comprises the heavy chain CDR3 amino acid sequence in SEQ ID NO: 11, the light chain CDR1 comprises the light chain CDR1 amino acid sequence in SEQ ID NO: 16, the light chain CDR2 comprises the light chain CDR2 amino acid sequence in SEQ ID NO: 16, and the light chain CDR3 comprises the light chain CDR3 amino acid sequence in SEQ ID NO: 16; (6) the heavy chain CDR1 comprises the heavy chain CDR1 amino acid sequence in SEQ ID NO: 10, the heavy chain CDR2 comprises the heavy chain CDR2 amino acid sequence in SEQ ID NO: 10, the heavy chain CDR3 comprises the heavy chain CDR3 amino acid sequence in SEQ ID NO: 10, the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17; (7) the heavy chain CDR1 comprises the heavy chain CDR1 amino acid sequence in SEQ ID NO: 12, the heavy chain CDR2 comprises the heavy chain CDR2 amino acid sequence in SEQ ID NO: 12, the heavy chain CDR3 comprises the heavy chain CDR3 amino acid sequence in SEQ ID NO: 12, the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17; (8) the heavy chain CDR1 comprises the heavy chain CDR1 amino acid sequence in SEQ ID NO: 13, the heavy chain CDR2 comprises the heavy chain CDR2 amino acid sequence in SEQ ID NO: 13, the heavy chain CDR3 comprises the heavy chain CDR3 amino acid sequence in SEQ ID NO: 13, the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17; (9) the heavy chain CDR1 comprises the heavy chain CDR1 amino acid sequence in SEQ ID NO: 22, the heavy chain CDR2 comprises the heavy chain CDR2 amino acid sequence in SEQ ID NO: 22, the heavy chain CDR3 comprises the heavy chain CDR3 amino acid sequence in SEQ ID NO: 22, the light chain CDR1 comprises the light chain CDR1 amino acid sequence in SEQ ID NO: 23, the light chain CDR2 comprises the light chain CDR2 amino acid sequence in SEQ ID NO: 23, and the light chain CDR3 comprises the light chain CDR3 amino acid sequence in SEQ ID NO: 23; (10) the heavy chain CDR1 comprises the heavy chain CDR1 amino acid sequence in SEQ ID NO: 30, the heavy chain CDR2 comprises the heavy chain CDR2 amino acid sequence in SEQ ID NO: 30, the heavy chain CDR3 comprises the heavy chain CDR3 amino acid sequence in SEQ ID NO: 30, the light chain CDR1 comprises the light chain CDR1 amino acid sequence in SEQ ID NO: 31, the light chain CDR2 comprises the light chain CDR2 amino acid sequence in SEQ ID NO: 31, and the light chain CDR3 comprises the light chain CDR3 amino acid sequence in SEQ ID NO: 31; (11) the heavy chain CDR1 comprises the heavy chain CDR1 amino acid sequence in SEQ ID NO: 38, the heavy chain CDR2 comprises the heavy chain CDR2 amino acid sequence in SEQ ID NO: 38, the heavy chain CDR3 comprises the heavy chain CDR3 amino acid sequence in SEQ ID NO: 38, the light chain CDR1 comprises the light chain CDR1 amino acid sequence in SEQ ID NO: 39, the light chain CDR2 comprises the light chain CDR2 amino acid sequence in SEQ ID NO: 39, and the light chain CDR3 comprises the light chain CDR3 amino acid sequence in SEQ ID NO: 39; (12) the heavy chain CDR1 comprises the heavy chain CDR1 amino acid sequence in SEQ ID NO: 44, the heavy chain CDR2 comprises the heavy chain CDR2 amino acid sequence in SEQ ID NO: 44, the heavy chain CDR3 comprises the heavy chain CDR3 amino acid sequence in SEQ ID NO: 44, the light chain CDR1 comprises the light chain CDR1 amino acid sequence in SEQ ID NO: 45, the light chain CDR2 comprises the light chain CDR2 amino acid sequence in SEQ ID NO: 45, and the light chain CDR3 comprises the light chain CDR3 amino acid sequence in SEQ ID NO: 45; or (13) the heavy chain CDR1 comprises the heavy chain CDR1 amino acid sequence in SEQ ID NO: 52, the heavy chain CDR2 comprises the heavy chain CDR2 amino acid sequence in SEQ ID NO: 52, the heavy chain CDR3 comprises the heavy chain CDR3 amino acid sequence in SEQ ID NO: 52, the light chain CDR1 comprises the light chain CDR1 amino acid sequence in SEQ ID NO: 53, the light chain CDR2 comprises the light chain CDR2 amino acid sequence in SEQ ID NO: 53, and the light chain CDR3 comprises the light chain CDR3 amino acid sequence in SEQ ID NO: 53.

In some embodiments, the anti-CD40 antibody or the antigen-binding fragment thereof provided in the present disclosure comprises a heavy chain CDR1, a heavy chain CDR2, a heavy chain CDR3, a light chain CDR1, a light chain CDR2, and a light chain CDR3, wherein (1) the heavy chain CDR1, the heavy chain CDR2, the heavy chain CDR3, the light chain CDR1, the light chain CDR2, and the light chain CDR3 comprise the amino acid sequences set forth in SEQ ID NOs: 1, 2, 5, 6, 7, and 8, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 1, 2, 5, 6, 7, and 8, respectively; (2) the heavy chain CDR1, the heavy chain CDR2, the heavy chain CDR3, the light chain CDR1, the light chain CDR2, and the light chain CDR3 comprise the amino acid sequences set forth in SEQ ID NOs: 1, 3, 5, 6, 7, and 8, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 1, 3, 5, 6, 7, and 8, respectively; (3) the heavy chain CDR1, the heavy chain CDR2, the heavy chain CDR3, the light chain CDR1, the light chain CDR2, and the light chain CDR3 comprise the amino acid sequences set forth in SEQ ID NOs: 1, 4, 5, 6, 7, and 8, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 1, 4, 5, 6, 7, and 8, respectively; (4) the heavy chain CDR1, the heavy chain CDR2, the heavy chain CDR3, the light chain CDR1, the light chain CDR2, and the light chain CDR3 comprise the amino acid sequences set forth in SEQ ID NOs: 18, 19, 20, 21, 7, and 8, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 18, 19, 20, 21, 7, and 8, respectively; (5) the heavy chain CDR1, the heavy chain CDR2, the heavy chain CDR3, the light chain CDR1, the light chain CDR2, and the light chain CDR3 comprise the amino acid sequences set forth in SEQ ID NOs: 24, 25, 26, 27, 28, and 29, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 24, 25, 26, 27, 28, and 29, respectively; (6) the heavy chain CDR1, the heavy chain CDR2, the heavy chain CDR3, the light chain CDR1, the light chain CDR2, and the light chain CDR3 comprise the amino acid sequences set forth in SEQ ID NOs: 32, 33, 34, 35, 36, and 37, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 32, 33, 34, 35, 36, and 37, respectively; (7) the heavy chain CDR1, the heavy chain CDR2, the heavy chain CDR3, the light chain CDR1, the light chain CDR2, and the light chain CDR3 comprise the amino acid sequences set forth in SEQ ID NOs: 40, 41, 42, 43, 36, and 37, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 40, 41, 42, 43, 36, and 37, respectively; or (8) the heavy chain CDR1, the heavy chain CDR2, the heavy chain CDR3, the light chain CDR1, the light chain CDR2, and the light chain CDR3 comprise the amino acid sequences set forth in SEQ ID NOs: 46, 47, 48, 49, 50, and 51, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 46, 47, 48, 49, 50, and 51, respectively.

In some embodiments, the anti-CD40 antibody or the antigen-binding fragment thereof provided in the present disclosure comprises a heavy chain variable region comprising the amino acid sequence set forth in SEQ ID NO: 9, 10, 11, 12, 13, 22, 30, 38, 44, or 52, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 9, 10, 11, 12, 13, 22, 30, 38, 44, or 52.

In some embodiments, the anti-CD40 antibody or the antigen-binding fragment thereof provided in the present disclosure comprises a light chain variable region comprising the amino acid sequence set forth in SEQ ID NO: 14, 15, 16, 17, 23, 31, 39, 45, or 53, or an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 14, 15, 16, 17, 23, 31, 39, 45, or 53.

In some embodiments, the anti-CD40 antibody or the antigen-binding fragment thereof provided in the present disclosure comprises a heavy chain variable region and a light chain variable region, wherein (1) the heavy chain variable region and the light chain variable region comprise the amino acid sequences set forth in SEQ ID NOs: 9 and 14, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 9 and 14, respectively; (2) the heavy chain variable region and the light chain variable region comprise the amino acid sequences set forth in SEQ ID NOs: 10 and 15, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 10 and 15, respectively; (3) the heavy chain variable region and the light chain variable region comprise the amino acid sequences set forth in SEQ ID NOs: 11 and 15, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 11 and 15, respectively; (4) the heavy chain variable region and the light chain variable region comprise the amino acid sequences set forth in SEQ ID NOs: 10 and 16, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 10 and 16, respectively; (5) the heavy chain variable region and the light chain variable region comprise the amino acid sequences set forth in SEQ ID NOs: 11 and 16, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 11 and 16, respectively; (6) the heavy chain variable region and the light chain variable region comprise the amino acid sequences set forth in SEQ ID NOs: 10 and 17, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 10 and 17, respectively; (7) the heavy chain variable region and the light chain variable region comprise the amino acid sequences set forth in SEQ ID NOs: 12 and 17, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 12 and 17, respectively; (8) the heavy chain variable region and the light chain variable region comprise the amino acid sequences set forth in SEQ ID NOs: 13 and 17, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 13 and 17, respectively; (9) the heavy chain variable region and the light chain variable region comprise the amino acid sequences set forth in SEQ ID NOs: 22 and 23, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 22 and 23, respectively; (10) the heavy chain variable region and the light chain variable region comprise the amino acid sequences set forth in SEQ ID NOs: 30 and 31, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 30 and 31, respectively; (11) the heavy chain variable region and the light chain variable region comprise the amino acid sequences set forth in SEQ ID NOs: 38 and 39, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 38 and 39, respectively; (12) the heavy chain variable region and the light chain variable region comprise the amino acid sequences set forth in SEQ ID NOs: 44 and 45, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 44 and 45, respectively; or (13) the heavy chain variable region and the light chain variable region comprise the amino acid sequences set forth in SEQ ID NOs: 52 and 53, or amino acid sequences having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequences set forth in SEQ ID NOs: 52 and 53, respectively.

In some embodiments, the anti-CD40 antibody or the antigen-binding fragment thereof provided in the present disclosure comprises a heavy chain comprising a heavy chain variable region and a heavy chain constant region, and a light chain comprising a light chain variable region and a light chain constant region, wherein the heavy chain variable region and the light chain variable region comprise the amino acid sequences of the heavy chain variable region and the light chain variable region described above, respectively; the heavy chain constant region comprises a human IgG1, IgG2, or IgG4 constant region, preferably an IgG1 or IgG4 constant region, and the light chain constant region comprises a human κ constant region or human λ constant region. In some specific embodiments, the heavy chain constant region comprises the amino acid sequence set forth in SEQ ID NO: 54, 55, or 56, or an amino acid sequence comprising 1, 2, 3, 4, or 5 amino acid substitutions, deletions, and additions compared to the amino acid sequence set forth in SEQ ID NO: 54, 55, or 56, and the light chain constant region comprises the amino acid sequence set forth in SEQ ID NO: 57, or an amino acid sequence comprising 1, 2, 3, 4, or 5 amino acid substitutions, deletions, and additions compared to the amino acid sequence set forth in SEQ ID NO: 57.

In some embodiments, the anti-CD40 antibody or the antigen-binding fragment thereof provided in the present disclosure comprises a heavy chain and a light chain, wherein (1) the heavy chain comprises the amino acid sequences set forth in SEQ ID NOs: 9 and 54, and the light chain comprises the amino acid sequences set forth in SEQ ID NOs: 14 and 57; (2) the heavy chain comprises the amino acid sequences set forth in SEQ ID NOs: 10 and 54, and the light chain comprises the amino acid sequences set forth in SEQ ID NOs: 15 and 57; (3) the heavy chain comprises the amino acid sequences set forth in SEQ ID NOs: 11 and 54, and the light chain comprises the amino acid sequences set forth in SEQ ID NOs: 15 and 57; (4) the heavy chain comprises the amino acid sequences set forth in SEQ ID NOs: 10 and 54, and the light chain comprises the amino acid sequences set forth in SEQ ID NOs: 16 and 57; (5) the heavy chain comprises the amino acid sequences set forth in SEQ ID NOs: 11 and 54, and the light chain comprises the amino acid sequences set forth in SEQ ID NOs: 16 and 57; (6) the heavy chain comprises the amino acid sequences set forth in SEQ ID NOs: 10 and 54, and the light chain comprises the amino acid sequences set forth in SEQ ID NOs: 17 and 57; (7) the heavy chain comprises the amino acid sequences set forth in SEQ ID NOs: 12 and 54, and the light chain comprises the amino acid sequences set forth in SEQ ID NOs: 17 and 57; (8) the heavy chain comprises the amino acid sequences set forth in SEQ ID NOs: 13 and 54, and the light chain comprises the amino acid sequences set forth in SEQ ID NOs: 17 and 57; (9) the heavy chain comprises the amino acid sequences set forth in SEQ ID NOs: 22 and 54, and the light chain comprises the amino acid sequences set forth in SEQ ID NOs: 23 and 57; (10) the heavy chain comprises the amino acid sequences set forth in SEQ ID NOs: 30 and 54, and the light chain comprises the amino acid sequences set forth in SEQ ID NOs: 31 and 57; (11) the heavy chain comprises the amino acid sequences set forth in SEQ ID NOs: 38 and 54, and the light chain comprises the amino acid sequences set forth in SEQ ID NOs: 39 and 57; (12) the heavy chain comprises the amino acid sequences set forth in SEQ ID NOs: 44 and 54, and the light chain comprises the amino acid sequences set forth in SEQ ID NOs: 45 and 57; or (13) the heavy chain comprises the amino acid sequences set forth in SEQ ID NOs: 52 and 54, and the light chain comprises the amino acid sequences set forth in SEQ ID NOs: 53 and 57.

In some embodiments, the anti-CD40 antibody or the antigen-binding fragment thereof provided in the present disclosure comprises, or consists of two heavy (H) chains and two light (L) chains that are linked by disulfide bonds, wherein each of the heavy chains comprises the heavy chain variable region (VH) and the heavy chain constant region described above, wherein the heavy chain variable region (VH) comprises framework regions (FRs) and the heavy chain complementarity determining regions (HCDRs) described above; each of the light chains comprises the light chain variable region (VL) and the light chain constant region described above, wherein the light chain variable region (VL) comprises FRs and the light chain complementarity determining regions (LCDRs) described above; the C-terminus of the heavy chain variable region is linked to the N-terminus of the heavy chain constant region, and the C-terminus of the light chain variable region is linked to the N-terminus of the light chain constant region. The antibody in the present disclosure may be, e.g., a full-length antibody of the IgG1, IgG2, or IgG4 isotype. In some other embodiments, the antibody in the present disclosure may be a single-chain antibody (scFv), or an antibody fragment, e.g., an Fab, an F(ab')₂ fragment, an Fd fragment, an Fv fragment, a dAb, or an isolated CDR. In one aspect, the present disclosure provides an isolated antibody or an antigen-binding fragment thereof that binds to CD40, wherein the antibody or the antigen-binding fragment thereof is produced by a hybridoma selected from the group consisting of the hybridomas designated herein as A01, A02, A03, B01, B02, and B03. Accordingly, the present disclosure further encompasses an antibody or an antigen-binding fragment thereof produced by hybridoma A01, A02, A03, B01, B02, or B03, and any hybridoma that produces the antibody disclosed herein.

In one aspect, the present disclosure provides an antibody or an antigen-binding fragment thereof that binds to the same epitope on CD40 as any of the exemplary anti-CD40 antibodies or the antigen-binding fragments thereof of the present disclosure. In some embodiments, the present disclosure provides an antibody or an antigen-binding fragment thereof that competes for binding to CD40 with any of the exemplary anti-CD40 antibodies or the antigen-binding fragments thereof of the present disclosure.

In another aspect, the present disclosure further provides a recombinant polypeptide or a fusion protein comprising one or more of the anti-CD40 antibodies or the antigen-binding fragments thereof of the present disclosure, and at least one additional functional fragment including, but not limited to, another peptide, protein, cytokine, or receptor ligand. Such recombinant polypeptides or fusion proteins can be prepared by genetic modification, chemical methods, etc. In the present disclosure, the term "fusion protein" generally refers to a new polypeptide sequence obtained by joining two or more identical or different polypeptide sequences, and particularly, to a recombinant polypeptide sequence, comprising one or more identical or different polypeptide sequences that are not naturally linked.

In another aspect, the present disclosure further provides a bispecific molecule comprising one or more of the anti-CD40 antibodies or the antigen-binding fragments thereof of the present disclosure and at least some additional functional portions (e.g., another antibody or antigen-binding fragment thereof) that differ in specificity from the antibodies or the antigen-binding fragments of the present disclosure. The bispecific molecule can bind to at least two different binding sites or targets. The "bispecific molecule" used herein encompasses a molecule with the specificity against two targets (i.e., a bispecific molecule), three targets (i.e., a trispecific molecule), four targets (i.e., a tetraspecific molecule), or more targets. Such bispecific molecules can be prepared by genetic modification, chemical methods, etc.

In another aspect, the present disclosure further provides an immunoconjugate, such as an antibody-drug conjugate (ADC), comprising the anti-CD40 antibody or the antigen-binding portion thereof of the present disclosure, wherein the anti-CD40 antibody or the antigen-binding portion thereof of the present disclosure is linked to a therapeutic agent (e.g., a cytotoxic agent or an imaging agent, etc.). The anti-CD40 antibody or the antigen-binding portion thereof of the present disclosure may be part of a chimeric antigen receptor (CAR). The present disclosure further provides an immune cell comprising the chimeric antigen receptor, e.g., a T cell (i.e., CAR-T cell). In addition, the present disclosure further provides a gene vector, comprising a gene encoding the anti-CD40 antibody or the antigen-binding portion thereof of the present disclosure and allowing the entry of the gene into a mammalian cell (preferably a human cell) and the expression therein. Such gene vectors include, but are not limited to, naked plasmid vectors, yeast vectors, adenoviral vectors, adeno-associated viral vectors, retroviral vectors, poxvirus vectors, rhabdovirus vectors, or baculovirus vectors. Techniques for inserting DNA into these gene vectors are well known to those of ordinary skills in the art.

In another aspect, the present disclosure further provides a pharmaceutical composition comprising the anti-CD40 antibody or the antigen-binding fragment thereof of the present disclosure, and one or more pharmaceutically acceptable carriers. In some other embodiments, the present disclosure further provides a pharmaceutical composition comprising the recombinant polypeptide, the fusion protein, the bispecific molecule, the immunoconjugate, the chimeric antigen receptor, or the gene vector in the present disclosure, and a pharmaceutically acceptable carrier.

In another aspect, the present disclosure further provides an isolated nucleic acid encoding the anti-CD40 antibody or the antigen-binding fragment thereof of the present disclosure. The present disclosure further provides an expression vector comprising the nucleic acid, and a host cell comprising the expression vector.

In another aspect, the present disclosure further provides a method for preparing an anti-CD40 antibody or an antigen-binding fragment thereof, comprising the following steps: (i) expressing the anti-CD40 antibody or the antigen-binding fragment thereof in a host cell, and (ii) isolating the anti-CD40 antibody or the antigen-binding fragment thereof from the host cell or a cell culture thereof.

In other aspects, the present disclosure provides a method for treating or preventing an immune disease in a subject, comprising administering to the subject a therapeutically effective amount of the anti-CD40 antibody or the antigen-binding portion thereof, the encoding nucleic acid, the pharmaceutical composition, the recombinant polypeptide, the fusion protein, the bispecific molecule, the immunoconjugate, the chimeric antigen receptor, or the gene vector in the present disclosure. In certain embodiments, the subject is a human.

In some embodiments, the immune disease includes, but is not limited to, an inflammatory disease, an allergic reaction, an autoimmune disease, or a transplantation-related disease. In some specific embodiments, the immune disease includes, but is not limited to: an allergic reaction, Addison's disease, ankylosing spondylitis, spondyloarthritis, asthma, atherosclerosis, coronary heart disease, autoimmune hepatitis, autoimmune parotitis, type I diabetes, epididymitis, nephritis, Reiter's syndrome, thyroiditis, Graves' disease, Guillain-Barre syndrome (GBS), Hashimoto's disease, hemolytic anemia, idiopathic thrombocytopenia, systemic lupus erythematosus, subacute cutaneous lupus erythematosus, multiple sclerosis, myasthenia gravis, psoriasis, scleroderma, arthritis, sarcoidosis, Sjogren's syndrome, xerophthalmia, hidradenitis suppurativa, transplantation-related disease, vasculitis, and/or inflammatory bowel disease.

In some embodiments, the method further comprises administering a second therapeutic agent including a non-steroidal anti-inflammatory drug (NSAID), a salicylate, hydroxychloroquine, sulfasalazine, a corticosteroid, a cytotoxic drug, or an immunosuppressive drug and/or antibody.

Other features and advantages of the present disclosure will become more apparent with reference to the following detailed description and examples, which shall not be understood as limiting. The content of all of the documents, Genbank records, patents and published patent applications cited in the present disclosure is expressly incorporated herein by reference.

### BRIEF DESCRIPTION OF THE DRAWINGS

FIGs. 1A and 1B show the activity of pured mouse anti-CD40 antibodies to block the binding of 293T-hCD40-NPκB cells to CD40L proteins;
FIG. 2 shows the inhibitory activity of pured mouse anti-CD40 antibodies against the apoptosis of Ramos cells induced by CD40L and IL-4;
FIG. 3 shows the binding activity of chimeric anti-CD40 antibodies Chi-A01, Chi-A02, and Chi-A03 to CHO-K1-hCD40 cells;
FIG. 4 shows the agonistic activity of chimeric anti-CD40 antibodies Chi-A01, Chi-A02, Chi-A03, Chi-B01, Chi-B02, and Chi-B03 for the apoptosis of Ramos cells, with CP-870893 shown as the positive control;
FIG. 5 shows the binding activity of humanized anti-CD40 antibodies hzA01-3.1, hzA01-3.3, and hzA01-3.4 to 293T-hCD40-NFκB cells;
FIG. 6 shows the activity of humanized anti-CD40 antibodies hzA01-1.1, hzA01-2.1, hzA01-3.1, hzA01-3.3, and hzA01-3.4 to block the binding of 293T-hCD40-NFκB cells to CHO-K1-hCD40L cells;
FIG. 7 shows the inhibitory activity of humanized anti-CD40 antibodies hzA01-1.1, hzA01-2.1, hzA01-3.1, hzA01-3.3, and hzA01-3.4 against the apoptosis of Ramos cells induced by CD40L and IL-4;
FIG. 8 shows the agonistic activity of humanized anti-CD40 antibodies hzA01-1.1, hzA01-2.1, hzA01-3.1, hzA01-3.3, and hzA01-3.4 for the apoptosis of Ramos cells, with CP-870893 shown as the positive control;
FIG. 9 shows the inhibitory activity of humanized anti-CD40 antibodies hzA01-3.1, hzA01-3.3, and hzA01-3.4 against the CD40L-induced expression of costimulatory molecule CD86 on human peripheral blood B lymphocytes;
FIG. 10 shows the inhibitory activity of humanized anti-CD40 antibodies hzA01-3.1, hzA01-3.3, and hzA01-3.4 against the proliferation of human peripheral blood B lymphocytes induced by CD40L and IL-4;
FIG. 11 shows the agonistic activity of humanized anti-CD40 antibodies hzA01-3.1, hzA01-3.3, and hzA01-3.4 for inducing the proliferation of human peripheral blood B lymphocytes, with CP-870893 shown as the positive control;
FIG. 12 shows the amount of IFNγ released in response to humanized anti-CD40 antibody hzA01-3.3 in the MLR reaction, wherein neither the anti-CD40 antibody nor the IgG4 isotype control antibody was added to the mDC and DC (without differentiation culture) groups, which were shown as the negative controls;
FIG. 13 shows the agonistic activity of humanized anti-CD40 antibody hzA01-3.3 on imDC cell maturation, with CP-870893 shown as the positive control;
FIG. 14 shows the ADCC effect mediated by humanized anti-CD40 antibody hzA01-3.3, with rituximab shown as the positive control;
FIG. 15 shows the CDC effect mediated by humanized anti-CD40 antibody hzA01-3.3, with rituximab shown as the positive control;
FIG. 16A shows the concentration of human anti-mouse IgM antibodies in NDG mice serum after the treatment with humanized anti-CD40 antibody hzA01-3.3, and FIG. 16B shows the concentration of human anti-mouse IgG antibodies in NDG mice serum after the treatment with humanized anti-CD40 antibody hzA01-3.3;
FIG. 17A shows the content of infiltrating human CD4⁺ T cells in the spleen of mice after treatment with humanized anti-CD40 antibody hzA01-3.3, and FIG. 17B shows the content of infiltrating human CD19⁺ B cells in the spleen of mice after treatment with humanized anti-CD40 antibody hzA01-3.3, wherein the comparisons were made with the IgG4 isotype control group by the t-test, and * denotes P ≤ 0.05, ** denotes P < 0.01, and *** denotes P < 0.001;
FIG. 18 shows the effect of humanized anti-CD40 antibody hzA01-3.3 on the body weight of a Sjogren's syndrome mouse model;
FIG. 19 shows the effect of humanized anti-CD40 antibody hzA01-3.3 on the saliva flow rate of the Sjogren's syndrome mouse model;

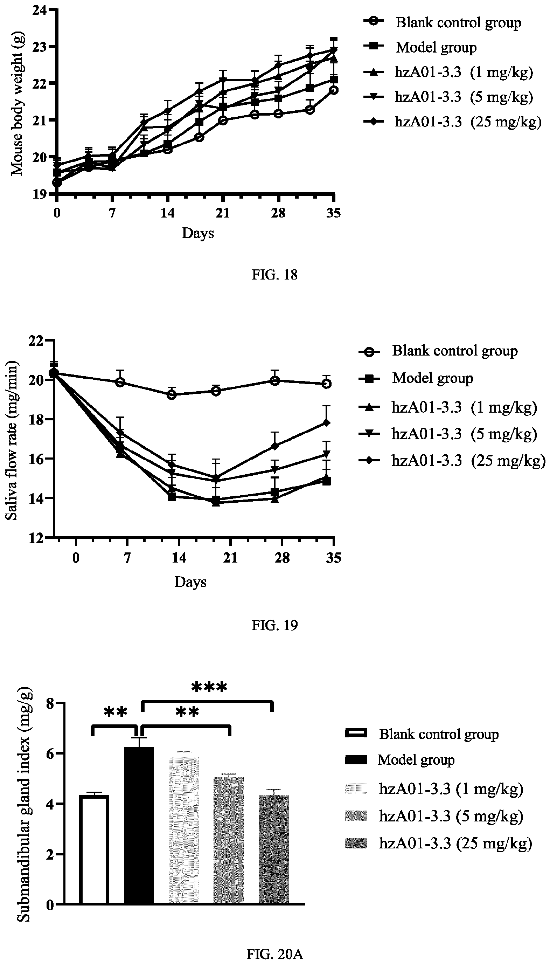

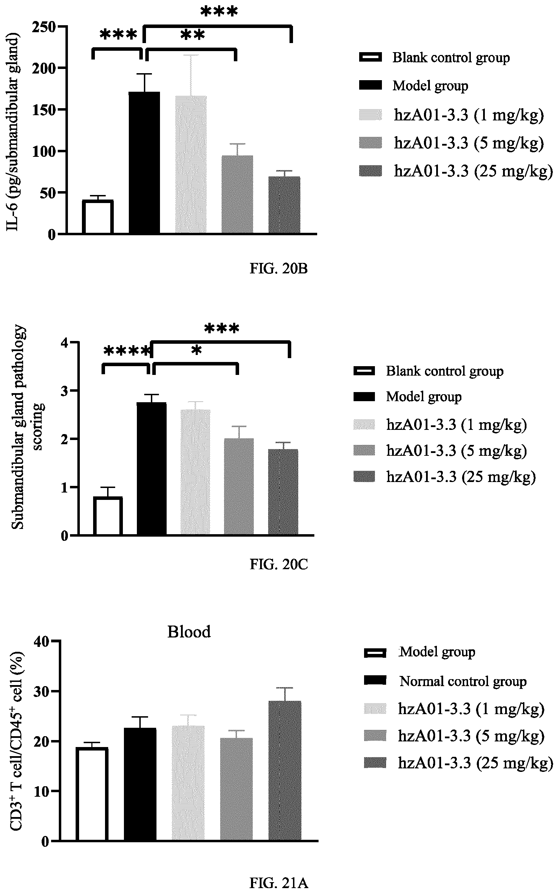

FIG. 20A shows the submandibular gland index of the Sjogren's syndrome mouse model after treatment with humanized anti-CD40 antibody hzA01-3.3, FIG. 20B shows the content of IL-6 in the submandibular gland of the Sjogren's syndrome mouse model after treatment with humanized anti-CD40 antibody hzA01-3.3, and FIG. 20C shows the submandibular gland pathological score of the Sjogren's syndrome mouse model after treatment with humanized anti-CD40 antibody hzA01-3.3, wherein the comparisons (the blank control group vs. the model group, and the hzA01-3.3 treatment group vs. the model group) were made by the t-test, and * denotes P ≤ 0.05, ** denotes P < 0.01, and *** denotes P < 0.001;
FIGs. 21A-21D show the proportions of viable CD3⁺ T cells, CD4⁺ T cells, CD8⁺ T cells, and CD19⁺ B cells to viable CD45⁺ cells in the blood of the Sjogren's syndrome mouse model after treatment with humanized anti-CD40 antibody hzA01-3.3, and FIGs. 21E-21H show the proportions of viable CD3⁺ T cells, CD4⁺ T cells, CD8⁺ T cells and CD19⁺ B cells to viable CD45⁺ cells in the spleen of the Sjogren's syndrome mouse model after treatment with humanized anti-CD40 antibody hzA01-3.3.

### DETAILED DESCRIPTION

It should be appreciated that the terms used herein are for the purpose of describing specific embodiments only rather than limiting. Unless otherwise defined, all of the technical and scientific terms used herein have the same meaning as commonly understood by those of ordinary skills in the art to which the present disclosure belongs.

The term "CD40" includes CD40 variants, homologs, orthologs, and paralogs. For example, in some examples, an antibody specific for human CD40 protein may cross-react with a CD40 protein of another species (e.g., monkey) in certain circumstances. In some other embodiments, an antibody specific for human CD40 protein may be completely specific for human CD40 protein and does not cross-react with proteins of other species or other types, or may only cross-react with CD40 proteins of some other species but not all the other species.

CD40 is known and may be referred to as B cell surface antigen CD40, Bp50, CD40L receptor, CDW40, MGC9013, p50, and tumor necrosis factor receptor superfamily member 5 (TNFRSF5). The terms "human CD40" and "hCD40" are used herein interchangeably and refer to a protein having the amino acid sequence of human CD40, for example, a CD40 protein comprising the amino acid sequence set forth in SEQ ID NO: 62. The terms "monkey CD40", "cyno CD40", and the like are used herein interchangeably and refer to a protein having the amino acid sequence of monkey CD40, for example, a CD40 protein comprising the amino acid sequence set forth in SEQ ID NO. 63.

As used herein, the term "antibody" refers to a binding protein having at least one antigen (e.g., CD40)-binding domain. The antibody or the antigen-binding fragment thereof of the present disclosure may be an intact antibody or any fragment thereof, including a monoclonal antibody or a fragment thereof, and an antibody variant or a fragment thereof. Examples of the antibody or the antigen-binding fragment thereof include a monospecific antibody, a bispecific antibody, a trispecific antibody, a multispecific antibody, an Fab fragment, an F(ab')2 fragment, an Fv fragment, an isolated CDR region, a single-chain Fv (scFv), and any other antibody fragments known in the art. The anti-CD40 antibody or the antigen-binding fragment thereof disclosed herein may be of the IgG1, IgG2, IgG3, or IgG4 isotypes. The term "isotype" refers to the class of antibodies encoded by the heavy chain constant region gene. In some embodiments, the anti-CD40 antibody or the antigen-binding fragment thereof disclosed herein is of the IgG1 and IgG4 isotypes. The anti-CD40 antibody or the antigen-binding fragment thereof of the present disclosure may be derived from any species, including but not limited to mouse, rat, rabbit, primates, llama, and human. The anti-CD40 antibody or the antigen-binding fragment thereof of the present disclosure may be a murine antibody, a chimeric antibody, a humanized antibody, or a human antibody. Unless otherwise stated, the "antibody" in the present disclosure includes a full-length antibody and any antigen-binding portion (i.e., "antigen-binding fragment") or single chain thereof. Generally, the full-length antibody is a glycoprotein comprising two heavy (H) chains and two light (L) chains, wherein the heavy chains and the light chains are linked by disulfide bonds. Each of the heavy chains consists of a heavy chain variable region (VH) and a heavy chain constant region. The heavy chain constant region consists of three domains: CH1, CH2, and CH3. Each of the light chains consists of a light chain variable region (VL) and a light chain constant region. The light chain constant region consists of one domain CL. The VH and VL regions can further be divided into hypervariable regions, (i.e., the complementarity determining regions or CDRs) and framework regions (FRs) with relatively conserved sequences. Each VH and VL consists of three CDRs and four FRs, arranged from the amino-terminus to the carboxyl-terminus in the following order: FR1, CDR1, FR2, CDR2, FR3, CDR3, FR4. The variable regions of the antibody comprise binding domains that interact with antigens. The constant regions of the antibody can mediate the binding of immunoglobulins to host tissues or factors, including various cells of the immune system (e.g., effector cells) and the first component (C1q) of the classical complement system. Meanwhile, as known to those skilled in the art, a special "full-length antibody", e.g., a nanobody, may have only heavy (H) chains but no light (L) chains.

The "antigen-binding fragment" or "antibody-binding portion" of an antibody refers to one or more fragments of the antibody that retain the functionality of specifically binding to an antigen (e.g., CD40 protein). It has been demonstrated that the antigen-binding functionality of an antibody can be implemented by fragments of a full-length antibody. Examples encompassed within the term "antigen-binding portion/fragment" of an antibody include: (i) an Fab fragment: a monovalent fragment consisting of VL, VH, CL and CH1 domains; (ii) an F(ab')2 fragment, a bivalent fragment comprising two Fab fragments linked by a disulfide bridge at the hinge region; (iii) an Fd fragment consisting of VH and CH1 domains; (iv) an Fv fragment consisting of VL and VH domains of a single arm of the antibody; (v) a dAb fragment consisting of VH domains (see Ward et al., Nature. 341:544-546 (1989)); (vi) an isolated complementarity determining region (CDR); and (vii) a nanobody, a heavy chain variable region comprising one variable domain and two constant domains. Furthermore, although the two domains VL and VH of the Fv fragment are encoded by different genes, the VL and VH domains can be joined via a linker by recombinant means into a single protein chain in which VL and VH pair to form a monovalent molecule (referred to as a single-chain Fv (scFv); see, Bird et al., Science. 242:423-426 (1988); Huston et al., Proc. Natl. Acad. Sci. 85:5879-5883 (1988)). Such single-chain antibodies are also included within the term antigen-binding portion/fragment. Furthermore, recombinant polypeptides, fusion proteins, and bispecific molecules comprising such antigen-binding portion/fragments are also included within the term antigen-binding portion/fragment. These antibody fragments can be obtained using conventional techniques known to those skilled in the art, and the fragments can be subjected to functional screening using the same method as full-length antibodies.

The "mouse antibody" or "murine antibody" refers to an antibody in which the framework regions and CDRs in the variable region are derived from mouse germline immunoglobulin sequences. Furthermore, if the antibody comprises a constant region, the constant region is also derived from mouse germline immunoglobulin sequences. The mouse antibody of the present disclosure may comprise amino acid residues not encoded by mouse germline immunoglobulin sequences (e.g., mutations introduced by *in vitro* random mutation or point mutation or by *in vivo* somatic mutation), but "mouse antibody" does not include antibodies in which CDR sequences derived from other mammalian species are inserted into mouse framework sequences.

The "chimeric antibody" refers to an antibody formed by combining genetic substances of non-human origin with genetic substances of human origin. More generally, the chimeric antibody refers to an antibody comprising genetic substances from one species and genetic substances from another species. For example, variable regions of both the light and heavy chain may be derived from the variable region of an antibody from one animal species (e.g., mouse, rat, etc.) while the constant portions are homologous to antibody sequences from another species (e.g., human). For example, to give a chimeric antibody, B cells or hybridoma cells of non-human origin may be used to produce the variable regions, while the constant regions in combination therewith are derived from a human. In the present disclosure, chimeric antibodies are also denoted as "Chi".

The "humanized antibody" refers to an antibody comprising complementarity determining regions (CDRs) derived from a non-human antibody, and framework and constant regions derived from a human antibody. For example, the humanized antibody that binds to CD40 provided herein may comprise CDRs derived from one or more murine antibodies as well as human framework and constant regions. Thus, in some embodiments, the humanized antibody provided herein binds to the same epitope on CD40 as the murine antibody from which the CDRs of the humanized antibody are derived. Exemplary humanized antibodies are provided herein. Additional humanized antibodies that bind to CD40 or variants thereof comprising the heavy and light chain CDRs provided herein may be generated using any human framework sequences, and are also included in the present disclosure. In some embodiments, framework sequences suitable for use in the present disclosure include those similar in structure to the framework sequences provided herein. Additional modifications may be made in the framework regions to alter the properties of the antibodies provided herein. Such additional framework region modifications may include chemical modifications, point mutations for reducing immunogenicity or removing T cell epitopes, or back mutations to residues in original germline sequences. In some embodiments, such modifications include those corresponding to the mutations exemplified herein, including reversions to germline sequences. For example, in some embodiments, one or more amino acids in the human VH and/or VL framework regions of the humanized antibodies provided herein are reverted to the corresponding amino acids in the parent murine antibodies. In the present disclosure, humanized antibodies are also denoted as "hz".

The term "Fc domain" or "Fc region" refers to an antibody sequence comprising CH2 and CH3 constant domains as defined according to the Kabat numbering system. The Fc region may be derived from a human IgG. For example, the Fc region may be derived from a human IgG1 or human IgG4 Fc region.

"Derived" as described herein, when used with respect to a molecule or polypeptide relative to a reference antibody or other binding proteins, means that a molecule or polypeptide can specifically bind to the same epitope as the reference antibody or other binding proteins.

"Isolated" means that a target compound (e.g., an antibody, an antigen-binding fragment, or a nucleic acid) has been isolated from its natural environment.

The "antibody specifically binding to an antigen" and "antibody specific for an antigen" are used herein interchangeably with the term "antibody that specifically binds to an antigen". The antibody "specifically binding to human CD40" refers to an antibody that binds to human CD40 (or possibly other CD40 of non-human species) but does not substantially bind to non-CD40. Preferably, the antibody binds to human CD40 with "high affinity", i.e., a K_{D} of 5.0 × 10⁻⁸ M or less, 1.0 × 10⁻⁸ M or less, preferably 5.0 × 10⁻⁹ M or less, more preferably 1.0 × 10⁻⁹ M or less.

The term "not substantially bind to" a protein or cell means the absence of binding to the protein or cell, or binding with high affinity, i.e., binding to the protein or cell with a K_{D} of 1.0 × 10⁻⁶ M or higher, preferably 1.0 × 10⁻⁵ M or higher, 1.0 × 10⁻⁴ M or higher, and more preferably 1.0 × 10⁻³ M or higher, 1.0 × 10⁻² M or higher.

The term "high affinity" for IgG refers to binding to an antigen with a K_{D} of 5.0 × 10⁻⁸ M or less, 1.0 × 10⁻⁸ M or less, preferably 5.0 × 10⁻⁹ M or less, more preferably 1.0 × 10⁻⁹ M or less. However, for other antibody isotypes, the "high-affinity" binding may be different. For example, the "high-affinity" binding of IgM isotype refers to binding to an antigen with a K_{D} of 1.0 × 10⁻⁶ M or less, preferably 1.0 × 10⁻⁷ M or less, more preferably 1.0 × 10⁻⁸ M or less. The "identity" refers to the similarity between two or more nucleic acid sequences or between two or more polypeptide sequences. The sequence identity of the present disclosure is at least 85%, 90% or 95%, preferably at least 95%. Non-limiting examples include: 85%, 86%, 87%, 88%, 89%, 90%, 91%, 92%, 93%, 94%, 95%, 96%, 97%, 98%, 99%, or 100%. Sequence comparison and percent identity determination between two sequences can be performed using the BLASTN/BLASTP algorithm on the National Center For Biotechnology Institute with default settings.

The antibody that "competes for binding" refers to an antibody that partially or completely blocks the binding of other antibodies to a target. Whether two antibodies compete with each other for binding to a target, i.e., whether and to what extent one antibody blocks the binding of the other antibody to the target, may be determined using competition assays known in the art, e.g., solid-phase direct or indirect radioimmunoassay (RIA), solid-phase direct or indirect enzyme immunoassay (EIA), sandwich competition assay, and the like. In certain embodiments, one antibody competes with the other antibody for binding to the target and blocks the binding of the other antibody to the target by at least 10%, 20%, 30%, 40%, 50%, 60%, 70%, 80%, 90%, or 100%.

Two or more antibodies "bind to the same epitope" means that the antibodies bind to the same segment of amino acid residues, as determined by a given method. Techniques for determining whether an antibody binds to "the same epitope on CD40" as the antibodies described herein include, for example, epitope mapping methods, such as X-ray diffraction analysis of the crystals of antigen: antibody complexes and hydrogen/deuterium exchange mass spectrometry (HDX-MS).

The term "EC₅₀", also known as half maximal effective concentration, refers to a concentration of the antibody that can cause 50% of the maximum effect after a specified exposure time. The term "IC₅₀", also known as half maximal inhibitory concentration, refers to a concentration of the antibody that inhibits a specific biological or biochemical function by 50% relative to the case where the antibody is absent. Both EC₅₀ and IC₅₀ may be measured by ELISA or FACS assay or any other method known in the art.

The "K_{D}" refers to the equilibrium dissociation constant, which is derived from the ratio of K_{d} to Kₐ (i.e., K_{d}/Kₐ) and expressed in molar concentration (M). The K_{D} value of an antibody may be determined using methods well established in the art. A preferred method for determining the K_{D} of an antibody is surface plasmon resonance, preferably using a biosensor system such as BIACORE^{®} surface plasmon resonance system for analysis.

The "cross-linking" refers to the high-order multimerization of CD40 on cells induced by the binding of an anti-CD40 antibody to FcyR (e.g., cis or trans FcyRIfb), thereby resulting in the induction of CD40 agonistic activity. The "patient" or "subject" includes any human or non-human animal. The term "non-human animal" includes all vertebrates, e.g., mammals and non-mammals, preferably mammals, e.g., non-human primates, sheep, dogs, cats, cows and horses.

The "effective dose" or "effective amount" refers to an amount sufficient to achieve, or at least partially achieve a desired effect. The therapeutically "effective amount" or "effective dose" of a drug or therapeutic agent refers to an amount sufficient to prevent or ameliorate symptoms associated with a disease or disorder, preferably an amount that causes a reduction in the severity of the symptoms of the disease or an increase in the frequency and duration of asymptomatic phases, or prevents damage or inability caused by the disease, when used alone or in combination with another therapeutic agent. The therapeutically effective amount is related to the disease to be treated, wherein the actual effective amount can be readily determined by those skilled in the art.

Unless otherwise stated, "about" in the present disclosure means the fluctuation within ±5%, preferably within ±2%, and more preferably within ±1% of the specified numerical range given. For example, a pH of about 5.5 means a pH of 5.5±5%, preferably a pH of 5.5±2%, and more preferably a pH of 5.5±1%. The use of singular forms encompasses plural forms unless otherwise specified.

The word "a" or "an" means "at least a" or "at least an", the phrase "at least one" means the same as "one or more", and "and/or" is used to refer to "and" or "or", unless otherwise specified.

As described herein, any percentage range, ratio range, or integer range shall be interpreted as including the value of any integer within the listed range, unless otherwise indicated.

In the present disclosure, unless the context dictates otherwise, the words "comprise", "include" and "contain" will be interpreted as including the steps or elements or a group of steps or elements but not excluding any other steps or elements or groups of steps or elements. "Consist of..." means including and being limited to what follows the phrase "consist of...". Thus, the phrase "consist of..." means that the listed elements are required or necessary and that no other elements can be present. "Substantially consist of..." means including any listed element that follows the phrase and being limited to other elements that do not interfere with or are favorable for the listed elements' activity or effects as detailed in the present disclosure. Thus, the phrase "substantially consist of..." means that the listed elements are required or necessary but other elements are optional and can be present or absent depending on whether they affect the listed elements' activity or effects.

Aspects of the present disclosure are described in more detail below.

The amino acid sequence IDs (SEQ ID NOs.) of the heavy chain variable region, the light chain variable region, and the CDRs of the exemplary antibodies or antigen-binding fragments thereof of the present disclosure are provided in Table 1 below. Some antibodies have identical CDRs, and some antibodies have identical VHs or VLs. The heavy chain constant region of the antibody may be a human IgG1, IgG2, or IgG4 constant region, preferably an IgG1 and IgG4 constant region, for example, comprising the amino acid sequence set forth in SEQ ID NO: 54, 55, or 56, or an amino acid sequence comprising 1, 2, 3, 4, or 5 amino acid substitutions, deletions, and additions compared to the amino acid sequence set forth in SEQ ID NO: 54, 55, or 56. The light chain constant region of the antibody may be a human κ constant region or a human λ constant region, for example, comprising the amino acid sequence set forth in SEQ ID NO: 57, or an amino acid sequence comprising 1, 2, 3, 4, or 5 amino acid substitutions, deletions, and additions compared to the amino acid sequence set forth in SEQ ID NO: 57. The antibodies may also comprise a mouse IgG1 or IgG4 heavy chain constant region and/or a mouse κ constant region or mouse λ constant region.

**Table 1. Amino acid sequence IDs (SEQ ID NOs.) of variable regions and CDRs**

| Antibody | HCDR1 | HCDR2 | HCDR3 | VH | LCDR1 | LCDR2 | LCDR3 | VL |
|---|---|---|---|---|---|---|---|---|
| Mouse and chimeric A01 | 1 | 2 | 5 | 9 | 6 | 7 | 8 | 14 |
| hzA01-1.1 | | 3 | | 10 | | | | 15 |
| hzA01-1.2 | | | | 11 | | | | |
| hzA01-2.1 | | | | 10 | | | | 16 |
| hzA01-2.2 | | | | 11 | | | | |
| hzA01-3.1 | | | | 10 | | | | 17 |
| hzA01-3.3 | | 4 | | 12 | | | | |
| hzA01-3.4 | | 2 | | 13 | | | | |
| Mouse and chimeric A02 | 18 | 19 | 20 | 22 | 21 | | | 23 |
| Mouse and chimeric A03 | 24 | 25 | 26 | 30 | 27 | 28 | 29 | 31 |
| Mouse and chimeric B01 | 32 | 33 | 34 | 38 | 35 | 36 | 37 | 39 |
| Mouse and chimeric B02 | 40 | 41 | 42 | 44 | 43 | | | 45 |
| Mouse and chimeric B03 | 46 | 47 | 48 | 52 | 49 | 50 | 51 | 53 |

Given the amino acid sequence of the variable region of an antibody, those skilled in the art can determine which residues constitute a particular CDR using conventional methods. It is well known to those skilled in the art that the CDRs of an antibody may be defined by a variety of methods, for example, by the numbering system/method of Kabat, Chothia, IMGT, AbM, or Contact; or by a combination of two or more of the numbering system/method of Kabat, Chothia, IMGT, AbM, or Contact (e.g., HCDR1 is defined by AbM, HCDR2 is defined by Kabat or AbM, HCDR3 is defined by IMGT or Kabat, and LCDR1-3 is defined by Kabat); it may also be defined by a combined numbering system comprising Kabat and Chothia, wherein the combined numbering system combines the ranges defined by Kabat and Chothia and takes a greater range on this basis (e.g., if Kabat defines HCDR1 as H31-H35, and Chothia defines HCDR1 as H26-H32, the combined system defines HCDR1 as H26-H35). The exact numbering and position of the CDRs varies among different numbering systems. It will be appreciated by those skilled in the art that, unless otherwise specified, the term "CDRs" or "complementarity determining regions" of a given antibody or a region thereof (e.g., a variable region) will be understood to encompass complementarity determining regions defined by any of the known schemes.

Although the CDRs claimed in the present disclosure are based on the sequences shown in Table 1 and Table 10, the corresponding amino acid sequences according to other CDR definition rules shall also fall within the protection scope of the present disclosure. For example, when CDRs are defined by the Kabat definition rules, the amino acid sequence of HCDR1 of mouse, chimeric, and humanized A01 is TSGVH (SEQ ID NO: 79); the amino acid sequence of HCDR2 of mouse, chimeric A01 and hzA01-3.4 is VIWAGGDTNYNSALMS (SEQ ID NO: 2); the amino acid sequence of HCDR2 of hzA01-1.1, hzA01-2.1, and hzA01-3.1 is VIWAGGDTNYNPSLKS (SEQ ID NO: 80); the amino acid sequence of HCDR2 of hzA01-1.2 and hzA01-2.2 is VIWAGGDTNYADSVKG (SEQ ID NO: 81); the amino acid sequence of HCDR2 of hzA01-3.3 is VIWAGGDTNYNSALKS (SEQ ID NO: 4); the amino acid sequence of HCDR3 of mouse, chimeric, and humanized A01 is HGHFDV (SEQ ID NO: 82); the amino acid sequence of LCDR1 of mouse, chimeric, and humanized A01 is RSSQSLVHSSGNTYLQ (SEQ ID NO: 6); the amino acid sequence of LCDR2 of mouse, chimeric, and humanized A01 is KVSNRFS (SEQ ID NO: 7); the amino acid sequence of LCDR3 of mouse, chimeric, and humanized A01 is SQTTHVPWT (SEQ ID NO: 8).

The VH and/or VL sequences (or CDR sequences) of other anti-CD40 antibodies that bind to human CD40 may be "mixed and paired" with the VH and/or VL sequences (or CDR sequences) of the anti-CD40 antibody or the antigen-binding portion thereof of the present disclosure. Preferably, when VH and VL chains (or CDRs thereof) are mixed and paired, the VH sequence in a particular VH/VL pair can be substituted with a structurally similar VH sequence. Likewise, it is preferred to substitute the VL sequence in a particular VH/VL pair with a structurally similar VL sequence.

Thus, in one embodiment, the antibody or the antigen-binding fragment thereof of the present disclosure comprises:
(a) a heavy chain variable region comprising an amino acid sequence listed in Table 1 and Table 10; and
(b) a light chain variable region comprising an amino acid sequence listed in Table 1 and Table 10, or the VL of another anti-CD40 antibody, wherein the antibody or the antigen-binding portion thereof specifically binds to human CD40.

In another embodiment, the antibody or the antigen-binding fragment thereof of the present disclosure comprises:
(a) the HCDR1, HCDR2, and HCDR3 listed in Table 1 and Table 10; and
(b) the LCDR1, LCDR2, and LCDR3 listed in Table 1 and Table 10, or the CDRs of the light chain variable region of another anti-CD40 antibody, wherein the antibody or the antigen-binding portion thereof specifically binds to human CD40.

In another embodiment, the antibody or the antigen-binding fragment thereof of the present disclosure comprises the HCDR2 listed in Table 1 and Table 10 and CDRs of other anti-CD40 antibodies, for example, an HCDR1 and/or an HCDR3 of other anti-CD40 antibodies, and/or an LCDR1, an LCDR2, and/or an LCDR3 of other anti-CD40 antibodies.

Furthermore, it is well known in the art that the CDR3 domain is independent of the CDR1 and/or CDR2 domains and can independently determine the antibody's binding specificity for identical antigens, and that multiple antibodies with the same binding specificity can be predicted based on the CDR3 sequence.

In another embodiment, the antibody or the antigen-binding fragment thereof of the present disclosure comprises the HCDR2 listed in Table 1 and Table 10, and the HCDR3 listed in Table 1 and Table 10 and/or the LCDR3 listed in Table 1, or an HCDR3 and/or an LCDR3 of another anti-CD40 antibody, wherein the antibody or the antigen-binding portion thereof specifically binds to human CD40. Such antibodies preferably (a) compete with the anti-CD40 antibody of the present disclosure for binding to CD40; (b) retain the functional properties; (c) bind to the same epitope; and/or (d) have similar binding affinities. In another embodiment, the antibody or the antigen-binding portion thereof of the present disclosure further comprises the LCDR2 listed in Table 1 and Table 10, or an LCDR2 of another anti-CD40 antibody, wherein the antibody or the antigen-binding portion thereof specifically binds to human CD40. In another embodiment, the antibody or the antigen-binding portion thereof of the present disclosure further comprises the HCDR1 listed in Table 1 and Table 10 and/or the LCDR1 listed in Table 1 and Table 10, or an HCDR1 and/or an LCDR1 of another anti-CD40 antibody, wherein the antibody or the antigen-binding portion thereof specifically binds to human CD40.

In another embodiment, the heavy chain variable region and/or the light chain variable region or the CDR1, CDR2, and CDR3 sequences of the antibody or the antigen-binding portion thereof of the present disclosure may comprise one or more conservative modifications. It will be appreciated in the art that some conservative sequence modifications do not eliminate the antigen-binding specificity.

Thus, in one embodiment, the antibody or the antigen-binding portion thereof of the present disclosure comprises a heavy chain variable region and/or a light chain variable region each comprising a CDR1, a CDR2, and a CDR3, wherein:
(a) the HCDR1 sequence comprises a sequence listed in Table 1 and Table 10, and/or conservative modifications thereof; and/or
(b) the HCDR2 sequence comprises a sequence listed in Table 1 and Table 10, and/or conservative modifications thereof; and/or
(c) the HCDR3 sequence comprises a sequence listed in Table 1 and Table 10, and/or conservative modifications thereof; and/or
(d) the LCDR1 and/or the LCDR2 and/or the LCDR3 sequences comprise sequences listed in Table 1 and Table 10; and/or conservative modifications thereof; and
(e) the antibody or the antigen-binding portion thereof specifically binds to human CD40.

The term "conservative sequence modification" as used herein refers to an amino acid modification that does not significantly affect or alter the binding properties of the antibody. Such conservative modifications include amino acid substitutions, additions, and deletions. Modifications can be introduced into the antibody of the present disclosure using standard techniques known in the art, e.g., point mutation and PCR-mediated mutation.

Conservative amino acid substitutions are those in which an amino acid residue is replaced with an amino acid residue having similar structural or chemical properties (e.g., similar side chains). Families of amino acid residues having similar side chains are known in the art. Such amino acid residue families include amino acids with basic side chains (e.g., lysine, arginine, and histidine), amino acids with acidic side chains (e.g., aspartic acid, and glutamic acid), amino acids with uncharged polar side chains (e.g., glycine, asparagine, glutamine, serine, threonine, tyrosine, cysteine, and tryptophan), amino acids with non-polar side chains (e.g., alanine, valine, leucine, isoleucine, proline, phenylalanine, and methionine), amino acids with β-branched side chains (e.g., threonine, valine, and isoleucine), and amino acids with aromatic side chains (e.g., tyrosine, phenylalanine, tryptophan, and histidine). Thus, one or more amino acid residues in the CDR regions of the anti-CD40 antibody or the antigen-binding portion thereof of the present disclosure can be substituted with other amino acid residues of the same side chain family, and the resulting antibody can be tested for functionality using the functional assays described herein.

The antibody or the antigen-binding fragment thereof of the present disclosure comprises variable region (including CDRs and/or framework regions) modifications, or the antibody or the antigen-binding portion thereof of the present disclosure may further comprise Fc modifications, for example, to alter the effector functionality of the antibody. Thus, one embodiment of the present disclosure provides an isolated anti-CD40 monoclonal antibody or an antigen-binding fragment thereof, comprising a heavy chain variable region comprising an HCDR1, an HCDR2, and an HCDR3 of the sequences described above in the present disclosure and/or a light chain variable region comprising an LCDR1, an LCDR2, and an LCDR3 of the sequences described above in the present disclosure, but comprises framework sequences different from the sequences described above in the present disclosure. Such framework sequences can be found in public DNA databases or public references including germline antibody gene sequences. Such framework sequences are preferably those that are structurally similar to the framework sequences used for the anti-CD40 antibody of the present disclosure. The CDR1, CDR2 and CDR3 sequences may be grafted into a framework region that comprises the same sequence as the germline immunoglobulin gene from which the framework sequences are derived, or the CDR sequences may be grafted into framework regions that comprise one or more mutations compared to the germline sequence. For example, in some cases, it may be beneficial to mutate residues in the framework regions; such mutations may maintain or enhance the antigen-binding ability of the antibody (see, e.g., U.S. Pat. Nos. 5,530,101; 5,585,089; 5,693,762, and 6,180,370).

Another type of variable region modification is to mutate the amino acid residues within the CDR1, CDR2, and/or CDR3 to improve one or more properties (e.g., affinity and physicochemical properties) of the target antibody. Mutations may be introduced by point mutation or PCR-mediated mutations, and the effect of the mutations on antibody binding or other functional properties may be assessed through *in vitro* or *in vivo* assays known in the art. The conservative modifications may be amino acid substitutions, additions, or deletions, preferably substitutions. Furthermore, typically no more than one, two, three, four, or five residues within each CDR are altered.

In one embodiment, the antibody or the antigen-binding fragment thereof provided in the present disclosure comprises a heavy chain variable region and a light chain variable region comprising: (a) an HCDR1 comprising the sequence of the present disclosure or an amino acid sequence comprising 1, 2, 3, 4 or 5 amino acid substitutions, deletions or additions; (b) an HCDR2 comprising the sequence of the present disclosure or an amino acid sequence comprising 1, 2, 3, 4 or 5 amino acid substitutions, deletions or additions; (c) an HCDR3 comprising the sequence of the present disclosure or an amino acid sequence comprising 1, 2, 3, 4 or 5 amino acid substitutions, deletions or additions; (d) an LCDR1 comprising the sequence of the present disclosure or an amino acid sequence comprising 1, 2, 3, 4 or 5 amino acid substitutions, deletions or additions; (e) an LCDR2 comprising the sequence of the present disclosure or an amino acid sequence comprising 1, 2, 3, 4 or 5 amino acid substitutions, deletions or additions; and (f) an LCDR3 comprising the sequence of the present disclosure or an amino acid sequence comprising 1, 2, 3, 4 or 5 amino acid substitutions, deletions or additions.

The antibody or the antigen-binding fragment thereof of the present disclosure comprises framework region modifications in the VH and/or the VL to improve antibody properties. In general, such framework region modifications may reduce the immunogenicity of the antibody. For example, one or more framework residues are "reverted" to the corresponding germline sequence. These residues can be identified by comparing the antibody framework sequence to the germline sequence of the resulting antibody.

Another type of framework region modification comprises mutating one or more residues of the framework regions or even one or more CDRs to remove T cell epitopes, thereby reducing the immunogenicity that an antibody may produce. This method is also known as "deimmunization" and is described in more detail in U.S. Patent Publication No. 20030153043.

Furthermore, the antibody or the antigen-binding fragment thereof of the present disclosure includes Fc modifications, which may be amino acid insertions, deletions, or substitutions and are typically used to alter one or more functional properties of the antibody, e.g., serum half-life, complement binding, Fc receptor binding, and/or antigen-dependent cellular cytotoxicity.

Furthermore, the antibody or the antigen-binding fragment thereof of the present disclosure may also be chemically modified (e.g., by linking one or more chemical functional groups), or modified to alter its glycosylation, so as to alter one or more functional properties of the antibody. In other embodiments, the Fc region is modified by PEGylation (e.g., by reacting the antibody or the fragment thereof with polyethylene glycol (PEG)).

In another embodiment, the glycosylation of the antibody or the antigen-binding fragment thereof of the present disclosure is altered. Such glycosylation modifications may be achieved, for example, by altering one or more glycosylation sites within the antibody sequence. For example, one or more amino acid replacements can be made to eliminate the glycosylation sites in framework regions of one or more variable regions and thus the glycosylation at those sites. Such deglycosylation may increase the affinity of the antibody for the antigen. See, e.g., U.S. Pat. Nos. 5,714,350 and 6,350,861.

The antibody or the antigen-binding fragment thereof provided in the present disclosure binds to CD40, thereby inhibiting CD40 activity. The "CD40 activity" includes, but is not limited to, the activation of B cells, e.g., the proliferation of B cells, the production of antibodies, isotype switching of antibodies, or differentiation into plasma cells; the activation of T cells, e.g., the proliferation or cytokine secretion of T cells; the activation of dendritic cells, e.g., the proliferation, differentiation, and maturation of dendritic cells; and the activation of macrophages. The CD40 activity may also be inhibited by interaction with other molecules. Furthermore, the "CD40 activity" also includes inhibiting the growth and/or proliferation of tumor cells, inducting the apoptosis of tumor cells, and the like.

In one embodiment, the anti-CD40 antibody or the antigen-binding fragment thereof provided in the present disclosure binds to CD40 and inhibits the activation of B cells. In one embodiment, the anti-CD40 antibody or the antigen-binding fragment thereof provided in the present disclosure binds to CD40 and inhibits the proliferation of B cells.

In one embodiment, the anti-CD40 antibody or the antigen-binding fragment thereof provided in the present disclosure binds to CD40 and inhibits the activation of T cells. In one embodiment, the anti-CD40 antibody or the antigen-binding fragment thereof provided in the present disclosure binds to CD40 and inhibits the proliferation of T cells and/or the production of cytokines. In one embodiment, the anti-CD40 antibody or the antigen-binding fragment thereof provided in the present disclosure binds to CD40 and inhibits the production of one or more cytokines selected from the group consisting of IL-2, IFNy, TNF, IL-1, IL-4, IL-5, IL-6, IL-12, IL-13, IL-17, and GM-CSF. In one embodiment, the anti-CD40 antibody or the antigen-binding fragment thereof provided in the present disclosure binds to CD40 and inhibits the production of cytokine IFNγ. In one embodiment, the anti-CD40 antibody or the antigen-binding fragment thereof provided in the present disclosure binds to CD40 and inhibits the production of cytokine IL-6.

Thus, in one aspect, the present disclosure provides a method for regulating immune response, comprising contacting T cells and antigen-presenting cells with the anti-CD40 antibody or the antigen-binding fragment thereof of the present disclosure. In one embodiment, the regulation of immune response by the anti-CD40 antibody or the antigen-binding fragment thereof provided in the present disclosure can be measured in a mixed lymphocyte reaction (MLR). In one embodiment, the anti-CD40 antibody or the antigen-binding fragment thereof provided in the present disclosure inhibits the production of cytokines by lymphocytes in the MLR. In another embodiment, the anti-CD40 antibody or the antigen-binding fragment thereof provided in the present disclosure inhibits the production of cytokine IFNγ in the MLR.

In one embodiment, the anti-CD40 antibody or the antigen-binding fragment thereof provided in the present disclosure binds to CD40 and inhibits the activation of dendritic cells. In one embodiment, the anti-CD40 antibody or the antigen-binding fragment thereof provided in the present disclosure binds to CD40 and inhibits the differentiation and maturation of dendritic cells.

In one embodiment, the anti-CD40 antibody or the antigen-binding fragment thereof provided in the present disclosure binds to CD40 and inhibits the apoptosis of tumor cells. In one embodiment, the anti-CD40 antibody or the antigen-binding fragment thereof provided in the present disclosure binds to CD40 and inhibits the apoptosis of Ramos cells.

The anti-CD40 antibody or the antigen-binding fragment thereof provided in the present disclosure shows no significant agonistic activity when binding to CD40. In one embodiment, the anti-CD40 antibody or the antigen-binding fragment thereof provided in the present disclosure shows no significant agonist activity for the apoptosis of tumor cells. In one embodiment, the anti-CD40 antibody or the antigen-binding fragment thereof provided in the present disclosure shows no significant agonist activity for the apoptosis of Ramos cells. In one embodiment, the anti-CD40 antibody or the antigen-binding fragment thereof provided in the present disclosure shows no significant agonist activity for the activation of B cells. In one embodiment, the anti-CD40 antibody or the antigen-binding fragment thereof provided in the present disclosure shows no significant agonist activity for the proliferation of B cells. In one embodiment, the anti-CD40 antibody or the antigen-binding fragment thereof provided in the present disclosure shows no significant agonist activity for the activation of dendritic cells. In one embodiment, the anti-CD40 antibody or the antigen-binding fragment thereof provided in the present disclosure shows no significant agonist activity for the differentiation and maturation of dendritic cells. A substance with "no significant agonist activity" shows the agonist activity detected in the assay that is not about 25% greater than, preferably not about 20%, 15%, 10%, 5%, 1%, 0.5% greater than or even not about 0.1% greater than the agonist activity induced by the native substance or the negative control, or the agonist activity detected in the assay that is at least 30%, 40%, 50%, 60%, 70%, 80%, 85%, 90%, 95% or 100% less than the agonist activity induced by the positive control. In one embodiment, a non-specific immunoglobulin that does not bind to CD40, for example, an IgG4 isotype control antibody, serves as a negative control. In one embodiment, an agonistic anti-CD40 antibody, for example, CP-870893, serves as a positive control.

The anti-CD40 antibody or the antigen-binding fragment thereof provided in the present disclosure shows good safety. The anti-CD40 antibody or the antigen-binding fragment thereof provided in the present disclosure has good therapeutic effects on Sjogren's syndrome.

In another aspect, the present disclosure provides a pharmaceutical composition comprising one or more of anti-CD40 antibodies or the antigen-binding fragments thereof of the present disclosure and a pharmaceutically acceptable carrier. As used herein, the "pharmaceutically acceptable carrier" includes any and all solvents, dispersion media, coatings, antibacterial agents, isotonizing agents, and combinations thereof that are physiologically compatible. The selection and use of suitable "pharmaceutically acceptable carriers" is taught in Gennaro, ed., Remington: The Science and Practice of Pharmacy, 20th Ed. (Lippincott Williams & Wilkins 2003). The antibody or the pharmaceutical composition may be administered by any suitable method or route. The routes of administration include, for example, intravenous, intramuscular, subcutaneous, parenteral, spinal, or epidermal administration (e.g., by injection or infusion). Depending on the route of administration, the active ingredient may be encapsulated in a material to protect it from acids and other natural conditions that may inactivate it. The phrase "parenteral administration" used herein refers to modes of administration other than enteral and topical administration that are typically performed by injection, including but not limited to, intravenous, intramuscular, intraarterial, intrathecal, intracapsular, intraorbital, intracardiac, intradermal, intraperitoneal, transtracheal, subcutaneous, subepidermal, intraarticular, subcapsular, subarachnoid, intraspinal, epidural, and intrasternal injection and infusion. Alternatively, the pharmaceutical composition may be administered through a non-parenteral route, for example, topical, epidermal or mucosal route, such as intranasal, oral, vaginal, rectal, sublingual or topical administration.

In the administration of the antibody, the dose may be in the range of about 0.0001 to 100 mg/kg of host body weight.

The anti-CD40 antibody or the antigen-binding portion thereof of the present disclosure has a variety of *in vitro* and *in vivo* applications relating to the diagnosis, treatment, and/or prevention of immune diseases. The "treatment" refers to a method for alleviating and/or stabilizing a symptom, disorder or condition, delaying and/or preventing the progression of a disease, and/or alleviating the severity of a disease. The anti-CD40 antibody or the antigen-binding portion thereof, the encoding nucleic acid, the pharmaceutical composition, the recombinant polypeptide, the fusion protein, the bispecific molecule, the immunoconjugate, the chimeric antigen receptor, or the gene vector of the present disclosure may be administered to a human subject to treat an immune disease in the subject.

In another aspect, the present disclosure provides a method for treating or preventing an immune disease in a subject, comprising: administering to the subject a therapeutically effective amount of the anti-CD40 antibody or the antigen-binding portion thereof, the encoding nucleic acid, the pharmaceutical composition, the recombinant polypeptide, the fusion protein, the bispecific molecule, the immunoconjugate, the chimeric antigen receptor, or the gene vector of the present disclosure. In some embodiments, the subject is a human.

The "immune disease" refers to any disease associated with the progression of immune responses in an individual, including cellular and/or humoral immune responses. In some specific embodiments, in the applications and method, the immune disease includes, but is not limited to, an inflammatory disease, an allergic reaction, an autoimmune disease, or a transplantation-related disease. The inflammatory disease refers to any disease, disorder or condition of excessive inflammatory symptoms, host tissue damage or loss of function due to excessive or uncontrolled inflammatory responses, including allergic inflammation of the skin, kidney, gastrointestinal and respiratory tracts, psoriasis, nephritis, epididymitis, inflammatory bowel disease, asthma, and the like. The autoimmune disease refers to any disease, disorder, or condition of the body that attacks and damages its tissues caused by the excessive activation of the immune system, for example, multiple sclerosis, arthritis, myasthenia gravis, psoriasis, scleroderma, autoimmune hepatitis, autoimmune parotitis, type I diabetes, and the like.

In some specific embodiments, in the applications and method, the immune disease includes, but is not limited to: an allergic reaction, Addison's disease, ankylosing spondylitis, spondyloarthritis, asthma, atherosclerosis, coronary heart disease, autoimmune hepatitis, autoimmune parotitis, type I diabetes, epididymitis, nephritis, Reiter's syndrome, thyroiditis, Graves' disease, Guillain-Barre syndrome (GBS), Hashimoto's disease, hemolytic anemia, idiopathic thrombocytopenia, systemic lupus erythematosus, subacute cutaneous lupus erythematosus, multiple sclerosis, myasthenia gravis, psoriasis, scleroderma, arthritis, sarcoidosis, Sjogren's syndrome, xerophthalmia, hidradenitis suppurativa, transplantation-related disease, vasculitis, and/or inflammatory bowel disease.

In some embodiments, examples of transplantation-related disease include, but are not limited to: graft immune rejection and graft-versus-host disease (GVHD).

In some embodiments, examples of inflammatory bowel disease include, but are not limited to: Crohn's disease and ulcerative colitis.

In some embodiments, examples of arthritis include, but are not limited to: rheumatoid arthritis, juvenile arthritis, and psoriatic arthritis.

In some embodiments, examples of nephritis include, but are not limited to: lupus nephritis.

In some embodiments, examples of psoriasis include, but are not limited to: vulgaris psoriasis, pustular psoriasis (such as palmoplantar psoriasis, generalized pustular psoriasis), erythrodermic psoriasis, and arthropathic psoriasis. In the applications and method described above, the anti-CD40 antibody or the antigen-binding portion thereof of the present disclosure may be administered alone or in combination with a second therapeutic agent. In some embodiments, the second therapeutic agent includes a non-steroidal anti-inflammatory drug (NSAID), a salicylate, hydroxychloroquine, sulfasalazine, a corticosteroid, a cytotoxic drug, or an immunosuppressive drug and/or antibody. The non-steroidal anti-inflammatory drug includes, but is not limited to ibuprofen, naproxen, diclofenac, indomethacin, ketorolac, meloxicam, piroxicam, tiaprofenic acid, and sulindac. The immunosuppressive drug and/or antibody include, but are not limited to cyclosporine, tacrolimus, rapamycin, mycophenolates mofetil, CTLA4-Ig fusions, anti-B lymphocyte stimulator antibodies, and anti-T cell antibodies (e.g., anti-CD-3 antibodies). The cytotoxic drug includes, but is not limited to methotrexate and cyclophosphamide. The combination of therapeutic agents discussed herein may be administered simultaneously as a single composition in a pharmaceutically acceptable carrier, or administered simultaneously as separate compositions, wherein each agent is in a pharmaceutically acceptable carrier. In another embodiment, the combination of therapeutic agents may be administered sequentially.

Furthermore, if the combination therapy is administered multiple times and the agents are administered sequentially, the sequence in the sequential administration at each time point may be reversed or maintained, and the sequential administration may be combined with simultaneous administration or any combination thereof.

Although the foregoing invention has been described in considerable detail by providing illustrations and examples for the purpose of clear understanding, it will be apparent to those of ordinary skills in the art in light of the teachings of the present disclosure that certain changes and modifications can be made to the present disclosure without departing from the spirit or scope of the appended claims. The present disclosure is further explained by the description of the following examples, which are not intended to be limiting. Those skilled in the art will readily identify a variety of noncritical parameters that may be changed or modified to produce substantially similar results. Unless otherwise indicated, the practice of the present disclosure will use conventional methods in protein chemistry, biochemistry, recombinant DNA technology, and pharmacology within the art.

The present disclosure further provides the following specific embodiments, which, however, are not intended to limit the scope of the present invention:
Embodiment 1. An isolated anti-CD40 antibody or an antigen-binding fragment thereof, comprising:
   (i) a heavy chain CDR1, a heavy chain CDR2, and a heavy chain CDR3, wherein
      (1) the heavy chain CDR1 comprises the heavy chain CDR1 amino acid sequence in SEQ ID NO: 9, the heavy chain CDR2 comprises the heavy chain CDR2 amino acid sequence in SEQ ID NO: 9, and the heavy chain CDR3 comprises the heavy chain CDR3 amino acid sequence in SEQ ID NO: 9;
      (2) the heavy chain CDR1 comprises the heavy chain CDR1 amino acid sequence in SEQ ID NO: 10, the heavy chain CDR2 comprises the heavy chain CDR2 amino acid sequence in SEQ ID NO: 10, and the heavy chain CDR3 comprises the heavy chain CDR3 amino acid sequence in SEQ ID NO: 10;
      (3) the heavy chain CDR1 comprises the heavy chain CDR1 amino acid sequence in SEQ ID NO: 11, the heavy chain CDR2 comprises the heavy chain CDR2 amino acid sequence in SEQ ID NO: 11, and the heavy chain CDR3 comprises the heavy chain CDR3 amino acid sequence in SEQ ID NO: 11;
      (4) the heavy chain CDR1 comprises the heavy chain CDR1 amino acid sequence in SEQ ID NO: 12, the heavy chain CDR2 comprises the heavy chain CDR2 amino acid sequence in SEQ ID NO: 12, and the heavy chain CDR3 comprises the heavy chain CDR3 amino acid sequence in SEQ ID NO: 12;
      (5) the heavy chain CDR1 comprises the heavy chain CDR1 amino acid sequence in SEQ ID NO: 13, the heavy chain CDR2 comprises the heavy chain CDR2 amino acid sequence in SEQ ID NO: 13, and the heavy chain CDR3 comprises the heavy chain CDR3 amino acid sequence in SEQ ID NO: 13;
      (6) the heavy chain CDR1 comprises the heavy chain CDR1 amino acid sequence in SEQ ID NO: 22, the heavy chain CDR2 comprises the heavy chain CDR2 amino acid sequence in SEQ ID NO: 22, and the heavy chain CDR3 comprises the heavy chain CDR3 amino acid sequence in SEQ ID NO: 22;
      (7) the heavy chain CDR1 comprises the heavy chain CDR1 amino acid sequence in SEQ ID NO: 30, the heavy chain CDR2 comprises the heavy chain CDR2 amino acid sequence in SEQ ID NO: 30, and the heavy chain CDR3 comprises the heavy chain CDR3 amino acid sequence in SEQ ID NO: 30;
      (8) the heavy chain CDR1 comprises the heavy chain CDR1 amino acid sequence in SEQ ID NO: 38, the heavy chain CDR2 comprises the heavy chain CDR2 amino acid sequence in SEQ ID NO: 38, and the heavy chain CDR3 comprises the heavy chain CDR3 amino acid sequence in SEQ ID NO: 38;
      (9) the heavy chain CDR1 comprises the heavy chain CDR1 amino acid sequence in SEQ ID NO: 44, the heavy chain CDR2 comprises the heavy chain CDR2 amino acid sequence in SEQ ID NO: 44, and the heavy chain CDR3 comprises the heavy chain CDR3 amino acid sequence in SEQ ID NO: 44; or
      (10) the heavy chain CDR1 comprises the heavy chain CDR1 amino acid sequence in SEQ ID NO: 52, the heavy chain CDR2 comprises the heavy chain CDR2 amino acid sequence in SEQ ID NO: 52, and the heavy chain CDR3 comprises the heavy chain CDR3 amino acid sequence in SEQ ID NO: 52; and/or
   (ii) a light chain CDR1, a light chain CDR2, and a light chain CDR3, wherein
      (1) the light chain CDR1 comprises the light chain CDR1 amino acid sequence in SEQ ID NO: 14, the light chain CDR2 comprises the light chain CDR2 amino acid sequence in SEQ ID NO: 14, and the light chain CDR3 comprises the light chain CDR3 amino acid sequence in SEQ ID NO: 14;
      (2) the light chain CDR1 comprises the light chain CDR1 amino acid sequence in SEQ ID NO: 15, the light chain CDR2 comprises the light chain CDR2 amino acid sequence in SEQ ID NO: 15, and the light chain CDR3 comprises the light chain CDR3 amino acid sequence in SEQ ID NO: 15;
      (3) the light chain CDR1 comprises the light chain CDR1 amino acid sequence in SEQ ID NO: 16, the light chain CDR2 comprises the light chain CDR2 amino acid sequence in SEQ ID NO: 16, and the light chain CDR3 comprises the light chain CDR3 amino acid sequence in SEQ ID NO: 16;
      (4) the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17;
      (5) the light chain CDR1 comprises the light chain CDR1 amino acid sequence in SEQ ID NO: 23, the light chain CDR2 comprises the light chain CDR2 amino acid sequence in SEQ ID NO: 23, and the light chain CDR3 comprises the light chain CDR3 amino acid sequence in SEQ ID NO: 23;
      (6) the light chain CDR1 comprises the light chain CDR1 amino acid sequence in SEQ ID NO: 31, the light chain CDR2 comprises the light chain CDR2 amino acid sequence in SEQ ID NO: 31, and the light chain CDR3 comprises the light chain CDR3 amino acid sequence in SEQ ID NO: 31;
      (7) the light chain CDR1 comprises the light chain CDR1 amino acid sequence in SEQ ID NO: 39, the light chain CDR2 comprises the light chain CDR2 amino acid sequence in SEQ ID NO: 39, and the light chain CDR3 comprises the light chain CDR3 amino acid sequence in SEQ ID NO: 39;
      (8) the light chain CDR1 comprises the light chain CDR1 amino acid sequence in SEQ ID NO: 45, the light chain CDR2 comprises the light chain CDR2 amino acid sequence in SEQ ID NO: 45, and the light chain CDR3 comprises the light chain CDR3 amino acid sequence in SEQ ID NO: 45; or
      (9) the light chain CDR1 comprises the light chain CDR1 amino acid sequence in SEQ ID NO: 53, the light chain CDR2 comprises the light chain CDR2 amino acid sequence in SEQ ID NO: 53, and the light chain CDR3 comprises the light chain CDR3 amino acid sequence in SEQ ID NO: 53.
Embodiment 2. The antibody or the antigen-binding fragment thereof according to embodiment 1, comprising:
   (i) the heavy chain CDR1, the heavy chain CDR2, and the heavy chain CDR3, wherein
      (1) the heavy chain CDR1, the heavy chain CDR2, and the heavy chain CDR3 comprise the amino acid sequences set forth in SEQ ID NOs: 1, 2, and 5, or amino acid sequences having at least 80% identity to the amino acid sequences set forth in SEQ ID NOs: 1, 2, and 5, respectively;
      (2) the heavy chain CDR1, the heavy chain CDR2, and the heavy chain CDR3 comprise the amino acid sequences set forth in SEQ ID NOs: 1, 3, and 5, or amino acid sequences having at least 80% identity to the amino acid sequences set forth in SEQ ID NOs: 1, 3, and 5, respectively;
      (3) the heavy chain CDR1, the heavy chain CDR2, and the heavy chain CDR3 comprise the amino acid sequences set forth in SEQ ID NOs: 1, 4, and 5, or amino acid sequences having at least 80% identity to the amino acid sequences set forth in SEQ ID NOs: 1, 4, and 5, respectively;
      (4) the heavy chain CDR1, the heavy chain CDR2, and the heavy chain CDR3 comprise the amino acid sequences set forth in SEQ ID NOs: 18, 19, and 20, or amino acid sequences having at least 80% identity to the amino acid sequences set forth in SEQ ID NOs: 18, 19, and 20, respectively;
      (5) the heavy chain CDR1, the heavy chain CDR2, and the heavy chain CDR3 comprise the amino acid sequences set forth in SEQ ID NOs: 24, 25, and 26, or amino acid sequences having at least 80% identity to the amino acid sequences set forth in SEQ ID NOs: 24, 25, and 26, respectively;
      (6) the heavy chain CDR1, the heavy chain CDR2, and the heavy chain CDR3 comprise the amino acid sequences set forth in SEQ ID NOs: 32, 33, and 34, or amino acid sequences having at least 80% identity to the amino acid sequences set forth in SEQ ID NOs: 32, 33, and 34, respectively;
      (7) the heavy chain CDR1, the heavy chain CDR2, and the heavy chain CDR3 comprise the amino acid sequences set forth in SEQ ID NOs: 40, 41, and 42, or amino acid sequences having at least 80% identity to the amino acid sequences set forth in SEQ ID NOs: 40, 41, and 42, respectively; or
      (8) the heavy chain CDR1, the heavy chain CDR2, and the heavy chain CDR3 comprise the amino acid sequences set forth in SEQ ID NOs: 46, 47, and 48, or amino acid sequences having at least 80% identity to the amino acid sequences set forth in SEQ ID NOs: 46, 47, and 48, respectively; and/or
   (ii) the light chain CDR1, the light chain CDR2, and the light chain CDR3, wherein
      (1) the light chain CDR1, the light chain CDR2, and the light chain CDR3 comprise the amino acid sequences set forth in SEQ ID NOs: 6, 7, and 8, or amino acid sequences having at least 80% identity to the amino acid sequences set forth in SEQ ID NOs: 6, 7, and 8, respectively;
      (2) the light chain CDR1, the light chain CDR2, and the light chain CDR3 comprise the amino acid sequences set forth in SEQ ID NOs: 21, 7, and 8, or amino acid sequences having at least 80% identity to the amino acid sequences set forth in SEQ ID NOs: 21, 7, and 8, respectively;
      (3) the light chain CDR1, the light chain CDR2, and the light chain CDR3 comprise the amino acid sequences set forth in SEQ ID NOs: 27, 28, and 29, or amino acid sequences having at least 80% identity to the amino acid sequences set forth in SEQ ID NOs: 27, 28, and 29, respectively;
      (4) the light chain CDR1, the light chain CDR2, and the light chain CDR3 comprise the amino acid sequences set forth in SEQ ID NOs: 35, 36, and 37, or amino acid sequences having at least 80% identity to the amino acid sequences set forth in SEQ ID NOs: 35, 36, and 37, respectively;
      (5) the light chain CDR1, the light chain CDR2, and the light chain CDR3 comprise the amino acid sequences set forth in SEQ ID NOs: 43, 36, and 37, or amino acid sequences having at least 80% identity to the amino acid sequences set forth in SEQ ID NOs: 43, 36, and 37, respectively; or
      (6) the light chain CDR1, the light chain CDR2, and the light chain CDR3 comprise the amino acid sequences set forth in SEQ ID NOs: 49, 50, and 51, or amino acid sequences having at least 80% identity to the amino acid sequences set forth in SEQ ID NOs: 49, 50, and 51, respectively.
Embodiment 3. The antibody or the antigen-binding fragment thereof according to embodiment 1, comprising:
   the heavy chain CDR1, the heavy chain CDR2, the heavy chain CDR3, the light chain CDR1, the light chain CDR2, and the light chain CDR3, wherein,
   (1) the heavy chain CDR1 comprises the heavy chain CDR1 amino acid sequence in SEQ ID NO: 9, the heavy chain CDR2 comprises the heavy chain CDR2 amino acid sequence in SEQ ID NO: 9, the heavy chain CDR3 comprises the heavy chain CDR3 amino acid sequence in SEQ ID NO: 9, the light chain CDR1 comprises the light chain CDR1 amino acid sequence in SEQ ID NO: 14, the light chain CDR2 comprises the light chain CDR2 amino acid sequence in SEQ ID NO: 14, and the light chain CDR3 comprises the light chain CDR3 amino acid sequence in SEQ ID NO: 14;
   (2) the heavy chain CDR1 comprises the heavy chain CDR1 amino acid sequence in SEQ ID NO: 10, the heavy chain CDR2 comprises the heavy chain CDR2 amino acid sequence in SEQ ID NO: 10, the heavy chain CDR3 comprises the heavy chain CDR3 amino acid sequence in SEQ ID NO: 10, the light chain CDR1 comprises the light chain CDR1 amino acid sequence in SEQ ID NO: 15, the light chain CDR2 comprises the light chain CDR2 amino acid sequence in SEQ ID NO: 15, and the light chain CDR3 comprises the light chain CDR3 amino acid sequence in SEQ ID NO: 15;
   (3) the heavy chain CDR1 comprises the heavy chain CDR1 amino acid sequence in SEQ ID NO: 11, the heavy chain CDR2 comprises the heavy chain CDR2 amino acid sequence in SEQ ID NO: 11, the heavy chain CDR3 comprises the heavy chain CDR3 amino acid sequence in SEQ ID NO: 11, the light chain CDR1 comprises the light chain CDR1 amino acid sequence in SEQ ID NO: 15, the light chain CDR2 comprises the light chain CDR2 amino acid sequence in SEQ ID NO: 15, and the light chain CDR3 comprises the light chain CDR3 amino acid sequence in SEQ ID NO: 15;
   (4) the heavy chain CDR1 comprises the heavy chain CDR1 amino acid sequence in SEQ ID NO: 10, the heavy chain CDR2 comprises the heavy chain CDR2 amino acid sequence in SEQ ID NO: 10, the heavy chain CDR3 comprises the heavy chain CDR3 amino acid sequence in SEQ ID NO: 10, the light chain CDR1 comprises the light chain CDR1 amino acid sequence in SEQ ID NO: 16, the light chain CDR2 comprises the light chain CDR2 amino acid sequence in SEQ ID NO: 16, and the light chain CDR3 comprises the light chain CDR3 amino acid sequence in SEQ ID NO: 16;
   (5) the heavy chain CDR1 comprises the heavy chain CDR1 amino acid sequence in SEQ ID NO: 11, the heavy chain CDR2 comprises the heavy chain CDR2 amino acid sequence in SEQ ID NO: 11, the heavy chain CDR3 comprises the heavy chain CDR3 amino acid sequence in SEQ ID NO: 11, the light chain CDR1 comprises the light chain CDR1 amino acid sequence in SEQ ID NO: 16, the light chain CDR2 comprises the light chain CDR2 amino acid sequence in SEQ ID NO: 16, and the light chain CDR3 comprises the light chain CDR3 amino acid sequence in SEQ ID NO: 16;
   (6) the heavy chain CDR1 comprises the heavy chain CDR1 amino acid sequence in SEQ ID NO: 10, the heavy chain CDR2 comprises the heavy chain CDR2 amino acid sequence in SEQ ID NO: 10, the heavy chain CDR3 comprises the heavy chain CDR3 amino acid sequence in SEQ ID NO: 10, the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17;
   (7) the heavy chain CDR1 comprises the heavy chain CDR1 amino acid sequence in SEQ ID NO: 12, the heavy chain CDR2 comprises the heavy chain CDR2 amino acid sequence in SEQ ID NO: 12, the heavy chain CDR3 comprises the heavy chain CDR3 amino acid sequence in SEQ ID NO: 12, the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17;
   (8) the heavy chain CDR1 comprises the heavy chain CDR1 amino acid sequence in SEQ ID NO: 13, the heavy chain CDR2 comprises the heavy chain CDR2 amino acid sequence in SEQ ID NO: 13, the heavy chain CDR3 comprises the heavy chain CDR3 amino acid sequence in SEQ ID NO: 13, the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17;
   (9) the heavy chain CDR1 comprises the heavy chain CDR1 amino acid sequence in SEQ ID NO: 22, the heavy chain CDR2 comprises the heavy chain CDR2 amino acid sequence in SEQ ID NO: 22, the heavy chain CDR3 comprises the heavy chain CDR3 amino acid sequence in SEQ ID NO: 22, the light chain CDR1 comprises the light chain CDR1 amino acid sequence in SEQ ID NO: 23, the light chain CDR2 comprises the light chain CDR2 amino acid sequence in SEQ ID NO: 23, and the light chain CDR3 comprises the light chain CDR3 amino acid sequence in SEQ ID NO: 23;
   (10) the heavy chain CDR1 comprises the heavy chain CDR1 amino acid sequence in SEQ ID NO: 30, the heavy chain CDR2 comprises the heavy chain CDR2 amino acid sequence in SEQ ID NO: 30, the heavy chain CDR3 comprises the heavy chain CDR3 amino acid sequence in SEQ ID NO: 30, the light chain CDR1 comprises the light chain CDR1 amino acid sequence in SEQ ID NO: 31, the light chain CDR2 comprises the light chain CDR2 amino acid sequence in SEQ ID NO: 31, and the light chain CDR3 comprises the light chain CDR3 amino acid sequence in SEQ ID NO: 31;
   (11) the heavy chain CDR1 comprises the heavy chain CDR1 amino acid sequence in SEQ ID NO: 38, the heavy chain CDR2 comprises the heavy chain CDR2 amino acid sequence in SEQ ID NO: 38, the heavy chain CDR3 comprises the heavy chain CDR3 amino acid sequence in SEQ ID NO: 38, the light chain CDR1 comprises the light chain CDR1 amino acid sequence in SEQ ID NO: 39, the light chain CDR2 comprises the light chain CDR2 amino acid sequence in SEQ ID NO: 39, and the light chain CDR3 comprises the light chain CDR3 amino acid sequence in SEQ ID NO: 39;
   (12) the heavy chain CDR1 comprises the heavy chain CDR1 amino acid sequence in SEQ ID NO: 44, the heavy chain CDR2 comprises the heavy chain CDR2 amino acid sequence in SEQ ID NO: 44, the heavy chain CDR3 comprises the heavy chain CDR3 amino acid sequence in SEQ ID NO: 44, the light chain CDR1 comprises the light chain CDR1 amino acid sequence in SEQ ID NO: 45, the light chain CDR2 comprises the light chain CDR2 amino acid sequence in SEQ ID NO: 45, and the light chain CDR3 comprises the light chain CDR3 amino acid sequence in SEQ ID NO: 45; or
   (13) the heavy chain CDR1 comprises the heavy chain CDR1 amino acid sequence in SEQ ID NO: 52, the heavy chain CDR2 comprises the heavy chain CDR2 amino acid sequence in SEQ ID NO: 52, the heavy chain CDR3 comprises the heavy chain CDR3 amino acid sequence in SEQ ID NO: 52, the light chain CDR1 comprises the light chain CDR1 amino acid sequence in SEQ ID NO: 53, the light chain CDR2 comprises the light chain CDR2 amino acid sequence in SEQ ID NO: 53, and the light chain CDR3 comprises the light chain CDR3 amino acid sequence in SEQ ID NO: 53.
Embodiment 4. The antibody or the antigen-binding fragment thereof according to any one of embodiments 1-3, comprising:
   the heavy chain CDR1, the heavy chain CDR2, the heavy chain CDR3, the light chain CDR1, the light chain CDR2, and the light chain CDR3, wherein,
   (1) the heavy chain CDR1, the heavy chain CDR2, the heavy chain CDR3, the light chain CDR1, the light chain CDR2, and the light chain CDR3 comprise the amino acid sequences set forth in SEQ ID NOs: 1, 2, 5, 6, 7, and 8, or amino acid sequences having at least 80% identity to the amino acid sequences set forth in SEQ ID NOs: 1, 2, 5, 6, 7, and 8, respectively;
   (2) the heavy chain CDR1, the heavy chain CDR2, the heavy chain CDR3, the light chain CDR1, the light chain CDR2, and the light chain CDR3 comprise the amino acid sequences set forth in SEQ ID NOs: 1, 3, 5, 6, 7, and 8, or amino acid sequences having at least 80% identity to the amino acid sequences set forth in SEQ ID NOs: 1, 3, 5, 6, 7, and 8, respectively;
   (3) the heavy chain CDR1, the heavy chain CDR2, the heavy chain CDR3, the light chain CDR1, the light chain CDR2, and the light chain CDR3 comprise the amino acid sequences set forth in SEQ ID NOs: 1, 4, 5, 6, 7, and 8, or amino acid sequences having at least 80% identity to the amino acid sequences set forth in SEQ ID NOs: 1, 4, 5, 6, 7, and 8, respectively;
   (4) the heavy chain CDR1, the heavy chain CDR2, the heavy chain CDR3, the light chain CDR1, the light chain CDR2, and the light chain CDR3 comprise the amino acid sequences set forth in SEQ ID NOs: 18, 19, 20, 21, 7, and 8, or amino acid sequences having at least 80% identity to the amino acid sequences set forth in SEQ ID NOs: 18, 19, 20, 21, 7, and 8, respectively;
   (5) the heavy chain CDR1, the heavy chain CDR2, the heavy chain CDR3, the light chain CDR1, the light chain CDR2, and the light chain CDR3 comprise the amino acid sequences set forth in SEQ ID NOs: 24, 25, 26, 27, 28, and 29, or amino acid sequences having at least 80% identity to the amino acid sequences set forth in SEQ ID NOs: 24, 25, 26, 27, 28, and 29, respectively;
   (6) the heavy chain CDR1, the heavy chain CDR2, the heavy chain CDR3, the light chain CDR1, the light chain CDR2, and the light chain CDR3 comprise the amino acid sequences set forth in SEQ ID NOs: 32, 33, 34, 35, 36, and 37, or amino acid sequences having at least 80% identity to the amino acid sequences set forth in SEQ ID NOs: 32, 33, 34, 35, 36, and 37, respectively;
   (7) the heavy chain CDR1, the heavy chain CDR2, the heavy chain CDR3, the light chain CDR1, the light chain CDR2, and the light chain CDR3 comprise the amino acid sequences set forth in SEQ ID NOs: 40, 41, 42, 43, 36, and 37, or amino acid sequences having at least 80% identity to the amino acid sequences set forth in SEQ ID NOs: 40, 41, 42, 43, 36, and 37, respectively; or
   (8) the heavy chain CDR1, the heavy chain CDR2, the heavy chain CDR3, the light chain CDR1, the light chain CDR2, and the light chain CDR3 comprise the amino acid sequences set forth in SEQ ID NOs: 46, 47, 48, 49, 50, and 51, or amino acid sequences having at least 80% identity to the amino acid sequences set forth in SEQ ID NOs: 46, 47, 48, 49, 50, and 51, respectively.
Embodiment 5. The antibody or the antigen-binding fragment thereof according to any one of embodiments 1-4, comprising:
   (i) a heavy chain variable region comprising the amino acid sequence set forth in SEQ ID NO: 9, 10, 11, 12, 13, 22, 30, 38, 44, or 52, or an amino acid sequence having at least 80% identity to the amino acid sequence set forth in SEQ ID NO: 9, 10, 11, 12, 13, 22, 30, 38, 44, or 52; and/or
   (ii) a light chain variable region comprising the amino acid sequence set forth in SEQ ID NO: 14, 15, 16, 17, 23, 31, 39, 45, or 53, or an amino acid sequence having at least 80% identity to the amino acid sequence set forth in SEQ ID NO: 14, 15, 16, 17, 23, 31, 39, 45, or 53.
Embodiment 6. The antibody or the antigen-binding fragment thereof according to any one of embodiments 1-5, comprising the heavy chain variable region and the light chain variable region, wherein,
   (1) the heavy chain variable region and the light chain variable region comprise the amino acid sequences set forth in SEQ ID NOs: 9 and 14, or amino acid sequences having at least 80% identity to the amino acid sequences set forth in SEQ ID NOs: 9 and 14, respectively;
   (2) the heavy chain variable region and the light chain variable region comprise the amino acid sequences set forth in SEQ ID NOs: 10 and 15, or amino acid sequences having at least 80% identity to the amino acid sequences set forth in SEQ ID NOs: 10 and 15, respectively;
   (3) the heavy chain variable region and the light chain variable region comprise the amino acid sequences set forth in SEQ ID NOs: 11 and 15, or amino acid sequences having at least 80% identity to the amino acid sequences set forth in SEQ ID NOs: 11 and 15, respectively;
   (4) the heavy chain variable region and the light chain variable region comprise the amino acid sequences set forth in SEQ ID NOs: 10 and 16, or amino acid sequences having at least 80% identity to the amino acid sequences set forth in SEQ ID NOs: 10 and 16, respectively;
   (5) the heavy chain variable region and the light chain variable region comprise the amino acid sequences set forth in SEQ ID NOs: 11 and 16, or amino acid sequences having at least 80% identity to the amino acid sequences set forth in SEQ ID NOs: 11 and 16, respectively;
   (6) the heavy chain variable region and the light chain variable region comprise the amino acid sequences set forth in SEQ ID NOs: 10 and 17, or amino acid sequences having at least 80% identity to the amino acid sequences set forth in SEQ ID NOs: 10 and 17, respectively;
   (7) the heavy chain variable region and the light chain variable region comprise the amino acid sequences set forth in SEQ ID NOs: 12 and 17, or amino acid sequences having at least 80% identity to the amino acid sequences set forth in SEQ ID NOs: 12 and 17, respectively;
   (8) the heavy chain variable region and the light chain variable region comprise the amino acid sequences set forth in SEQ ID NOs: 13 and 17, or amino acid sequences having at least 80% identity to the amino acid sequences set forth in SEQ ID NOs: 13 and 17, respectively;
   (9) the heavy chain variable region and the light chain variable region comprise the amino acid sequences set forth in SEQ ID NOs: 22 and 23, or amino acid sequences having at least 80% identity to the amino acid sequences set forth in SEQ ID NOs: 22 and 23, respectively;
   (10) the heavy chain variable region and the light chain variable region comprise the amino acid sequences set forth in SEQ ID NOs: 30 and 31, or amino acid sequences having at least 80% identity to the amino acid sequences set forth in SEQ ID NOs: 30 and 31, respectively;
   (11) the heavy chain variable region and the light chain variable region comprise the amino acid sequences set forth in SEQ ID NOs: 38 and 39, or amino acid sequences having at least 80% identity to the amino acid sequences set forth in SEQ ID NOs: 38 and 39, respectively;
   (12) the heavy chain variable region and the light chain variable region comprise the amino acid sequences set forth in SEQ ID NOs: 44 and 45, or amino acid sequences having at least 80% identity to the amino acid sequences set forth in SEQ ID NOs: 44 and 45, respectively; or
   (13) the heavy chain variable region and the light chain variable region comprise the amino acid sequences set forth in SEQ ID NOs: 52 and 53, or amino acid sequences having at least 80% identity to the amino acid sequences set forth in SEQ ID NOs: 52 and 53, respectively.
Embodiment 7. The antibody or the antigen-binding fragment thereof according to any one of embodiments 1-6, further comprising a heavy chain constant region and a light chain constant region, wherein the heavy chain constant region comprises the amino acid sequence set forth in SEQ ID NO: 54, 55, or 56, or an amino acid sequence comprising 1, 2, 3, 4, or 5 amino acid substitutions, deletions, and additions compared to the amino acid sequence set forth in SEQ ID NO: 54, 55, or 56, and the light chain constant region comprises the amino acid sequence set forth in SEQ ID NO: 57, or an amino acid sequence comprising 1, 2, 3, 4, or 5 amino acid substitutions, deletions, and additions compared to the amino acid sequence set forth in SEQ ID NO: 57.
Embodiment 8. The antibody or the antigen-binding fragment thereof according to any one of embodiments 1-7, wherein the antibody or the antigen-binding fragment thereof: (a) binds to human CD40; (b) binds to monkey CD40; (c) blocks the interaction between CD40 and CD40L; and/or (d) inhibits CD40 activity.
Embodiment 9. The antibody or the antigen-binding fragment thereof according to any one of embodiments 1-8, wherein the antibody or the antigen-binding fragment thereof is chimeric or humanized.
Embodiment 10. The antibody or the antigen-binding fragment thereof according to any one of embodiments 1-9, wherein the antibody or the antigen-binding fragment thereof is of the IgG1, IgG2, or IgG4 isotype.
Embodiment 11. The antibody or the antigen-binding fragment thereof according to any one of embodiments 1-10, wherein the antibody or the antigen-binding fragment thereof is selected from the group consisting of a monoclonal antibody, a monospecific antibody, a bispecific antibody, a trispecific antibody, a multispecific antibody, an Fab fragment, an F(ab')₂ fragment, an Fd fragment, an Fv fragment, a dAb fragment, an isolated CDR region, a single-chain Fv molecule, or a combination thereof.
Embodiment 12. An isolated antibody or an antigen-binding fragment thereof, wherein the antibody or the antigen-binding fragment thereof binds to the same epitope as the antibody or the antigen-binding fragment thereof according to any one of embodiments 1-11.
Embodiment 13. An isolated antibody or an antigen-binding fragment thereof, wherein the antibody or the antigen-binding fragment thereof competes with the antibody or the antigen-binding fragment thereof according to any one of embodiments 1-11 for binding to CD40.
Embodiment 14. An isolated nucleic acid molecule encoding the antibody or the antigen-binding fragment thereof according to any one of embodiments 1-11.
Embodiment 15. An expression vector, comprising the nucleic acid molecule according to embodiment 14.
Embodiment 16. A host cell, comprising the nucleic acid molecule according to embodiment 14 or the expression vector according to embodiment 15.
Embodiment 17. A recombinant polypeptide or fusion protein, comprising the antibody or the antigen-binding fragment thereof according to any one of embodiments 1-11.
Embodiment 18. A bispecific molecule, comprising the antibody or the antigen-binding fragment thereof according to any one of embodiments 1-11.
Embodiment 19. An immunoconjugate, comprising the antibody or the antigen-binding fragment thereof according to any one of embodiments 1-11 and a therapeutic agent, e.g., a cytotoxic agent or an imaging agent, linked thereto.
Embodiment 20. A pharmaceutical composition, comprising the antibody or the antigen-binding fragment thereof according to any one of embodiments 1-11, and one or more pharmaceutically acceptable carriers.
Embodiment 21. A method for treating or preventing an immune disease in a subject in need, comprising: administering to the subject a therapeutically effective amount of the antibody or the antigen-binding fragment thereof according to any one of embodiments 1-11, or the pharmaceutical composition according to embodiment 20.
Embodiment 22. The method according to embodiment 21, wherein the immune disease includes, but is not limited to, an inflammatory disease, an allergic reaction, an autoimmune disease, or a transplantation-related disease.
Embodiment 23. The method according to embodiment 21, wherein the immune disease includes, but is not limited to, an allergic reaction, Addison's disease, ankylosing spondylitis, spondyloarthritis, asthma, atherosclerosis, coronary heart disease, autoimmune hepatitis, autoimmune parotitis, type I diabetes, epididymitis, nephritis, Reiter's syndrome, thyroiditis, Graves' disease, Guillain-Barre syndrome, Hashimoto's disease, hemolytic anemia, idiopathic thrombocytopenia, systemic lupus erythematosus, subacute cutaneous lupus erythematosus, multiple sclerosis, myasthenia gravis, psoriasis, scleroderma, arthritis, sarcoidosis, Sjogren's syndrome, xerophthalmia, hidradenitis suppurativa, transplantation-related disease, vasculitis, and/or inflammatory bowel disease.
Embodiment 24. The method according to any one of embodiments 21-23, wherein the method further comprises administering a therapeutically effective amount of a second therapeutic agent.
Embodiment 25. The method according to embodiment 24, wherein the second therapeutic agent includes a non-steroidal anti-inflammatory drug, a salicylate, hydroxychloroquine, sulfasalazine, a corticosteroid, a cytotoxic drug, or an immunosuppressive drug and/or antibody.

### Examples

Although the present disclosure has been described in detail herein above through general explanations and specific embodiments, it will be apparent to those skilled in the art that modifications or improvements can be made based on the present disclosure. Accordingly, such modifications and improvements made without departing from the spirit of the present disclosure all fall within the protection scope of the present disclosure.

### Example 1. Preparation of mouse anti-CD40 monoclonal antibody

### Preparation of recombinant proteins

cDNAs encoding the human CD40 recombinant protein (hCD40-mFc, SEQ ID NO: 64) and monkey CD40 recombinant protein (cynoCD40-mFc, SEQ ID NO: 65) containing a mouse antibody heavy chain Fc were obtained by gene synthesis, and subcloned into expression vector pcDNA3.1(+) separately. The vector was transfected into CHO cells for transient expression. Cell culture supernatants were collected and recombinant proteins hCD40-mFc and cynoCD40-mFc were purified using a MabSelect Sure LX purification column (GE).

### Construction of stable cell lines

cDNAs encoding the full-length hCD40 (SEQ ID NO: 62) and cynoCD40 (SEQ ID NO: 63) were obtained by gene synthesis separately, and then subcloned into expression vector pcDNA3.1(+) to give pcDNA3.1-hCD40 and pcDNA3.1-cynoCD40 recombinant plasmids. Then, according to the instructions for Lipofectamin3000 transfection reagent (Thermo, Cat. No.: L3000015), the two recombinant plasmids were transfected into CHO-K1 cells separately to give stable cell lines CHO-K1-hCD40 and CHO-K1-cynoCD40.

cDNAs encoding the full-length hCD40L (SEQ ID NO: 66) and cynoCD40L (SEQ ID NO: 67) were obtained by gene synthesis separately, and then subcloned into expression vector pcDNA3.1(+) to give pcDNA3.1 -hCD40L and pcDNA3.1-cynoCD40L recombinant plasmids. Then, according to the instructions for Lipofectamin3000 transfection reagent (Thermo, Cat. No.: L3000015), the two recombinant plasmids were transfected into CHO-K1 cells separately to give stable cell lines CHO-K1-hCD40L and CHO-K1-cynoCD40L, respectively.

According to the instructions for Lipofectamin3000 transfection reagent (Thermo, Cat. No.: L3000015), plasmid pGL4.32 [luc2P/NF-κB-RE/Hygro] Vector (Promega, Cat. No.: E8491) was transfected into HEK293T cells to give a stable cell line 293T-NFκB. The encoding sequence of full-length hCD40 (SEQ ID NO: 62) was inserted into the pcDNA3.1/Zeo (+) vector at BamHI and XhoI sites to give recombinant plasmid pcDNA3.1/Zeo(+)-hCD40. According to the instructions for Lipofectamin3000 transfection reagent (Thermo, Cat. No.: L3000015), recombinant plasmid pcDNA3.1/Zeo(+)-hCD40 was transfected into 293T-NFκB cells to give a stable cell line 293T-hCD40-NFκB.

### Immunization of mice

The pured recombinant protein hCD40-mFc (SEQ ID NO: 64) was used as the antigen. hCD40-mFc was thoroughly mixed with complete Freund's adjuvant (Sigma, Cat. No.: F5881-10×10mL) in a volume ratio of 1:1 and the mixture was emulsified. The mice were immunized by subcutaneous injection. 2-4 weeks after the primary immunization, hCD40-mFc or cynoCD40-mFc and Alum adjuvant (Thermo, Cat. No.: 77161) were thoroughly mixed in a volume ratio of 1:1 and the mixture was emulsified. Booster immunization was performed every 2 weeks for 6 weeks by subcutaneous injection and intramuscular injection alternately. After the completion of the immunization, serum was collected from each mouse for the serum titer assay of specific anti-CD40 antibodies. Mice with a higher serum titer were selected for subsequent spleen cell fusion.

### Preparation and screening of hybridomas

The mice were subjected to pre-fusion intensive immunization by intraperitoneal injection of hCD40-mFc 3-4 days before the fusion. On the day of fusion, the spleen was collected aseptically, subjected to grinding and red blood cell lysis, and then resuspended in an electrofusion buffer (BTX, Cat. No.: 47-0001) to give a single-cell suspension. The suspension was mixed with myeloma cells SP2/0 in the logarithmic growth phase in a cell number ratio of 2:1. The cell fusion was conducted on an electrofusion system (BTX). The fused cells were mixed well with a hybridoma medium (Gibco, Cat. No.: 12045-076) containing 1× HAT, and cultured at 37 °C/5% CO₂ for 7 days to give hybridoma cells. Hybridoma cell culture supernatant was collected for screening of mouse antibodies.

Hybridoma cells with binding activity for hCD40 as detected by ELISA and FACS were selected for expansion culture, and subcloned by limiting dilution after the expansion to a certain number of cells. The subcloned hybridoma cells were then incubated at 37 °C/5% CO₂ for 7 days and the subcloned hybridoma cell culture supernatant was collected for further screening of mouse antibodies.

### Expression and purification of mouse antibodies

The screened hybridoma cells were cultured for 10 days. The hybridoma cell culture supernatant was collected by centrifugation, and loaded onto a protein G column (Genscript, Cat. No.: L00209-10). The protein G column was rinsed with a PBS buffer, and then antibodies bound to the protein G column were eluted with 100 mM glycine (pH 2.8). The eluate was immediately neutralized with 1 M Tris-HC. Subsequently, the mouse antibodies were transferred into a PBS buffer by ultrafiltration.

### Example 2. Characterization of mouse anti-CD40 antibodies

### Determination of binding activity of anti-CD40 antibodies by ELISA

An hCD40-His protein (Aero, Cat. No.: CD0-H5228) was diluted with a PBS buffer (pH 7.4) to a concentration of 0.1 µg/mL, immobilized on a 96-well plate at 100 µL/well, and incubated overnight at 4 °C. The 96-well plate was washed with PBST (PBS containing 0.5% of Tween-20) and then blocked at room temperature for 2 h by adding a blocking buffer (PBST containing 1% of BSA) at 200 µL/well. The blocking buffer was discarded. The hybridoma cell culture supernatant was added at 100 µL/well and the mixture was incubated at room temperature for 2 h. After the 96-well plate was washed with PBST, an HRP-conjugated goat anti-mouse IgG (H+L) antibody (Jackson Immuno, Cat. No.: 115-035-062) diluted in a ratio volume of 1:10000 was added, and the mixture was incubated at room temperature for 1 h. After the 96-well plate was washed with PBST, a TMB solution was added at 100 µL/well, and the mixture was incubated at room temperature in the dark for 5 min. The reaction was stopped by adding 0.5 M H₂SO₄. The OD450 value was read on a Bio-rad iMark microplate reader.

### Detection of binding activity of anti-CD40 antibodies by FACS

A CHO-K1-hCD40 cell suspension at a cell concentration of 5×10⁵ cells/mL was added into a 96-well U-bottom plate at 100 µL/well, before the hybridoma cell culture supernatant was added at 100 µL/well. The mixture was mixed well, and then incubated at 4 °C for 1 h. The cells were washed with PBS containing 2% of FBS, and then a PE-conjugated goat anti-mouse IgG (H+L) antibody (Abeam, Cat. No.: ab97041) diluted at a volume ratio of 1:500 was added, and the mixture was incubated at 4 °C for 1 h. The cells were washed with PBS containing 2% of FBS and then resuspended in PBS containing 2% of FBS. The binding of anti-CD40 antibodies to CHO-K1-hCD40 cells was analyzed by detecting the mean fluorescence intensity (MFI) of staining on a flow cytometer (BD Accuri C6).

### Activity of anti-CD40 antibodies to block binding of 293T hCD40-NFxB cells to CD40L

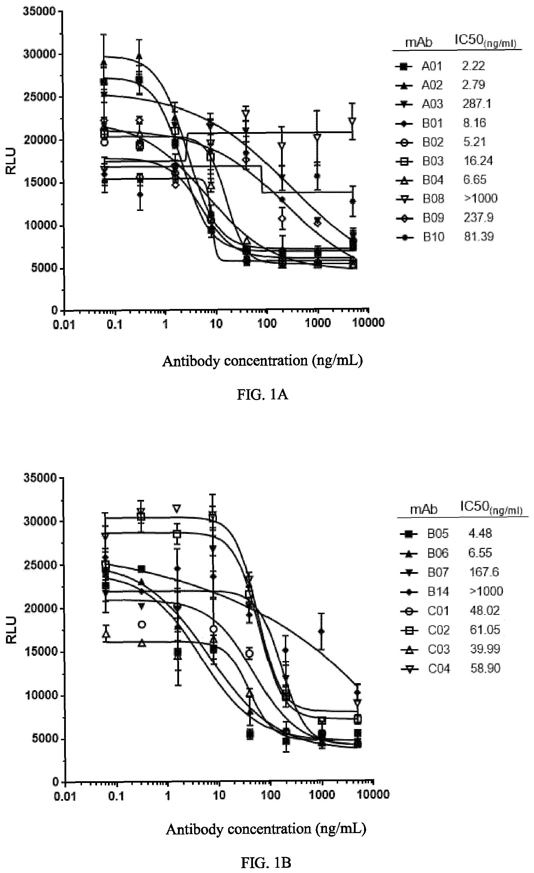

Upon the interaction of hCD40 on 293T-hCD40-NFκB cells with CD40L, the expression of fluorescence signals was up-regulated. Specifically, 293T-hCD40-NFκB cells in the logarithmic growth phase were resuspended in a DMEM medium containing 2% of FBS, and the cell concentration was adjusted to 5×10⁵ cells/mL. The cells were added into a white 384-well plate at 10 µL/well, and the hybridoma cell culture supernatant or serially 5-fold diluted pured mouse anti-CD40 antibodies in a final concentration range of 0.064 ng/mL-5000 ng/mL were added. The mixture was incubated at 37 °C for 20 min while shaking. Subsequently, 10 µL of a human CD40L protein (Aero, Cat. No.: CDL-H52Db) at a final concentration of 10 µg/mL was added into each well, and the mixture was incubated at 37 °C for 20 min while shaking. The 384-well plate was let stand for incubation at 37 °C/5% CO₂ for 5-6 h. After the incubation, a detection reagent was added according to the instructions for a Luciferase Assay System (Vazyme, Cat. No.: DD1201-03), and the fluorescence signals were read on a microplate reader (Thermo Varioskan Flash). The blocking activity of anti-CD40 antibodies on CD40L-mediated up-regulation of fluorescence signals in 293T-hCD40-NFκB cells was analyzed by relative light unit (RLU), and the IC₅₀ value was calculated by Graphpad Prism. As shown in FIGs. 1A and 1B, a plurality of pured mouse anti-CD40 antibodies had the activity to block the binding of 293T-hCD40-NFκB cells to CD40L.

### Inhibitory activity of anti-CD40 antibodies against Ramos cell apoptosis

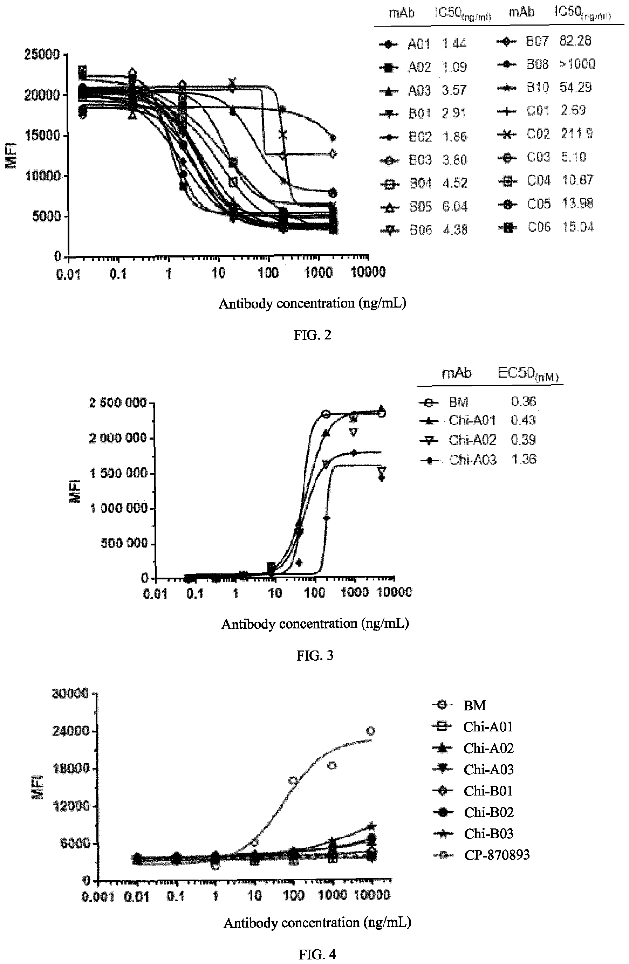

The MAPK activating pathway mediated by CD40/CD40L is a potential mechanism for inhibiting tumor cell proliferation and inducing tumor cell apoptosis. Ramos (Burkitt lymphoma cell) cell, a human B lymphoma cell endogenously expressing CD40, was used as the model to detect the inhibitory activity of anti-CD40 antibodies on Ramos cell apoptosis. Specifically, a Ramos cell suspension at a cell concentration of 1×10⁶ cells/mL was added into a 96-well U-bottom plate at 50 µL/well, before serially 10-fold diluted pured mouse anti-CD40 antibodies in a final concentration range of 0.02 ng/mL-2000 ng/mL were added. A human CD40L protein (Aero, Cat. No.: CDL-H52Db) at a final concentration of 2 µg/mL and a recombinant human IL-4 (Aero, Cat. No.: IL4-H4218) at a final concentration of 60 ng/mL were added. The mixture was mixed well, and incubated overnight at 37 °C/5% CO₂. The cells were washed with PBS containing 2% of FBS, before a PE-conjugated mouse anti-human CD95 antibody (Biolegend, Cat. No.: 305608) was added. The mixture was mixed well, and incubated at 4 °C for 30 min. The cells were washed with PBS containing 2% of FBS and then resuspended. Fluorescence signals were detected on a flow cytometer (Sartorius IQue3). The expression of the tumor cell apoptosis molecule CD95 was analyzed by the mean fluorescence intensity (MFI) of staining, and the IC₅₀ value was calculated by Graphpad Prism. As shown in FIG. 2, a plurality of pured mouse anti-CD40 antibodies had the activity to inhibit Ramos cell apoptosis induced by CD40L and IL-4.

### Example 3. Preparation of chimeric anti-CD40 antibodies

### Construction of chimeric antibodies

The total RNA of the hybridoma cells screened in Example 2 (e.g., A01, A02, A03, B01, B02, B03, etc.) was isolated according to the instructions for an RNA extraction kit (Takara, Cat. No.: 9767), and a first-strand cDNA was synthesized by a reverse transcription kit (Thermo, Cat. No.: K1652). The first-strand cDNA was used as the template, and mixed with a mouse IgG primer and a Kappa primer separately. The cDNA was then cloned and sequenced by polymerase chain reaction (PCR) to give the sequences of the variable regions of the mouse IgG antibodies.

The DNA sequences of the VH and VL of the mouse antibodies were linked to DNA sequences of the heavy chain constant region of a human IgG4 (SEQ ID NO: 54) and a kappa light chain constant region (SEQ ID NO: 57), respectively, by chemical synthesis. The sequences were then cloned into a pcDNA3.1(+) vector to construct recombinant human-mouse chimeric antibodies. These chimeric antibodies were designated as Chi-A01, Chi-A02, Chi-A03, Chi-B01, Chi-B02, and Chi-B03, respectively.

### Construction of reference antibody

Iscalimab (CFZ533) was selected as the reference antibody. DNA sequences of the heavy chain and light chain of the antibody were obtained by chemical synthesis based on the amino acid sequences described in U.S. Pat. No. US9221913B2. The sequences were cloned into a pcDNA3.1(+) vector to construct the reference antibody (the amino acid sequences of the heavy chain and light chain are set forth in SEQ ID NOs: 58 and 59 of the present disclosure with the heavy chain variable region and light chain variable region underlined), which was also referred to as BM (benchmark) herein.

### Expression and purification of chimeric antibodies and reference antibody

The chimeric antibodies and the reference antibody were transiently transfected into cells using an expiCHO system (Gibco, Cat. No.: A29129) according to the instructions for its transfection kit. The transfected cells were cultured at 37 °C/8% CO₂ for 6 days while shaking. The cell culture supernatant was collected by centrifugation, and loaded onto a protein A column (G.E. Healthcare, Cat. No.: 17-5474). The protein A column was washed with 10 column volumes of PBS buffer. The antibodies bound to the protein A column were then eluted with an acetic acid buffer (300 mM acetic acid, pH 3.6), and the eluate was immediately neutralized with 1 M Tris-HCl. Subsequently, the chimeric antibodies were transferred into the PBS buffer by ultrafiltration.

### Example 4. Characterization of chimeric anti-CD40 antibodies

### Detection of binding activity of anti-CD40 antibodies by FACS

The procedures are detailed in Example 2, except for some minor changes as follows: the hybridoma cell culture supernatant was replaced with serially 5-fold diluted chimeric anti-CD40 antibodies and BM in a final concentration range of 0.064 ng/mL-5000 ng/mL; the PE-conjugated goat anti-mouse IgG (H+L) antibody was replaced with a PE-conjugated donkey anti-human IgG (H+L) (Abeam, Cat. No.: ab102439).

FIG. 3 shows the binding activity of chimeric antibodies to CHO-K1-hCD40 cells, wherein the binding EC₅₀ values of Chi-A01 and Chi-A02 to CHO-K1-hCD40 cells are comparable to that of BM.

### Inhibitory activity of anti-CD40 antibodies against Ramos cell apoptosis

The procedures are detailed in Example 2, except for some minor changes as follows: the serially 10-fold diluted pured mouse antibodies in a final concentration range of 0.02 ng/mL-2000 ng/mL were replaced with serially 5-fold diluted chimeric anti-CD40 antibodies, BM, and an IgG4 isotype control antibody in a final concentration range of 0.064 ng/mL-1000 ng/mL.

Tables 2 and 3 show the inhibitory activity of chimeric antibodies on Ramos cell apoptosis induced by CD40L and IL-4, wherein the concentration of Chi-A01 and Chi-A02 inhibiting 50% of the tumor cell apoptosis induced by CD40L and IL-4 (IC₅₀) was about 5 pM, while the IC₅₀ of BM was 18.32 pM (about 3-4 times that of Chi-A01 and Chi-A02), indicating that Chi-A01 and Chi-A02 had superior inhibitory activity on Ramos cell apoptosis to BM.

Inhibition % = (MFI of Ramos cell apoptosis induced by co-incubation with CD40L and IL-4 - MFI of Ramos cell apoptosis induced by co-incubation with CD40L, IL-4, and anti-CD40 antibodies)/(MFI of Ramos cell apoptosis induced by co-incubation with CD40L and IL-4 - MFI of natural Ramos cell apoptosis after incubation in culture medium only) × 100%. According to the formula, the average maximum inhibition rates of Chi-A01 and Chi-A02 are comparable to that of BM.

**Table 2. Inhibitory activity of chimeric antibodies Chi-A01, Chi-A02, and Chi-A03 against Ramos cell apoptosis**

| Concentration, ng/mL | BM | | Chi-A01 | | Chi-A02 | | Chi-A03 | | IgG4 isotype control | |
|---|---|---|---|---|---|---|---|---|---|---|
| | IC₅₀ | Inhibition, % | IC₅₀ | Inhibition, % | IC₅₀ | Inhibition, % | IC₅₀ | Inhibition, % | IC₅₀ | Inhibition, % |
| 1000 | 18.32 pM | 98 | 5.08 pM | 95 | 4.99 pM | 94 | 134 pM | 98 | NA | -3 |
| 200 | | 99 | | 96 | | 97 | | 90 | | -6 |
| 40 | | 97 | | 95 | | 97 | | 66 | | -20 |
| 8 | | 85 | | 96 | | 98 | | 18 | | 0 |
| 1.6 | | 41 | | 82 | | 85 | | -10 | | -10 |
| 0.32 | | 12 | | 17 | | 20 | | -9 | | -9 |
| 0.064 | | 4 | | 0 | | 5 | | -8 | | -12 |

**Table 3. Inhibitory activity of chimeric antibodies Chi-B01, Chi-B02, and Chi-B03 against Ramos cell apoptosis**

| Concentration, ng/mL | BM | | Chi-A01 | | Chi-B01 | | Chi-B02 | | Chi-B03 | |
|---|---|---|---|---|---|---|---|---|---|---|
| | IC₅₀ | Inhibition, % | IC₅₀ | Inhibition, % | IC₅₀ | Inhibition, % | IC₅₀ | Inhibition, % | IC₅₀ | Inhibition, % |
| 1000 | 24.71 pM | 101 | 4.13 pM | 100 | 30.14 pM | 93 | 27.49 pM | 75 | 11.87 pM | 70 |
| 200 | | 100 | | 101 | | 96 | | 88 | | 84 |
| 40 | | 98 | | 99 | | 93 | | 92 | | 90 |
| 8 | | 72 | | 101 | | 55 | | 59 | | 90 |
| 1.6 | | 5 | | 79 | | 2 | | 5 | | 31 |
| 0.32 | | -10 | | 39 | | -12 | | -5 | | 16 |
| 0.064 | | -24 | | 10 | | -33 | | -16 | | -8 |
| 0.0128 | | -26 | | 11 | | -24 | | -18 | | -10 |

| | | | | | | | | | | |
|---|---|---|---|---|---|---|---|---|---|---|
| Note: NA denotes no IC₅₀ available. | | | | | | | | | | |

### Agonistic activity of anti-CD40 antibodies on Ramos cell apoptosis

An agonistic anti-CD40 antibody CP-870893 (prepared in-house, see SEQ ID NOs:60 and 61 of the present disclosure for its heavy chain and light chain sequences) is known to produce a stimulation signal upon binding to Ramos cells, and such signal induces Ramos cell apoptosis. The agonistic activity of chimeric anti-CD40 antibodies was detected by Ramos cells. Specifically, a Ramos cell suspension with a cell concentration of 5×10⁵ cells/mL was added into a 96-well U-bottom plate at 100 µL/well, and serially 10-fold diluted anti-CD40 antibodies in a final concentration range of 0.01 ng/mL-10000 ng/mL was added. The mixture was mixed well, and then incubated overnight at 37 °C/5% CO₂. The cells were washed with PBS containing 2% of FBS, before a PE-conjugated mouse anti-human CD95 antibody (Biolegend, Cat. No.: 305608) was added. The mixture was mixed well, and incubated at 4 °C for 30 min. The cells were washed with PBS containing 2% of FBS and then resuspended. Fluorescence signals were detected on a flow cytometer (Sartorius IQue3). The expression of the tumor cell apoptosis molecule CD95 was analyzed by the mean fluorescence intensity (MFI) of staining. The results are shown in FIG. 4. Chimeric antibodies Chi-A02, Chi-B02, and Chi-B03 exhibited weak agonistic activity on Ramos cell apoptosis, while chimeric antibodies Chi-A01, Chi-A03, Chi-B01, and BM exhibited no agonistic activity on Ramos cell apoptosis.

### Example 5. Design and preparation of humanized anti-CD40 antibodies

### Design of humanized antibodies

Human germline antibody sequence with the highest sequence homology to the heavy and the light chain variable region sequences of mouse antibody A01 were screened in protein databases by alignment and analysis for CDR-grafting. The complementarity determining regions (CDRs) of the mouse antibody were grafted between the framework regions of the screened human germline antibody sequence, and the amino acid residues in the CDRs and/or framework regions were further mutated to give more candidate variable region sequences.

Light chain VL: The humanized sequence design using the combination of human germline framework sequences IGKV1-39*01 and IGKJ1*01 constructed light chain variant hzA01L1 (SEQ ID NO: 15); the humanized sequence design using the combination of human germline framework sequences IGKV3-20*01 and IGKJ1*01 constructed light chain variant hzA01L2 (SEQ ID NO: 16); the humanized sequence design using the combination of human germline framework sequences IGKV2-30*02 and IGKJ2*02 constructed light chain variant hzA01L3 (SEQ ID NO: 17).

Heavy chain VH: The humanized sequence design using the combination of human germline framework sequences IGHV4-4*08 and IGHJ3*01 constructed heavy chain variants hzA01H1, hzA01H3, and hzA01H4 (see SEQ ID NO: 10, SEQ ID NO: 12, and SEQ ID NO: 13, respectively); the humanized sequence design using the combination of human germline framework sequences IGHV3-33*01 and IGHJ3*01 constructed heavy chain variant hzA01H2 (SEQ ID NO: 11).

### Construction of humanized antibodies

The DNA sequences of VH and VL of the humanized antibodies described above were linked to the DNA sequences of the heavy chain constant region of a human IgG4 (SEQ ID NO: 54) and a kappa light chain constant region (SEQ ID NO: 57), respectively, by chemical synthesis. The sequences were then cloned into a pcDNA3.1(+) vector to construct recombinant humanized antibodies. Table 4 shows the pairing of VH and VL sequences for the humanized antibodies.

**Table 4. Variable region sequences of humanized antibodies**

| Antibody | VL | VH |
|---|---|---|
| hzA01-1.1 | hzA01L1 | hzA01H1 |
| hzA01-1.2 | hzA01L1 | hzA01H2 |
| hzA01-2.1 | hzA01L2 | hzA01H1 |
| hzA01-2.2 | hzA01L2 | hzA01H2 |
| hzA01-3.1 | hzA01L3 | hzA01H1 |
| hzA01-3.3 | hzA01L3 | hzA01H3 |
| hzA01-3.4 | hzA01L3 | hzA01H4 |

### Expression and purification of humanized antibodies

The humanized antibodies were transiently transfected into cells using an expiCHO expression system (Gibco, Cat. No.: A29129) according to the instructions for its transfection kits. The transfected cells were cultured at 37 °C/8% CO₂ for 6 days while shaking. The cell culture supernatant was collected by centrifugation, and then co-incubated with protein A magnetic beads (GenScript, Cat. No.: L00273) at room temperature for 2 h while shaking. The beads were washed with PBS solution. The antibodies bound to the protein A magnetic beads were eluted with acetic acid buffer (300 mM acetic acid, pH 3.6), and the eluate was immediately neutralized with 1 M Tris-HCl. Subsequently, the humanized antibodies were transferred into the PBS buffer by ultrafiltration.

### Example 6. Characterization of humanized anti-CD40 antibodies

### Determination of affinity of anti-CD40 antibodies

The affinity of humanized anti-CD40 antibodies to an hCD40-His protein (Aero, Cat. No.: CD0-H5228) and a CynoCD40-His protein (Aero, Cat. No.: CD0-C52H6) was detected by a ForteBio molecular interaction system. Firstly, an AHC biosensor (Fortebio, Cat. No.: 18-5060) was activated, and then immersed in an anti-CD40 antibody solution at a final concentration of 5 µg/mL to capture the antibodies. Serially 2-fold diluted hCD40-His protein and CynoCD40-His protein in a final concentration range of 1.56 nM-100 nM were then immobilized on the AHC biosensor. The response signals were detected in real time on a Fortebio molecular interaction system (Fortebio Octet RED96e) to give association and dissociation curves. After the completion of dissociation in each cycle, the AHC biosensor was regenerated for the next capture; the procedure was repeated until the affinity of the different antibodies for CD40 was determined. The resulting data were analyzed by Fortebio Data Analysis 11.0 software using a 1:1 (Langmuir) binding model to determine the Kₐ (Kₒₙ) and K_{d} (K_{dis}) values, and the dissociation constant K_{D} was calculated by K_{D} = K_{d}/Kₐ. See www.fortebio.com for more detailed detection procedures and related information. The results are shown in Table 5. The binding affinity K_{D} values of humanized antibodies hzA01-3.1, hzA01-3.3, and hzA01-3.4 to human or cynomolgus monkey CD40 antigen were between 0.1 nM and 1 nM, comparable to the affinity of chimeric Chi-A01.

**Table 5. Detection of affinity of humanized anti-CD40 antibodies to CD40 by Fortebio**

| Antibody | hCD40-His protein | | | CynoCD40-His protein | | |
|---|---|---|---|---|---|---|
| | Kₒₙ (1/MS) | K_{dis} (1/s) | K_{D} (M) | Kₒₙ (1/Ms) | K_{dis} (1/s) | K_{D} (M) |
| BM | 4.74E+05 | 5.61E-05 | 1.18E-10 | 7.69E+05 | 4.30E-05 | 5.59E-11 |
| Chi-A01 | 1.85E+06 | 2.83E-04 | 1.53E-10 | 1.70E+06 | 2.76E-04 | 1.62E-10 |
| Chi-A03 | 5.86E+05 | 1.47E-03 | 2.51E-09 | ND | ND | ND |
| Chi-B01 | 3.21E+05 | 2.46E-03 | 7.66E-09 | ND | ND | ND |
| hzA01-1.1 | 1.25E+06 | 1.01E-03 | 8.08E-10 | 1.60E+06 | 9.23E-04 | 5.76E-10 |
| hzA01-1.2 | 8.08E+05 | 4.84E-02 | 5.99E-08 | ND | ND | ND |
| hzA01-2.1 | 1.45E+06 | 2.55E-03 | 1.76E-09 | 1.88E+06 | 2.82E-03 | 1.50E-09 |
| hzA01-2.2 | 8.93E+05 | 1.01E-01 | 1.13E-07 | ND | ND | ND |
| hzA01-3.1 | 1.64E+06 | 6.01E-04 | 3.66E-10 | 1.88E+06 | 6.86E-04 | 3.65E-10 |
| hzA01-3.3 | 1.63E+06 | 4.14E-04 | 2.55E-10 | 1.99E+06 | 4.84E-04 | 2.43E-10 |
| hzA01-3.4 | 1.72E+06 | 4.54E-04 | 2.64E-10 | 2.03E+06 | 5.32E-04 | 2.62E-10 |

| | | | | | | |
|---|---|---|---|---|---|---|
| Note: ND denotes not detected. | | | | | | |

### Detection of binding activity of anti-CD40 antibodies by FACS

The procedures are detailed in Example 2, except for some minor changes as follows: the CHO-K1-hCD40 cells were replaced with 293T-hCD40-NFκB cells; the hybridoma cell culture supernatant was replaced with serially 3-fold diluted anti-CD40 antibodies in a final concentration range of 0.006 ng/mL-1000 ng/mL; the PE-conjugated goat anti-mouse IgG (H+L) antibody was replaced with a PE-conjugated donkey anti-human IgG (H+L) antibody (Abeam, Cat. No.: ab102439); the flow cytometer (BD Accuri C6) was replaced with another flow cytometer (Sartorius IQue3).

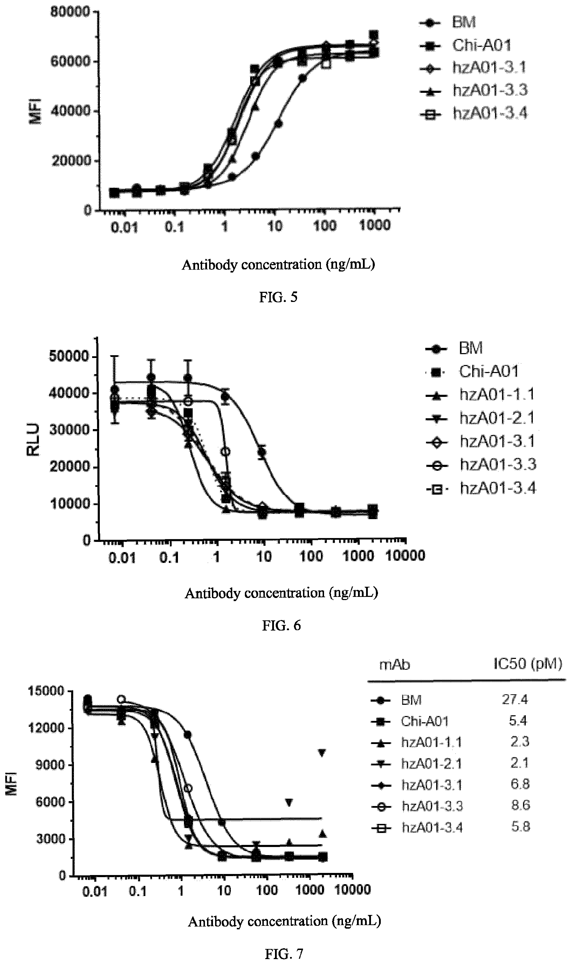

The results are shown in FIG. 5. In the concentration range of 10-100 ng/mL, the binding of the humanized antibodies hzA01-3.1, hzA01-3.3, and hzA01-3.4 to 293T-hCD40-NFκB cells was saturated, and the MFI value corresponding to the saturated concentration was the same as that of BM.

### Activity of anti-CD40 antibodies to block binding of CHO-K1-hCD40L cells to hCD40-mFc and CHO-K1-CynoCD40L cells to cvnoCD40-mFc

A CHO-K1-hCD40L cell suspension and a CHO-K1-CynoCD40L cell suspension at a cell concentration of 3×10⁵ cells/mL were separately added into a 96-well U-bottom plate at 100 µL/well. An hCD40-mFc protein at a final concentration of 2 µg/mL (prepared in-house, SEQ ID NO: 64) or a cynoCD40-mFc protein at a final concentration of 2 µg/mL (prepared in-house, SEQ ID NO: 65) was added correspondingly, before serially 2-fold diluted anti-CD40 antibodies in a final concentration range of 312.5 ng/mL-40000 ng/mL were added. The mixture was mixed well, and then incubated at 4 °C for 1 h. The cells were washed with PBS containing 2% of FBS, and then a PE-conjugated goat anti-mouse IgG (H+L) antibody (Abeam, Cat. No.: ab97041) diluted at a volume ratio of 1:500 was added, and the mixture was incubated at 4 °C for 0.5 h. The cells were washed with PBS containing 2% of PBS, and then resuspended in PBS containing 2% of FBS. The fluorescence signals were detected on a flow cytometer (Sartorius IQue3). The activity of anti-CD40 antibodies to block the binding of CHO-K1-hCD40L cells to an hCD40-mFc protein and CHO-K1-cynoCD40L cells to a cynoCD40-mFc protein was analyzed by the mean fluorescence intensity (MFI) of staining, and the IC₅₀ value was calculated by GraphPad Prism software. The results are shown in Table 6. The blocking IC₅₀ value of humanized antibodies hzA01-3.1 and hzA01-3.4 at the cellular level are comparable to that of BM.

**Table 6. Activity of humanized anti-CD40 antibodies to block binding of CHO-K1-hCD40L cells and CHO-K1-CynoCD40L cells to CD40**

| Antibody | Activity to block binding of CHO-K1-hCD40L cells to hCD40-mFc protein (IC₅₀, nM) | Activity to block binding of CHO-K1-cynoCD40L cells to cynoCD40-mFc protein (IC₅₀, nM) |
|---|---|---|
| BM | 20.55 | 24.14 |
| Chi-A01 | 21.27 | 25.22 |
| hzA01-3.1 | 22.83 | 25.51 |
| hzA01-3.3 | 38.42 | 35.77 |
| hzA01-3.4 | 19.81 | 22.87 |

### Activity of anti-CD40 antibodies to block binding of 293T-hCD40-NFκB cells to CHO-K1-hCD40L cells

A diluted 293T-hCD40-NFκB cell suspension at a cell concentration of 2.5×10⁵ cells/mL was added into a 96-well plate at 20 µL/well. Serially 6-fold diluted anti-CD40 antibodies in a final concentration range of 0.007 ng/mL-2000 ng/mL were added. Meanwhile, a CHO-K1-hCD40L cell suspension was added in a cell number ratio of 1:1. The mixture was well mixed, and then incubated at room temperature for 20 min. Subsequently, the 96-well plate was let stand for incubation at 37 °C/5% CO₂ for 5-6 h. After the incubation, a detection reagent was added according to the instructions for a Luciferase Assay System (Vazyme, Cat. No.: DD1201-03), and the cell fluorescence signals were detected on a microplate reader (Thermo Varioskan Flash). The blocking activity of anti-CD40 antibodies on CD40L-mediated up-regulation of fluorescence signals in 293T-hCD40-NFxB cells was analyzed by relative light unit (RLU). The results are shown in FIG. 6. In the concentration range of 0-2 µg/mL, humanized antibodies hzA01-1.1, hzA01-2.1, hzA01-3.1, hzA01-3.3, and hzA01-3.4 all exhibited significant blocking activity.

### Inhibitory activity of anti-CD40 antibodies against Ramos cell apoptosis

The procedures are detailed in Example 2, except for some minor changes as follows: the serially 10-fold diluted pured mouse anti-CD40 antibodies in a final concentration range of 0.02 ng/mL-2000 ng/mL were replaced with serially 6-fold diluted anti-CD40 antibodies in a final concentration range of 0.007 ng/mL-2000 ng/mL.

The results are shown in FIG. 7. In the concentration range of 0-2 µg/mL, humanized antibodies hzA01-3.1, hzA01-3.3, and hzA01-3.4 all exhibited persistent inhibitory activity with IC₅₀ values 3-4 times lower than that of BM.

### Agonistic activity of anti-CD40 antibodies on Ramos cell apoptosis

The procedures are detailed in Example 4, except for some minor changes as follows: the serially 10-fold diluted anti-CD40 antibodies in a final concentration range of 0.01 ng/mL-10000 ng/mL were replaced with serially 5-fold diluted anti-CD40 antibodies in a final concentration range of 0.01 nM-2000 nM.

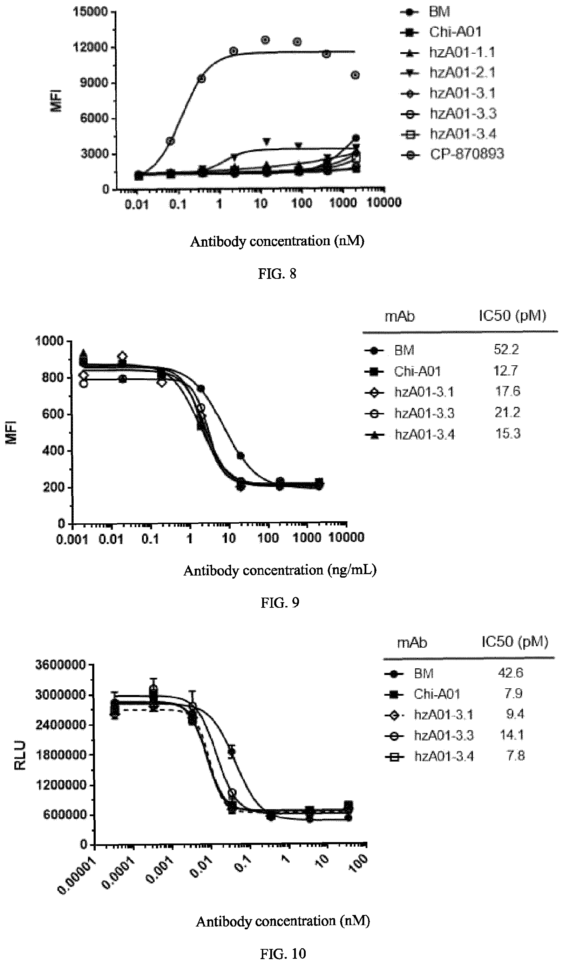

The results are shown in FIG. 8. Humanized antibodies hzA01-1.1 and hzA01-2.1 exhibited weak agonistic activity, and in the concentration range of 0.01 nM-2000 nM, humanized antibodies hzA01-3.1, hzA01-3.3, and hzA01-3.4 exhibited no significant agonistic activity compared to that of BM.

### Inhibitory activity of anti-CD40 antibodies against activation of peripheral blood B lymphocytes

CD40 expressed on human peripheral blood-derived B lymphocytes (PBMC-B) can bind to CD40L and induce the activation of B cells, thereby causing the up-regulation of cell surface activation signals (e.g., CD86). B lymphocytes from healthy human PBMCs were isolated using a human B lymphocyte purification kit (Stemcell, Cat. No.: 17954). A human peripheral blood B lymphocyte suspension at a cell concentration of 1×10⁶ cells/mL was added into a 96-well U-bottom plate at 50 µL/well, before serially 10-fold diluted anti-CD40 antibodies in a final concentration range of 0.002 ng/mL-2000 ng/mL were added. Subsequently, a CHO-K1-hCD40L cell suspension was added in a cell number ratio of 1:1. The mixture was incubated overnight at 37 °C/5% CO₂. The cells were washed with a PBS solution containing 2% of FBS, before a PE-conjugated mouse anti-human CD86 antibody (Biolegend, Cat. No.: 305438) was added. The mixture was mixed well, and incubated at 4 °C for 30 min. The cells were washed with PBS containing 2% of FBS and resuspended. The mean fluorescence intensity (MFI) of costimulatory molecule CD86 expressed on B cells was detected with a flow cytometer (Sartorius IQue3), and the IC₅₀ value was analyzed and calculated by Graphpad Prism. The results are shown in FIG. 9. Humanized antibodies hzA01-3.1, hzA01-3.3, and hzA01-3.4 all exhibited inhibitory activity on CD40L-induced CD86 expression on human peripheral blood B lymphocytes with an IC₅₀ value about 2-4 times lower than that of BM.

### Inhibitory activity of anti-CD40 antibodies on proliferation of peripheral blood B lymphocytes

CD40 expressed on human peripheral blood-derived B lymphocytes (PBMC-B) can bind to CD40L and induce the proliferation of B cells. A human peripheral blood B lymphocyte suspension at a cell concentration of 1×10⁶ cells/mL was added into a 96-well U-bottom plate at 50 µL/well, before serially 10-fold diluted anti-CD40 antibodies in a final concentration range of 0.000035 nM-35 nM were added. A human CD40L protein (Aero, Cat. No.: CDL-H52Db) at a final concentration of 2 µg/mL and a recombinant human IL-4 protein (Aero, Cat. No.: IL4-H4218) at a final concentration of 60 ng/mL were added. The mixture was mixed well, and then incubated at 37 °C/5% CO₂ for 5 days. The cells were collected, washed with a PBS buffer, and resuspended. A CellTiter Glo luciferase cell activity detection reagent (Promega, Cat. No.: G7572) was added, and the fluorescence signal of the cells was detected on a microplate reader (Thermo Varioskan Flash). The proliferation of human peripheral blood B lymphocytes was analyzed by relative light unit (RLU), and the IC₅₀ value was calculated by Graphpad Prism. The results are shown in FIG. 10. Humanized antibodies hzA01-3.1, hzA01-3.3, and hzA01-3.4 can inhibit the proliferation of human peripheral blood B lymphocytes induced by CD40L and IL-4 with IC₅₀ values about 3-6 times lower than that of BM.

### Activity of anti-CD40 antibodies to induce proliferation of peripheral blood B lymphocytes

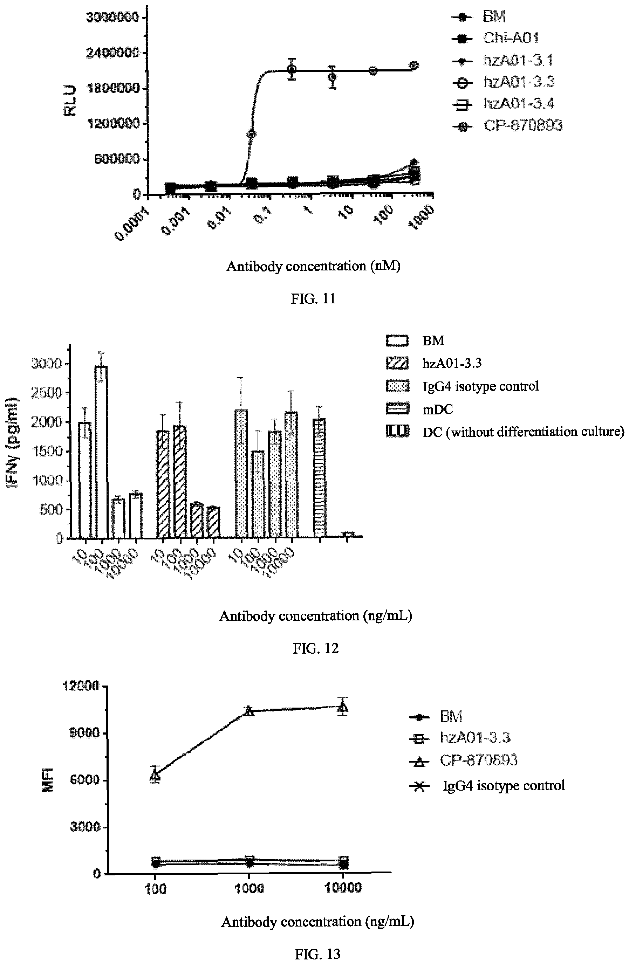

A healthy human peripheral blood B lymphocyte suspension with a cell concentration of 1 × 10⁶ cells/mL was added into a 96-well U-bottom plate at 50 µL/well, and serially 10-fold diluted anti-CD40 antibodies in a final concentration range of 0.00035 nM-350 nM was added. The mixture was then incubated at 37 °C/5% CO₂ for 5 days. The cells were collected, washed with a PBS buffer, and resuspended. A CellTiter Glo luciferase cell activity detection reagent (Promega, Cat. No.: G7572) was added, and the fluorescence signal of the cells was detected on a microplate reader (Thermo Varioskan Flash). The proliferation of peripheral blood B lymphocytes was analyzed by relative light unit (RLU). The results are shown in FIG. 11. Humanized antibody hzA01-3.1 can induce weak proliferation at high concentration (350 nM), while in the concentration range of 0-350 nM, humanized antibodies hzA01-3.3 and hzA01-3.4 exhibited no significant agonistic activity compared to that of BM.

### Inhibitory effect of anti-CD40 antibodies on mixed lymphocyte reaction (MLR)

Monocytes were isolated from healthy human PBMCs according to the instructions for a monocyte isolation kit (Stemcell, Cat. No.: 19359), and then the concentration of cells was adjusted to 1×10⁶ cells/mL. A DC cell differentiation inducer was added according to the instructions for a DC cell culture kit (Stemcell, Cat. No.: 10985), and the cells were incubated at 37 °C/5% CO₂ for 5 days. Then a DC cell maturation stimulator was added, and the cells were further cultured for 2 days to differentiate monocytes into fully mature DC cells (mDCs).

CD4⁺ T cells were isolated from healthy human PBMCs according to the instructions for EasySep^{™}CD4 positive T cell isolation kit (Stemcell, Cat. No.: 17952), and then the concentration of the cells was adjusted to 2×10⁶ cells/mL. mDC cells were added into a 96-well U-bottom plate at 1×10⁴ cells/well, and serially 10-fold diluted anti-CD40 antibodies or IgG4 isotype control antibody in a final concentration range of 10 ng/mL-10000 ng/mL was added. The mixture was mixed well and incubated at room temperature for 30 min. Subsequently, a CD4⁺ T cell suspension was added at an mDC:CD4⁺ T cell number ratio of 1:20. The mixture was mixed well and then cultured at 37 °C/5% CO₂ for 96 h. Subsequently, a culture supernatant was collected by centrifugation.

According to the instructions for an IFNγ detection kit (R&D, Cat. No.: DY285B), IFNγ in the culture supernatant was detected by ELISA, the OD450 absorbance value was read on a microplate reader (Bio-rad iMark), and the concentration of IFNγ was calculated and analyzed by Graphpad Prism. The results are shown in FIG. 12. Humanized antibody hzA01-3.3 exhibited an inhibitory effect on mixed lymphocyte reaction in a concentration-dependent manner within a concentration range of 10 ng/mL-10000 ng/mL, and the released amount of IFNγ at the saturated concentrations (1000 ng/mL-10000 ng/mL) was lower than that of BM, indicating that hzA01-3.3 has better inhibitory activity against mixed lymphocyte reaction than BM at the saturation concentrations.

### Agonistic activity of anti-CD40 antibodies on dendritic cell maturation

Monocytes were isolated from PBMCs according to the instructions for a monocyte isolation kit (Stemcell, Cat. No.: 19359), and cultured under induction with a DC cell differentiation inducer (Stemcell, Cat. No.: 10988) for 5 days. Immature DC cells (imDC) were collected, and the concentration of the cells was adjusted to 1×10⁶ cells/mL. The imDC cell suspension was added into a 96-well plate at 50 µL/well, before serially 10-fold diluted anti-CD40 antibodies or IgG4 isotype control antibody in a final concentration range of 100 ng/mL-10000 ng/mL was added. The mixture was incubated overnight at 37 °C/5% CO₂. The cells were collected, washed with PBS containing 2% of FBS and resuspended. An APC-conjugated mouse anti-human CD83 antibody (BD, Cat. No.: 551073) was added. The mixture was mixed well and incubated at 4 °C for 30 min. The cells were then washed with PBS containing 2% FBS and resuspended. The fluorescence signal of the cells was detected on a flow cytometer (Sartorius IQue3). The expression of CD83 on the cell surface was analyzed by the mean fluorescence intensity (MFI) of staining. The expression of CD83 is a specific marker of mature DC cells. The results are shown in FIG. 13. CP-870893 exhibited a significant effect on stimulating the maturation of imDC cells, while neither humanized antibody hzA01-3.3 nor BM exhibited significant agonistic activity on the maturation of imDC cells.

### Example 7. Fc effect of anti-CD40 antibodies

Cells, e.g., human B cells, DC cells, and PBMCs, express Fc receptors (FcRs) on the cell surface. When the Fc-terminus of an anti-CD40 antibody binds to an FcR, it may mediate antibody Fc crosslinking to exert the agonistic activity, or mediate antibody-dependent cell-mediated cytotoxicity (ADCC) or complement-dependent cytotoxicity (CDC) to cause B cell depletion.

A Ramos cell suspension at a cell concentration of 1×10⁶ cells/mL was added into a 96-well U-bottom plate at 50 µL/well, before serially 10-fold diluted anti-CD40 antibodies in a final concentration range of 1 µg/mL-10 µg/mL were added. The mixture was mixed well and incubated at room temperature for 20 min. Subsequently, an anti-human IgG Fcy antibody (Jackson Immuno, Cat. No.: 109-005-190) at a final concentration of 2 µg/mL was added. The mixture was mixed well and incubated overnight at 37 °C/5% CO₂. The cells were collected by centrifugation, washed with PBS containing 2% of FBS, and resuspended. The fluorescence signals of the cells were detected on a flow cytometer (Sartorius IQue3). The expression of apoptosis molecule CD95 on Ramos cells was analyzed by the mean fluorescence intensity (MFI) of staining. The results are shown in Table 7. In the concentration range of 1 µg/mL-10 µg/mL, humanized antibody hzA01-3.3 exhibited no significantly higher cross-linking stimulatory activity than that of BM, and the cross-linking agonistic activity was much lower than that of CP-870893.

**Table 7. Determination of Fc crosslinking activity of anti-CD40 antibodies**

| Antibody | MFI value | |
|---|---|---|
| | 1 µg/mL antibody | 10 µg/mL antibody |
| BM | 12499 | 12077 |
| hzA01-3.3 | 14089 | 12289 |
| CP-870893 | 136374 | 135373 |

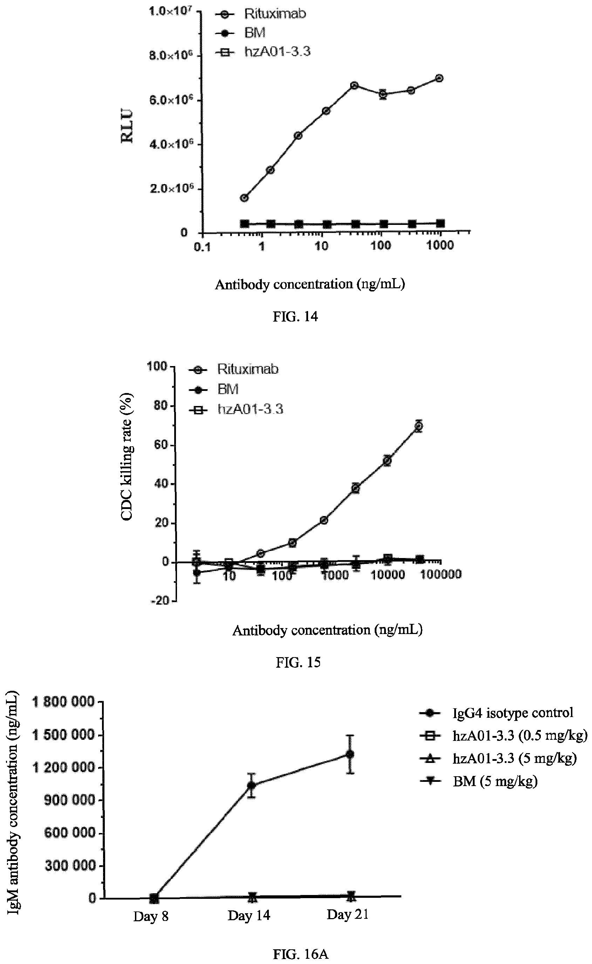

The ADCC effect mediated by anti-CD40 antibodies was evaluated using Ramos cell as the target cell and Jurkat-NFAT-Luc cell (Promega) as the effector cell. Specifically, a Ramos cell suspension at a cell concentration of 1×10⁶ cells/mL was added into a 96-well plate at 50 µL/well, before serially 3-fold diluted anti-CD40 antibodies or rituximab in a final concentration range of 0.46 ng/mL-1000 µg/mL was added. The mixture was mixed well and incubated at room temperature for 20 min. Subsequently, a Jurkat-NFAT-Luc cell suspension at a cell concentration of 5×10⁶ cells/mL was added to the 96-well plate at 100 µL/well, wherein the number ratio of the target cells to the effector cells was 1:10. The mixture was mixed well and incubated at 37 °C for 5 h. The cells were collected by centrifugation and washed with PBS. A Luc fluorescence detection reagent (RHINO BIO, Cat. No.: RA-GL03) was added, and the fluorescence signal of the cells was detected on a microplate reader (Thermo Varioskan Flash). The ADCC effect mediated by anti-CD40 antibodies was analyzed by relative light unit (RLU) value. The results are shown in FIG. 14. Rituximab exhibited a significant ADCC effect, while neither humanized antibody hzA01-3.3 nor BM exhibited ADCC activity.

A Ramos cell suspension at a cell concentration of 1×10⁶ cells/mL was added into a 96-well plate at 50 µL/well, before serially 4-fold diluted anti-CD40 antibodies or rituximab in a final concentration range of 2.4 ng/mL-40000 ng/mL was added at 50 µL/well. Meanwhile, a human serum complement protein (Quidel, Cat. No.: A112) was added at 25 µL/well. The mixture was mixed well and incubated overnight at 37 °C/5% CO₂. Detection reagents were added according to the instructions for CellTiter Glo luciferase cell activity detection kit (Promega, Cat. No.: G7572), and the fluorescence signal of the cells was detected on a microplate reader (Thermo Varioskan Flash). The viable cells were analyzed by relative light unit (RLU). The results show that rituximab exhibited significant CDC killing activity, while neither humanized antibody hzA01-3.3 nor BM exhibited CDC killing effect (FIG. 15). The CDC killing rate % = (fluorescence value of target cells - fluorescence value of antibody group) ÷ (fluorescence value of target cells - fluorescence value of target cells treated with detection reagent) × 100%.

### Example 8. In vivo drug effect of humanized anti-CD40 antibodies

Healthy human PBMCs were transplanted into NDG immunodeficient mice for immune reconstitution to construct a mouse model producing human antibodies. On one hand, human PBMCs produce human anti-mouse IgG antibodies and human anti-mouse IgM antibodies in response to mouse antigens, and on the other hand, after stimulation with exogenous proteins such as KLH, human PBMCs in mice produce anti-KLH specific antibodies. The construction method of the mouse model producing humanized antibodies was as follows: female NDG immunodeficient mice aged 6-8 weeks (purchased from Biocytogen) were selected. On day 0, each mouse was injected with 1×10⁷ healthy human PBMCs via the tail vein. On days 0-5, each mouse was injected with 10 µg of recombinant human IL-4 (Aero, Cat. No.: IL4-H4218) intraperitoneally daily. On days 0 and 7, each mouse was injected with 50 µg of KLH protein (Sigma, Cat. No.: H7017-20MG) intraperitoneally. Mice were grouped according to Table 8. On day 0, each mouse was intraperitoneally injected with an antibody drug once weekly for 3 consecutive doses.

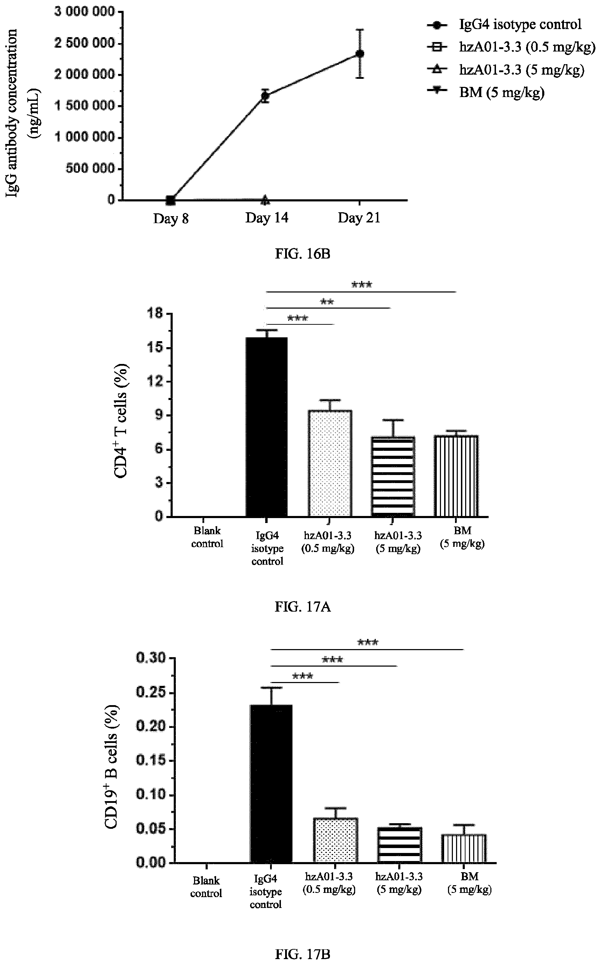

On days 8, 14, and 21, blood samples were collected from the mice and separated to give serum samples. The concentrations of human anti-mouse IgG antibodies and human anti-mouse IgM antibodies in the mouse serum were determined according to the instructions for a human IgG ELISA kit (Thermo, Cat. No.: BMS2091) and a human IgM ELISA kit (Novus, Cat. No.: NBP2-60477), respectively. The OD450 absorbance value was read on a microplate reader (Bio-rad iMark), and the concentrations of human anti-mouse IgG antibodies and human anti-mouse IgM antibodies in the serum were calculated and analyzed by Graphpad Prism. In addition, the titer of anti-KLH antibodies in the mouse serum was detected by the following method: a KLH protein (Sigma, Cat. No.: H7017-20MG) at a concentration of 2 µg/mL was added into a 96-well plate at 100 µL/well and incubated overnight at 4 °C. The 96-well plate was washed with PBST, and blocked at room temperature for 2 h by adding a blocking buffer at 200 µL/well (PBST containing 1% of BSA). The blocking buffer was discarded. Serially diluted mouse serum was added at 100 µL/well, and the mixture was incubated at room temperature for 2 h. The 96-well plate was washed with PBST, and an HRP-conjugated goat anti-human IgG (H+L) antibody (Jackson Immuno, Cat. No.: 109-035-088) diluted in a volume ratio of 1:10000 was added. Subsequently, the mixture was incubated at room temperature for 1 h. The 96-well plate was washed with PBST, and then a TMB solution was added at 100 µL/well. Subsequently, the mixture was incubated at room temperature in the dark for 5 min, and the reaction was stopped by adding 0.5 M H₂SO₄. The 450 nM absorbance was read on a Bio-rad iMark microplate reader. The results show that the humanized antibody hzA01-3.3 significantly inhibited the production of human anti-mouse IgG antibodies and human anti-mouse IgM antibodies compared to the IgG4 isotype group (FIGs. 16A and 16B); the hzA01-3.3 treatment group did not produce specific antibodies to KLH (data not shown).

On day 22, the mice were sacrificed and the spleen was collected, weighed, and analyzed for spleen coefficients in each group of mice (spleen coefficient = spleen weight/mouse body weight × 1000). The results are shown in Table 8. The spleens of mice in the IgG4 isotype control group were significantly enlarged and the spleen coefficients were significantly increased. The splenomegaly in mice of hzA01-3.3 (0.5 mg/kg) and hzA01-3.3 (5 mg/kg) groups was significantly relieved, and hzA01-3.3 relieved the spleen coefficients in a dose-dependent manner. The spleen coefficients of hzA01-3.3 (5 mg/kg) and BM (5 mg/kg) groups were comparable.

**Table 8. Determination of spleen coefficients in NDG mice**

| Mice group (n) | Average spleen weight (g) | Average body weight (g) | Spleen coefficient |
|---|---|---|---|
| Blank control, n = 3 | 0.04±0.01 | 23.33±2.71 | 1.81±0.40 |
| IgG4 isotype control, n = 4 | 0.24±0.03 | 22.50±1.27 | 10.82±1.14 |
| hzA01-3.3 (0.5 mg/kg), n=5 | 0.15±0.03 | 23.14±1.65 | 6.31±1.06 |
| hzA01-3.3 (5 mg/kg), n=5 | 0.13±0.04 | 22.56±2.16 | 5.50±1.42 |
| BM (5 mg/kg), n=5 | 0.13±0.03 | 24.04±0.38 | 5.58±1.09 |

| | | | |
|---|---|---|---|
| Note: Mice in the blank control were NDG mice without immune reconstitution and treatment; the IgG4 Isotype control was Anti-HEL-Human IgG4 (S228P L235E) Isotype-control (Biointron, Cat. No.: B109805). | | | |

In addition, the human T lymphocyte and B lymphocyte infiltrations in the spleen of mice in all groups were measured by flow cytometry. Specifically, the spleen was taken to prepare a single-cell suspension, and the cell concentration was adjusted to 1×10⁶ cells/mL. The suspension was added into a 96-well U-bottom plate at 100 µL/well, and a PE-conjugated mouse anti-human CD4 antibody (Biolegend, Cat. No.: 980804) and an APC-conjugated mouse anti-human CD19 antibody (BD, Cat. No.: 555415) were separately added. The mixture was mixed well and incubated at 4 °C for 30 min. The cells were washed with PBS containing 2% FBS and resuspended, and the fluorescence signal of the cells was detected on a flow cytometer (Thermo Attune Nxt). The human CD4⁺ T cell and CD19⁺ B cell infiltrations in the spleen of mice were measured by the percentage (%) of stained cells. The results show (FIG. 17) that on day 22, the spleens of mice in the IgG4 isotype control group were infiltrated with a large number of human CD4⁺ T cells and human CD19⁺ B cells, while the T cell and B cell infiltrations in spleen in hzA01-3.3 (0.5 mg/kg) and hzA01-3.3 (5 mg/kg) groups were significantly lower than those of the IgG4 isotype group, and hzA01-3.3 reduced the human T cell and B cell infiltrations in a dose-dependent manner.

### Example 9. Pharmacodynamic assessment of anti-CD40 antibodies in submandibular gland protein-induced hCD40/hCD40L transgenic mouse Sjogren's syndrome model

The antigen was prepared by the following procedures: The submandibular glands were collected from 20 C57BL/6 mice (6-8 weeks old, female; Biocytogen Pharmaceuticals Co., Ltd.). The envelope and connective tissue of the submandibular glands were separated and washed with PBS (Servicebio, Cat. No.: G4202-500ML) at 4 °C. The submandibular glands were mechanically homogenized at a low temperature and centrifuged. Submandibular gland proteins were obtained from the supernatant and used as the antigen.

The hCD40/hCD40L mouse Sjogren's syndrome model was constructed by the following procedures: on days 0 and 7, the antigen was mixed with a complete Freund's adjuvant (Sigma, Cat. No.: F5881) in a volume ratio of 1:1 to prepare a submandibular gland protein emulsion, which was injected subcutaneously at two points in the back of hCD40/hCD40L mice (Beijing Vital River Laboratory Animal Technology Co., Ltd.) in a total amount of 200 µL; on day 14, the antigen was mixed with an incomplete Freund's adjuvant (Sigma, Cat. No.: F5506) in a volume ratio of 1:1 to prepare a submandibular gland protein emulsion, which was injected subcutaneously at the two points in the back of hCD40/hCD40L mice in a total amount of 200 µL.

The hCD40/hCD40L mice were divided into 5 groups by the mean value of initial saliva volume, and were administered twice weekly for a total of 10 doses (on days 0, 4, 7, 11, 14, 18, 21, 25, 28, and 32, respectively) starting on day 0. The study was ended on day 35. During this study, the body weight was measured every 2 weeks. The grouping and administration regimens are shown in Table 9.

**Table 9. Grouping and administration regimens**

| Group | Treatment | Dosage (mg/kg) | Administration concentration (mg/mL) | Route and frequency of administration | Number of animals |
|---|---|---|---|---|---|
| Blank control group | Normal saline | N/A | N/A | i.v., BIW | 5 |
| Model group | Normal saline | N/A | N/A | i.v., BIW | 10 |
| hzA01-3.3 (1 mg/kg) | hzA01-3.3 | 1 | 0.1 | i.v., BIW | 10 |
| hzA01-3.3 (5 mg/kg) | hzA01-3.3 | 5 | 0.5 | i.v., BIW | 10 |
| hzA01-3.3 (25 mg/kg) | hzA01-3.3 | 25 | 2.5 | i.v., BIW | 10 |

| | | | | | |
|---|---|---|---|---|---|
| Note: Mice in the blank control group were hCD40/hCD40L mice without antigen immunization; N/A means not applicable; i.v. means intravenous injection; BIW means twice weekly. | | | | | |

The pharmacodynamic assessment of hzA01-3.3 was performed by saliva flow rate measurement, submandibular gland index calculation, IL-6 detection in submandibular glands, flow cytometry assays for blood and spleen, and submandibular gland pathological HE staining examination. The specific procedures were as follows:
On days 3, 6, 13, 19, 27, and 34, mice were anesthetized with 1% pentobarbital sodium solution at a dose of 60 mg/kg and injected intraperitoneally with pilocarpine (Aladdin, Cat. No.: P129614) at 1.25 mg/kg. Saliva was collected within 15 minutes after the injection and saliva flow rate was calculated according to the formula saliva flow rate = [cotton ball wet weight (mg) - cotton ball dry weight (mg)]/15 min. The saliva flow rate data was subjected to a T-test using GraphPad Prism.
On day 35, 100 µL of blood was collected from each mouse. A PE-labeled anti-mouse CD45 antibody (Biolegend, Cat. No.: 103106), Brilliant Violet 510^{™}-labeled anti-mouse CD3 antibody (Biolegend, Cat. No.: 100234), an FITC-labeled anti-mouse CD4 antibody (Biolegend, Cat. No.: 100510), a PE/Cyanine7-labeled anti-mouse CD8a antibody (Biolegend, Cat. No.: 100722), and an APC-labeled anti-mouse CD19 antibody (Biolegend, Cat. No.: 115512) were added into the blood sample, and the mixture was incubated at 4 °C for 30 min in the dark. A red blood cell lysis buffer (Biolegend, Cat. No.: 420301) was added, and the mixture was incubated at room temperature in the dark for 30 min. Subsequently, the cells were washed with PBS and resuspended, and the fluorescence signal was detected on a flow cytometer.
On day 35, the spleen was collected from the mice and prepared into a single-cell suspension, and the cell density was adjusted to 1 × 10⁸ cells/mL. 100 µL of the suspension was taken, a red blood cell lysis buffer (Biolegend, Cat. No.: 420301) was added, and the mixture was let stand at room temperature in the dark for 30 min. After PBS washing, a PE-labeled anti-mouse CD45 antibody (Biolegend, Cat. No.: 103106), Brilliant Violet 510^{™}-labeled anti-mouse CD3 antibody (Biolegend, Cat. No.: 100234), an FITC-labeled anti-mouse CD4 antibody (Biolegend, Cat. No.: 100510), a PE/Cyanine7-labeled anti-mouse CD8a antibody (Biolegend, Cat. No.: 100722), and an APC-labeled anti-mouse CD 19 antibody (Biolegend, Cat. No.: 115512) were added into the blood sample, and the mixture was incubated at 4 °C for 30 min in the dark. Subsequently, the cells were washed with PBS and resuspended, and the fluorescence signal was detected on a flow cytometer.
On day 35, the submandibular glands were collected from the mice and weighed. The submandibular gland index was calculated according to the formula: submandibular gland index = weight of submandibular glands (mg)/body weight of mice (g). One submandibular gland was taken and fixed in 10% neutral formalin, and pathological HE staining and the submandibular gland pathological scoring were performed. The submandibular gland pathological scores were subjected to a T-test using GraphPad Prism. The other submandibular gland was mechanically homogenized and centrifuged at a low temperature to give a supernatant. The content of IL-6 in the supernatant was detected according to the instructions for a mouse IL-6 ELISA kit (Abeam, Cat. No.: ab222503).

The results of the body weight changes (FIG. 18) show that the weight gain of animals in all groups was comparable to that of the blank control group, indicating that hzA01-3.3 had no significant adverse effects at all doses.

The results of the saliva flow rates (FIG. 19) show that the saliva flow rates of hzA01-3.3 (5 mg/kg) and hzA01-3.3 (25 mg/kg) groups exhibited a rebounding trend; compared to the model group, the saliva flow rate of hzA01-3.3 (25 mg/kg) group was significantly higher on day 27 and day 34 (P < 0.05), indicating that hzA01-3.3 could improve the saliva flow rate of mice.

The results of the submandibular gland index, the content of IL-6 in submandibular glands, and the submandibular gland pathological scoring (FIGs. 20A-20C) show that all indices of mice in hzA01-3.3 (1 mg/kg), hzA01-3.3 (5 mg/kg), and hzA01-3.3 (25 mg/kg) groups exhibited a decreasing trend; compared to the model group, all indices of mice in hzA01-3.3 (5 mg/kg) and hzA01-3.3 (25 mg/kg) groups were significantly decreased, indicating that hzA01-3.3 could improve the submandibular gland index, inhibit the secretion of IL-6 in submandibular glands, and reduce pathological scores of submandibular glands.

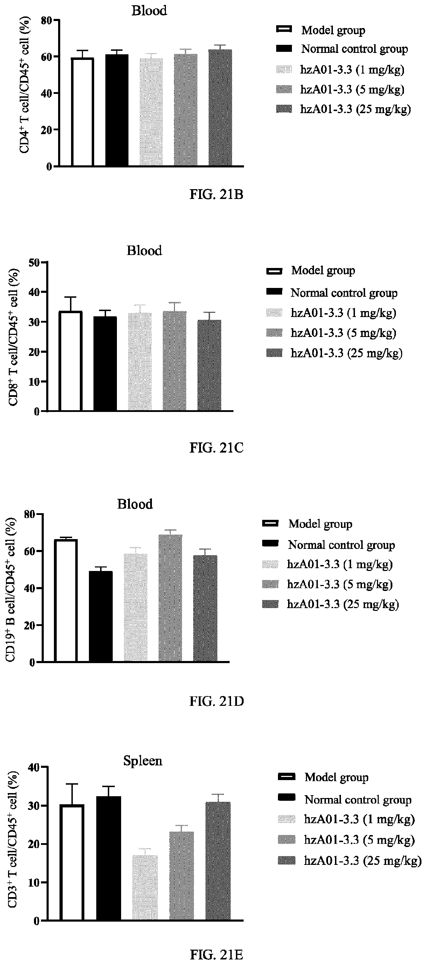

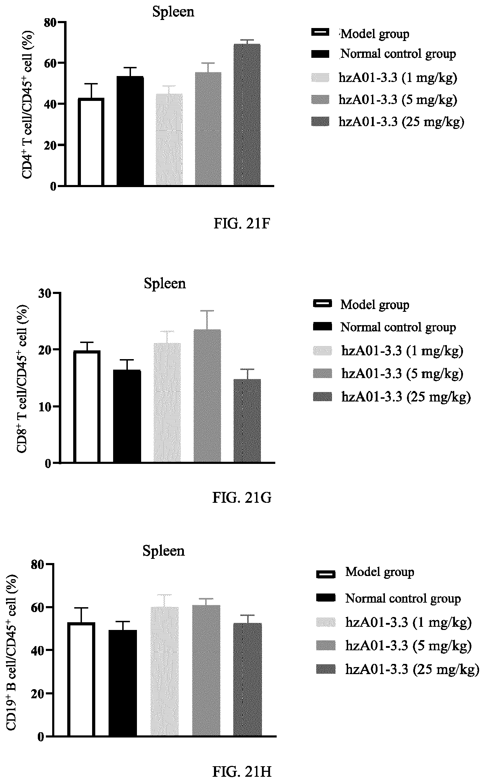

The results of blood and spleen flow cytometry (FIGs. 21A-21H) showed that the percentages of CD3⁺ T cells, CD4⁺ T cells, CD8⁺ T cells, and CD 19⁺ B cells in the blood and spleen of animals in the hzA01-3.3 treatment groups exhibited no significant changes compared to those in the blank control group, indicating that hzA01-3.3 has no specific effect on the changes in the number of the cells described above. It is expected that hzA01-3.3 may have good safety or/and good efficacy in clinical use.

The sequence information of the present disclosure is summarized in Table 10.

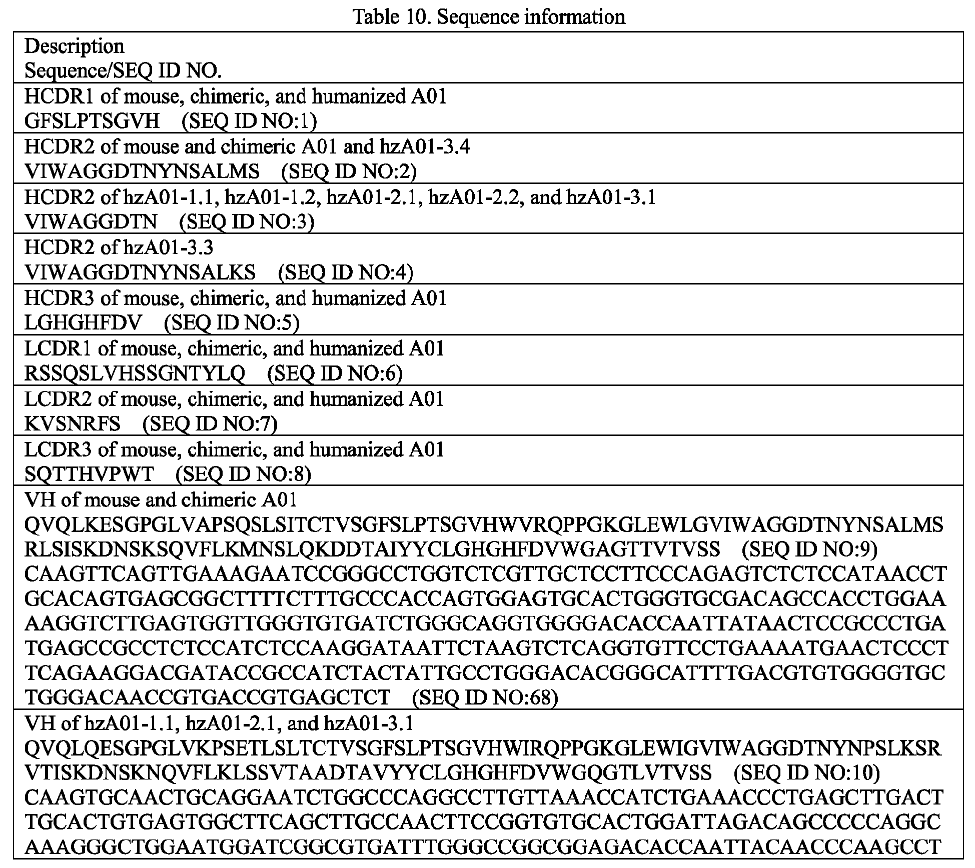

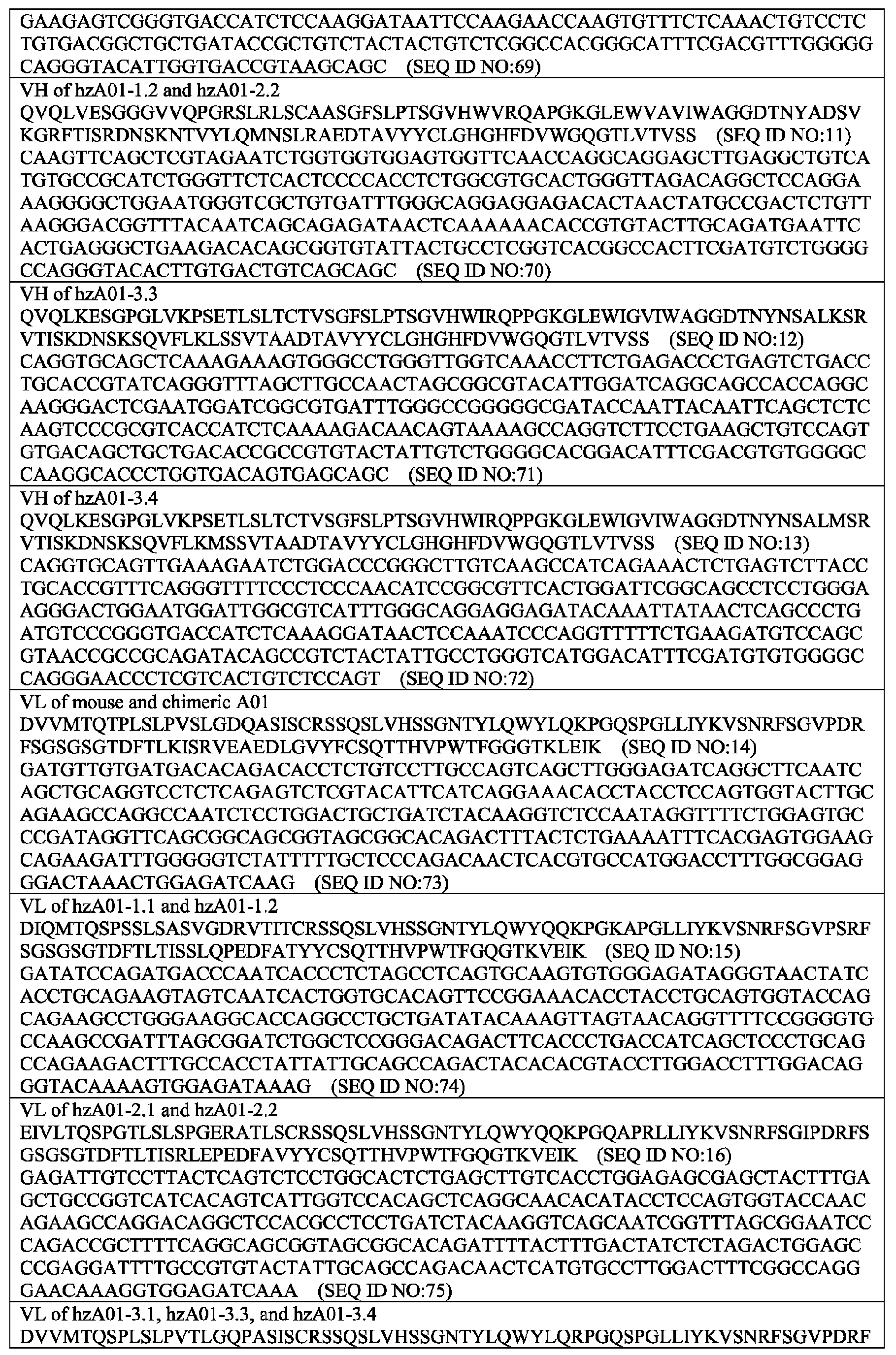

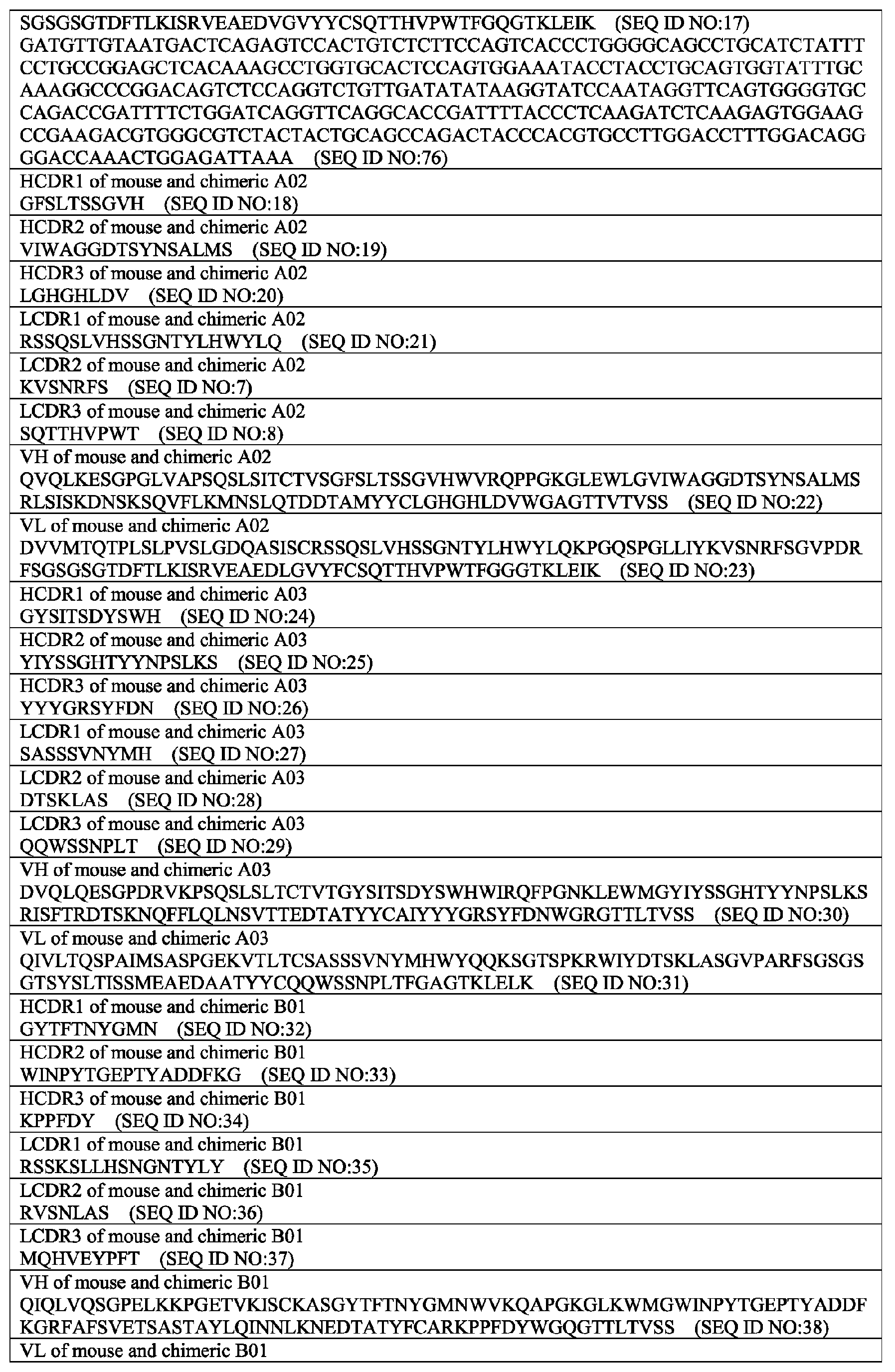

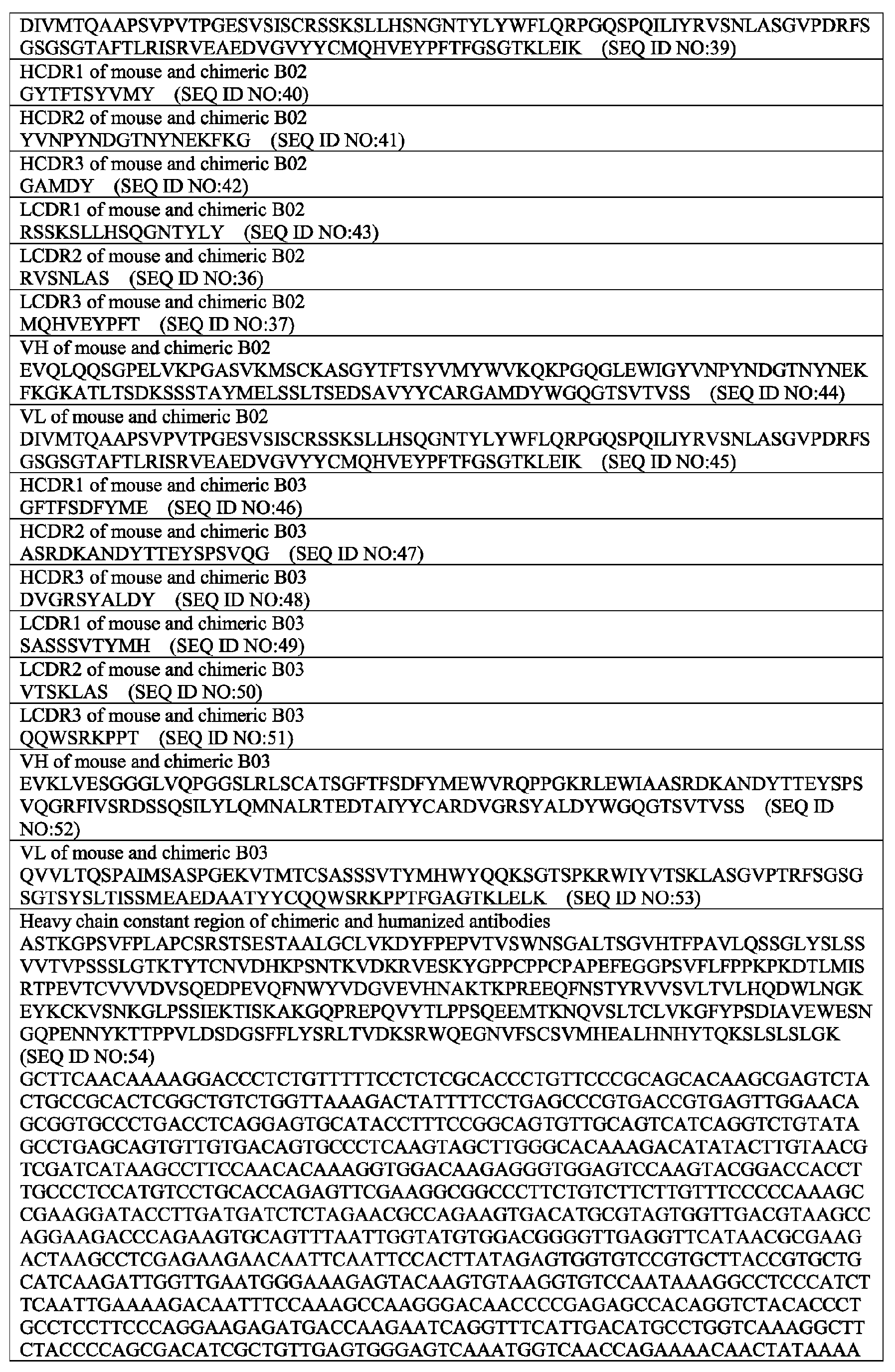

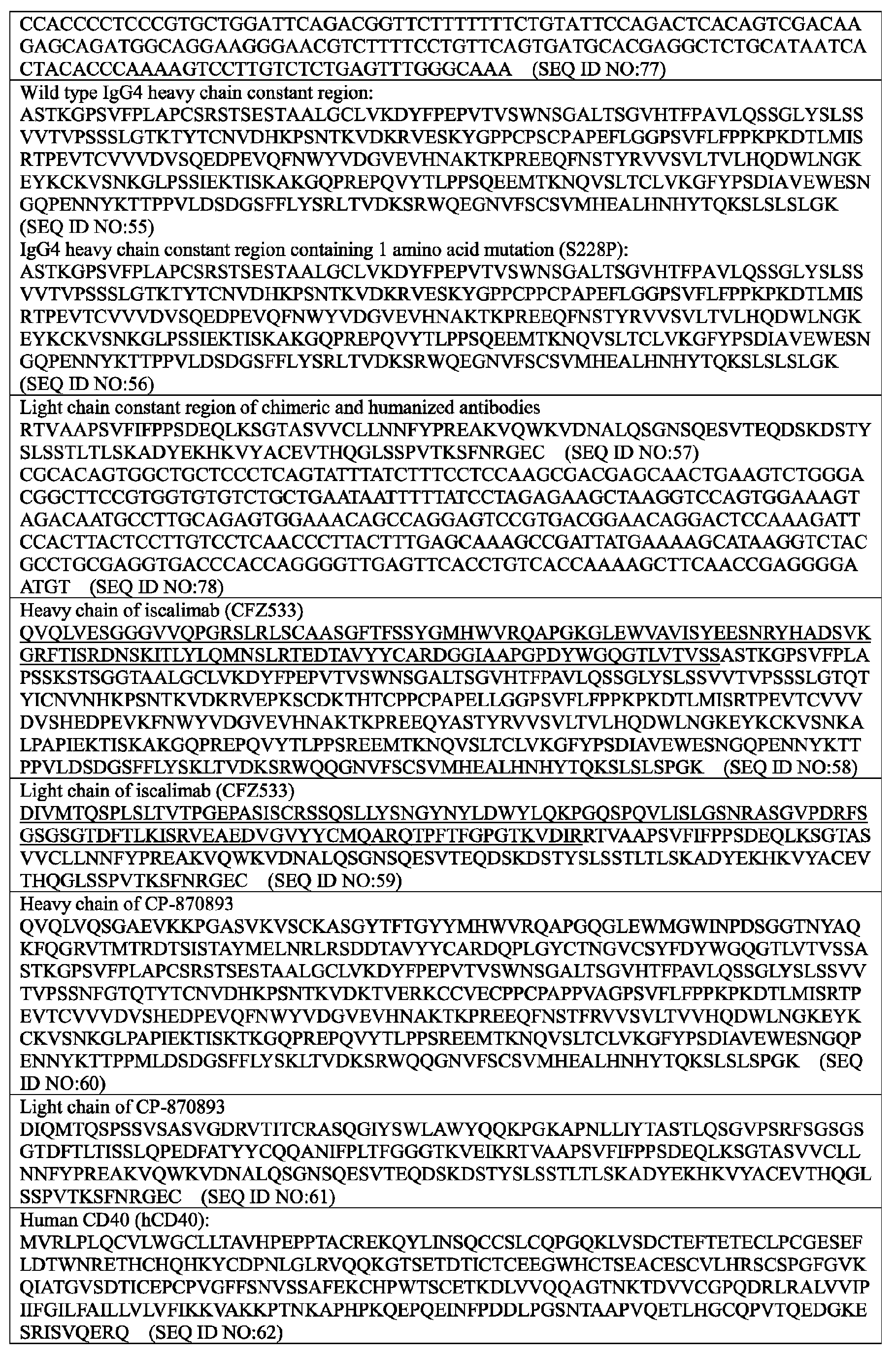

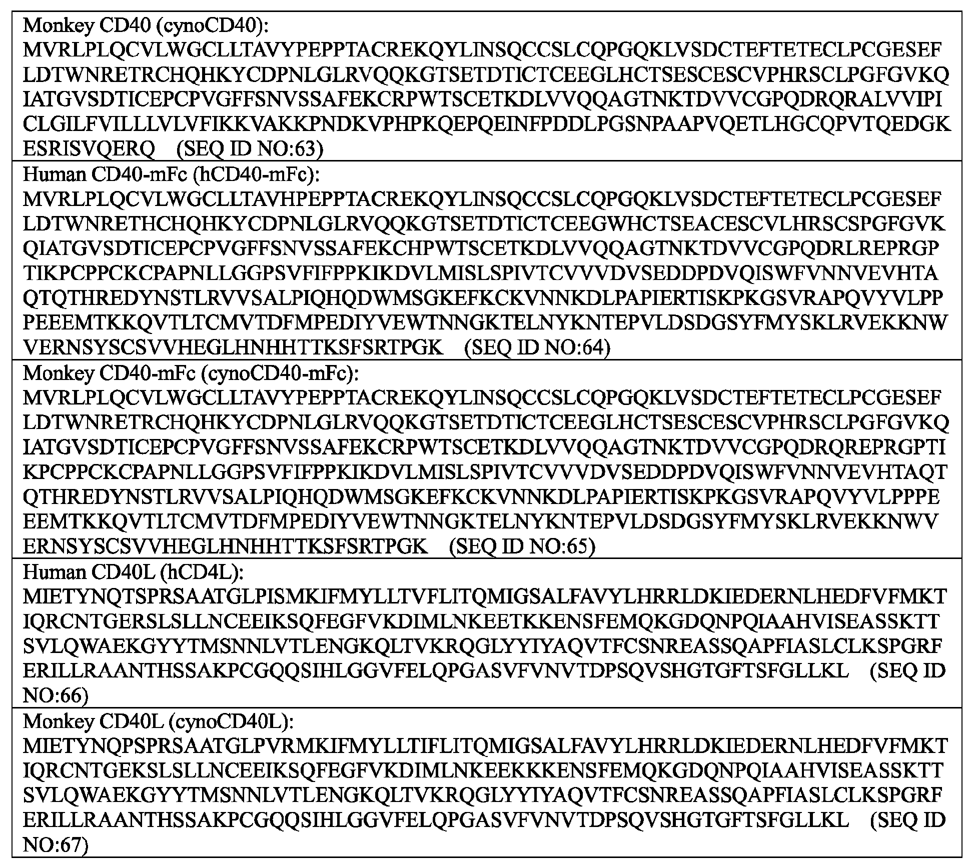

## Claims

1. An isolated anti-CD40 antibody or an antigen-binding fragment thereof, comprising:
(i) a heavy chain CDR1, a heavy chain CDR2, and a heavy chain CDR3, wherein
(1) the heavy chain CDR1 comprises the heavy chain CDR1 amino acid sequence in SEQ ID NO: 9, the heavy chain CDR2 comprises the heavy chain CDR2 amino acid sequence in SEQ ID NO: 9, and the heavy chain CDR3 comprises the heavy chain CDR3 amino acid sequence in SEQ ID NO: 9;
(2) the heavy chain CDR1 comprises the heavy chain CDR1 amino acid sequence in SEQ ID NO: 10, the heavy chain CDR2 comprises the heavy chain CDR2 amino acid sequence in SEQ ID NO: 10, and the heavy chain CDR3 comprises the heavy chain CDR3 amino acid sequence in SEQ ID NO: 10;
(3) the heavy chain CDR1 comprises the heavy chain CDR1 amino acid sequence in SEQ ID NO: 11, the heavy chain CDR2 comprises the heavy chain CDR2 amino acid sequence in SEQ ID NO: 11, and the heavy chain CDR3 comprises the heavy chain CDR3 amino acid sequence in SEQ ID NO: 11;
(4) the heavy chain CDR1 comprises the heavy chain CDR1 amino acid sequence in SEQ ID NO: 12, the heavy chain CDR2 comprises the heavy chain CDR2 amino acid sequence in SEQ ID NO: 12, and the heavy chain CDR3 comprises the heavy chain CDR3 amino acid sequence in SEQ ID NO: 12;
(5) the heavy chain CDR1 comprises the heavy chain CDR1 amino acid sequence in SEQ ID NO: 13, the heavy chain CDR2 comprises the heavy chain CDR2 amino acid sequence in SEQ ID NO: 13, and the heavy chain CDR3 comprises the heavy chain CDR3 amino acid sequence in SEQ ID NO: 13;
(6) the heavy chain CDR1 comprises the heavy chain CDR1 amino acid sequence in SEQ ID NO: 22, the heavy chain CDR2 comprises the heavy chain CDR2 amino acid sequence in SEQ ID NO: 22, and the heavy chain CDR3 comprises the heavy chain CDR3 amino acid sequence in SEQ ID NO: 22;
(7) the heavy chain CDR1 comprises the heavy chain CDR1 amino acid sequence in SEQ ID NO: 30, the heavy chain CDR2 comprises the heavy chain CDR2 amino acid sequence in SEQ ID NO: 30, and the heavy chain CDR3 comprises the heavy chain CDR3 amino acid sequence in SEQ ID NO: 30;
(8) the heavy chain CDR1 comprises the heavy chain CDR1 amino acid sequence in SEQ ID NO: 38, the heavy chain CDR2 comprises the heavy chain CDR2 amino acid sequence in SEQ ID NO: 38, and the heavy chain CDR3 comprises the heavy chain CDR3 amino acid sequence in SEQ ID NO: 38;
(9) the heavy chain CDR1 comprises the heavy chain CDR1 amino acid sequence in SEQ ID NO: 44, the heavy chain CDR2 comprises the heavy chain CDR2 amino acid sequence in SEQ ID NO: 44, and the heavy chain CDR3 comprises the heavy chain CDR3 amino acid sequence in SEQ ID NO: 44; or
(10) the heavy chain CDR1 comprises the heavy chain CDR1 amino acid sequence in SEQ ID NO: 52, the heavy chain CDR2 comprises the heavy chain CDR2 amino acid sequence in SEQ ID NO: 52, and the heavy chain CDR3 comprises the heavy chain CDR3 amino acid sequence in SEQ ID NO: 52; and/or
(ii) a light chain CDR1, a light chain CDR2, and a light chain CDR3, wherein
(1) the light chain CDR1 comprises the light chain CDR1 amino acid sequence in SEQ ID NO: 14, the light chain CDR2 comprises the light chain CDR2 amino acid sequence in SEQ ID NO: 14, and the light chain CDR3 comprises the light chain CDR3 amino acid sequence in SEQ ID NO: 14;
(2) the light chain CDR1 comprises the light chain CDR1 amino acid sequence in SEQ ID NO: 15, the light chain CDR2 comprises the light chain CDR2 amino acid sequence in SEQ ID NO: 15, and the light chain CDR3 comprises the light chain CDR3 amino acid sequence in SEQ ID NO: 15;
(3) the light chain CDR1 comprises the light chain CDR1 amino acid sequence in SEQ ID NO: 16, the light chain CDR2 comprises the light chain CDR2 amino acid sequence in SEQ ID NO: 16, and the light chain CDR3 comprises the light chain CDR3 amino acid sequence in SEQ ID NO: 16;
(4) the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17;
(5) the light chain CDR1 comprises the light chain CDR1 amino acid sequence in SEQ ID NO: 23, the light chain CDR2 comprises the light chain CDR2 amino acid sequence in SEQ ID NO: 23, and the light chain CDR3 comprises the light chain CDR3 amino acid sequence in SEQ ID NO: 23;
(6) the light chain CDR1 comprises the light chain CDR1 amino acid sequence in SEQ ID NO: 31, the light chain CDR2 comprises the light chain CDR2 amino acid sequence in SEQ ID NO: 31, and the light chain CDR3 comprises the light chain CDR3 amino acid sequence in SEQ ID NO: 31;
(7) the light chain CDR1 comprises the light chain CDR1 amino acid sequence in SEQ ID NO: 39, the light chain CDR2 comprises the light chain CDR2 amino acid sequence in SEQ ID NO: 39, and the light chain CDR3 comprises the light chain CDR3 amino acid sequence in SEQ ID NO: 39;
(8) the light chain CDR1 comprises the light chain CDR1 amino acid sequence in SEQ ID NO: 45, the light chain CDR2 comprises the light chain CDR2 amino acid sequence in SEQ ID NO: 45, and the light chain CDR3 comprises the light chain CDR3 amino acid sequence in SEQ ID NO: 45; or
(9) the light chain CDR1 comprises the light chain CDR1 amino acid sequence in SEQ ID NO: 53, the light chain CDR2 comprises the light chain CDR2 amino acid sequence in SEQ ID NO: 53, and the light chain CDR3 comprises the light chain CDR3 amino acid sequence in SEQ ID NO: 53.

2. The antibody or the antigen-binding fragment thereof according to claim 1, comprising:
(i) the heavy chain CDR1, the heavy chain CDR2, and the heavy chain CDR3, wherein
(1) the heavy chain CDR1, the heavy chain CDR2, and the heavy chain CDR3 comprise the amino acid sequences set forth in SEQ ID NOs: 1, 2, and 5, or amino acid sequences having at least 80% identity to the amino acid sequences set forth in SEQ ID NOs: 1, 2, and 5, respectively;
(2) the heavy chain CDR1, the heavy chain CDR2, and the heavy chain CDR3 comprise the amino acid sequences set forth in SEQ ID NOs: 1, 3, and 5, or amino acid sequences having at least 80% identity to the amino acid sequences set forth in SEQ ID NOs: 1, 3, and 5, respectively;
(3) the heavy chain CDR1, the heavy chain CDR2, and the heavy chain CDR3 comprise the amino acid sequences set forth in SEQ ID NOs: 1, 4, and 5, or amino acid sequences having at least 80% identity to the amino acid sequences set forth in SEQ ID NOs: 1, 4, and 5, respectively;
(4) the heavy chain CDR1, the heavy chain CDR2, and the heavy chain CDR3 comprise the amino acid sequences set forth in SEQ ID NOs: 18, 19, and 20, or amino acid sequences having at least 80% identity to the amino acid sequences set forth in SEQ ID NOs: 18, 19, and 20, respectively;
(5) the heavy chain CDR1, the heavy chain CDR2, and the heavy chain CDR3 comprise the amino acid sequences set forth in SEQ ID NOs: 24, 25, and 26, or amino acid sequences having at least 80% identity to the amino acid sequences set forth in SEQ ID NOs: 24, 25, and 26, respectively;
(6) the heavy chain CDR1, the heavy chain CDR2, and the heavy chain CDR3 comprise the amino acid sequences set forth in SEQ ID NOs: 32, 33, and 34, or amino acid sequences having at least 80% identity to the amino acid sequences set forth in SEQ ID NOs: 32, 33, and 34, respectively;
(7) the heavy chain CDR1, the heavy chain CDR2, and the heavy chain CDR3 comprise the amino acid sequences set forth in SEQ ID NOs: 40, 41, and 42, or amino acid sequences having at least 80% identity to the amino acid sequences set forth in SEQ ID NOs: 40, 41, and 42, respectively; or
(8) the heavy chain CDR1, the heavy chain CDR2, and the heavy chain CDR3 comprise the amino acid sequences set forth in SEQ ID NOs: 46, 47, and 48, or amino acid sequences having at least 80% identity to the amino acid sequences set forth in SEQ ID NOs: 46, 47, and 48, respectively; and/or
(ii) the light chain CDR1, the light chain CDR2, and the light chain CDR3, wherein
(1) the light chain CDR1, the light chain CDR2, and the light chain CDR3 comprise the amino acid sequences set forth in SEQ ID NOs: 6, 7, and 8, or amino acid sequences having at least 80% identity to the amino acid sequences set forth in SEQ ID NOs: 6, 7, and 8, respectively;
(2) the light chain CDR1, the light chain CDR2, and the light chain CDR3 comprise the amino acid sequences set forth in SEQ ID NOs: 21, 7, and 8, or amino acid sequences having at least 80% identity to the amino acid sequences set forth in SEQ ID NOs: 21, 7, and 8, respectively;
(3) the light chain CDR1, the light chain CDR2, and the light chain CDR3 comprise the amino acid sequences set forth in SEQ ID NOs: 27, 28, and 29, or amino acid sequences having at least 80% identity to the amino acid sequences set forth in SEQ ID NOs: 27, 28, and 29, respectively;
(4) the light chain CDR1, the light chain CDR2, and the light chain CDR3 comprise the amino acid sequences set forth in SEQ ID NOs: 35, 36, and 37, or amino acid sequences having at least 80% identity to the amino acid sequences set forth in SEQ ID NOs: 35, 36, and 37, respectively;
(5) the light chain CDR1, the light chain CDR2, and the light chain CDR3 comprise the amino acid sequences set forth in SEQ ID NOs: 43, 36, and 37, or amino acid sequences having at least 80% identity to the amino acid sequences set forth in SEQ ID NOs: 43, 36, and 37, respectively; or
(6) the light chain CDR1, the light chain CDR2, and the light chain CDR3 comprise the amino acid sequences set forth in SEQ ID NOs: 49, 50, and 51, or amino acid sequences having at least 80% identity to the amino acid sequences set forth in SEQ ID NOs: 49, 50, and 51, respectively.

3. The antibody or the antigen-binding fragment thereof according to claim 1, comprising:
the heavy chain CDR1, the heavy chain CDR2, the heavy chain CDR3, the light chain CDR1, the light chain CDR2, and the light chain CDR3, wherein,
(1) the heavy chain CDR1 comprises the heavy chain CDR1 amino acid sequence in SEQ ID NO: 9, the heavy chain CDR2 comprises the heavy chain CDR2 amino acid sequence in SEQ ID NO: 9, the heavy chain CDR3 comprises the heavy chain CDR3 amino acid sequence in SEQ ID NO: 9, the light chain CDR1 comprises the light chain CDR1 amino acid sequence in SEQ ID NO: 14, the light chain CDR2 comprises the light chain CDR2 amino acid sequence in SEQ ID NO: 14, and the light chain CDR3 comprises the light chain CDR3 amino acid sequence in SEQ ID NO: 14;
(2) the heavy chain CDR1 comprises the heavy chain CDR1 amino acid sequence in SEQ ID NO: 10, the heavy chain CDR2 comprises the heavy chain CDR2 amino acid sequence in SEQ ID NO: 10, the heavy chain CDR3 comprises the heavy chain CDR3 amino acid sequence in SEQ ID NO: 10, the light chain CDR1 comprises the light chain CDR1 amino acid sequence in SEQ ID NO: 15, the light chain CDR2 comprises the light chain CDR2 amino acid sequence in SEQ ID NO: 15, and the light chain CDR3 comprises the light chain CDR3 amino acid sequence in SEQ ID NO: 15;
(3) the heavy chain CDR1 comprises the heavy chain CDR1 amino acid sequence in SEQ ID NO: 11, the heavy chain CDR2 comprises the heavy chain CDR2 amino acid sequence in SEQ ID NO: 11, the heavy chain CDR3 comprises the heavy chain CDR3 amino acid sequence in SEQ ID NO: 11, the light chain CDR1 comprises the light chain CDR1 amino acid sequence in SEQ ID NO: 15, the light chain CDR2 comprises the light chain CDR2 amino acid sequence in SEQ ID NO: 15, and the light chain CDR3 comprises the light chain CDR3 amino acid sequence in SEQ ID NO: 15;
(4) the heavy chain CDR1 comprises the heavy chain CDR1 amino acid sequence in SEQ ID NO: 10, the heavy chain CDR2 comprises the heavy chain CDR2 amino acid sequence in SEQ ID NO: 10, the heavy chain CDR3 comprises the heavy chain CDR3 amino acid sequence in SEQ ID NO: 10, the light chain CDR1 comprises the light chain CDR1 amino acid sequence in SEQ ID NO: 16, the light chain CDR2 comprises the light chain CDR2 amino acid sequence in SEQ ID NO: 16, and the light chain CDR3 comprises the light chain CDR3 amino acid sequence in SEQ ID NO: 16;
(5) the heavy chain CDR1 comprises the heavy chain CDR1 amino acid sequence in SEQ ID NO: 11, the heavy chain CDR2 comprises the heavy chain CDR2 amino acid sequence in SEQ ID NO: 11, the heavy chain CDR3 comprises the heavy chain CDR3 amino acid sequence in SEQ ID NO: 11, the light chain CDR1 comprises the light chain CDR1 amino acid sequence in SEQ ID NO: 16, the light chain CDR2 comprises the light chain CDR2 amino acid sequence in SEQ ID NO: 16, and the light chain CDR3 comprises the light chain CDR3 amino acid sequence in SEQ ID NO: 16;
(6) the heavy chain CDR1 comprises the heavy chain CDR1 amino acid sequence in SEQ ID NO: 10, the heavy chain CDR2 comprises the heavy chain CDR2 amino acid sequence in SEQ ID NO: 10, the heavy chain CDR3 comprises the heavy chain CDR3 amino acid sequence in SEQ ID NO: 10, the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17;
(7) the heavy chain CDR1 comprises the heavy chain CDR1 amino acid sequence in SEQ ID NO: 12, the heavy chain CDR2 comprises the heavy chain CDR2 amino acid sequence in SEQ ID NO: 12, the heavy chain CDR3 comprises the heavy chain CDR3 amino acid sequence in SEQ ID NO: 12, the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17;
(8) the heavy chain CDR1 comprises the heavy chain CDR1 amino acid sequence in SEQ ID NO: 13, the heavy chain CDR2 comprises the heavy chain CDR2 amino acid sequence in SEQ ID NO: 13, the heavy chain CDR3 comprises the heavy chain CDR3 amino acid sequence in SEQ ID NO: 13, the light chain CDR1 comprises the light chain CDR1 amino acid sequence in SEQ ID NO: 17, the light chain CDR2 comprises the light chain CDR2 amino acid sequence in SEQ ID NO: 17, and the light chain CDR3 comprises the light chain CDR3 amino acid sequence in SEQ ID NO: 17;
(9) the heavy chain CDR1 comprises the heavy chain CDR1 amino acid sequence in SEQ ID NO: 22, the heavy chain CDR2 comprises the heavy chain CDR2 amino acid sequence in SEQ ID NO: 22, the heavy chain CDR3 comprises the heavy chain CDR3 amino acid sequence in SEQ ID NO: 22, the light chain CDR1 comprises the light chain CDR1 amino acid sequence in SEQ ID NO: 23, the light chain CDR2 comprises the light chain CDR2 amino acid sequence in SEQ ID NO: 23, and the light chain CDR3 comprises the light chain CDR3 amino acid sequence in SEQ ID NO: 23;
(10) the heavy chain CDR1 comprises the heavy chain CDR1 amino acid sequence in SEQ ID NO: 30, the heavy chain CDR2 comprises the heavy chain CDR2 amino acid sequence in SEQ ID NO: 30, the heavy chain CDR3 comprises the heavy chain CDR3 amino acid sequence in SEQ ID NO: 30, the light chain CDR1 comprises the light chain CDR1 amino acid sequence in SEQ ID NO: 31, the light chain CDR2 comprises the light chain CDR2 amino acid sequence in SEQ ID NO: 31, and the light chain CDR3 comprises the light chain CDR3 amino acid sequence in SEQ ID NO: 31;
(11) the heavy chain CDR1 comprises the heavy chain CDR1 amino acid sequence in SEQ ID NO: 38, the heavy chain CDR2 comprises the heavy chain CDR2 amino acid sequence in SEQ ID NO: 38, the heavy chain CDR3 comprises the heavy chain CDR3 amino acid sequence in SEQ ID NO: 38, the light chain CDR1 comprises the light chain CDR1 amino acid sequence in SEQ ID NO: 39, the light chain CDR2 comprises the light chain CDR2 amino acid sequence in SEQ ID NO: 39, and the light chain CDR3 comprises the light chain CDR3 amino acid sequence in SEQ ID NO: 39;
(12) the heavy chain CDR1 comprises the heavy chain CDR1 amino acid sequence in SEQ ID NO: 44, the heavy chain CDR2 comprises the heavy chain CDR2 amino acid sequence in SEQ ID NO: 44, the heavy chain CDR3 comprises the heavy chain CDR3 amino acid sequence in SEQ ID NO: 44, the light chain CDR1 comprises the light chain CDR1 amino acid sequence in SEQ ID NO: 45, the light chain CDR2 comprises the light chain CDR2 amino acid sequence in SEQ ID NO: 45, and the light chain CDR3 comprises the light chain CDR3 amino acid sequence in SEQ ID NO: 45; or
(13) the heavy chain CDR1 comprises the heavy chain CDR1 amino acid sequence in SEQ ID NO: 52, the heavy chain CDR2 comprises the heavy chain CDR2 amino acid sequence in SEQ ID NO: 52, the heavy chain CDR3 comprises the heavy chain CDR3 amino acid sequence in SEQ ID NO: 52, the light chain CDR1 comprises the light chain CDR1 amino acid sequence in SEQ ID NO: 53, the light chain CDR2 comprises the light chain CDR2 amino acid sequence in SEQ ID NO: 53, and the light chain CDR3 comprises the light chain CDR3 amino acid sequence in SEQ ID NO: 53.

4. The antibody or the antigen-binding fragment thereof according to any one of claims 1-3, comprising:
the heavy chain CDR1, the heavy chain CDR2, the heavy chain CDR3, the light chain CDR1, the light chain CDR2, and the light chain CDR3, wherein,
(1) the heavy chain CDR1, the heavy chain CDR2, the heavy chain CDR3, the light chain CDR1, the light chain CDR2, and the light chain CDR3 comprise the amino acid sequences set forth in SEQ ID NOs: 1, 2, 5, 6, 7, and 8, or amino acid sequences having at least 80% identity to the amino acid sequences set forth in SEQ ID NOs: 1, 2, 5, 6, 7, and 8, respectively;
(2) the heavy chain CDR1, the heavy chain CDR2, the heavy chain CDR3, the light chain CDR1, the light chain CDR2, and the light chain CDR3 comprise the amino acid sequences set forth in SEQ ID NOs: 1, 3, 5, 6, 7, and 8, or amino acid sequences having at least 80% identity to the amino acid sequences set forth in SEQ ID NOs: 1, 3, 5, 6, 7, and 8, respectively;
(3) the heavy chain CDR1, the heavy chain CDR2, the heavy chain CDR3, the light chain CDR1, the light chain CDR2, and the light chain CDR3 comprise the amino acid sequences set forth in SEQ ID NOs: 1, 4, 5, 6, 7, and 8, or amino acid sequences having at least 80% identity to the amino acid sequences set forth in SEQ ID NOs: 1, 4, 5, 6, 7, and 8, respectively;
(4) the heavy chain CDR1, the heavy chain CDR2, the heavy chain CDR3, the light chain CDR1, the light chain CDR2, and the light chain CDR3 comprise the amino acid sequences set forth in SEQ ID NOs: 18, 19, 20, 21, 7, and 8, or amino acid sequences having at least 80% identity to the amino acid sequences set forth in SEQ ID NOs: 18, 19, 20, 21, 7, and 8, respectively;
(5) the heavy chain CDR1, the heavy chain CDR2, the heavy chain CDR3, the light chain CDR1, the light chain CDR2, and the light chain CDR3 comprise the amino acid sequences set forth in SEQ ID NOs: 24, 25, 26, 27, 28, and 29, or amino acid sequences having at least 80% identity to the amino acid sequences set forth in SEQ ID NOs: 24, 25, 26, 27, 28, and 29, respectively;
(6) the heavy chain CDR1, the heavy chain CDR2, the heavy chain CDR3, the light chain CDR1, the light chain CDR2, and the light chain CDR3 comprise the amino acid sequences set forth in SEQ ID NOs: 32, 33, 34, 35, 36, and 37, or amino acid sequences having at least 80% identity to the amino acid sequences set forth in SEQ ID NOs: 32, 33, 34, 35, 36, and 37, respectively;
(7) the heavy chain CDR1, the heavy chain CDR2, the heavy chain CDR3, the light chain CDR1, the light chain CDR2, and the light chain CDR3 comprise the amino acid sequences set forth in SEQ ID NOs: 40, 41, 42, 43, 36, and 37, or amino acid sequences having at least 80% identity to the amino acid sequences set forth in SEQ ID NOs: 40, 41, 42, 43, 36, and 37, respectively; or
(8) the heavy chain CDR1, the heavy chain CDR2, the heavy chain CDR3, the light chain CDR1, the light chain CDR2, and the light chain CDR3 comprise the amino acid sequences set forth in SEQ ID NOs: 46, 47, 48, 49, 50, and 51, or amino acid sequences having at least 80% identity to the amino acid sequences set forth in SEQ ID NOs: 46, 47, 48, 49, 50, and 51, respectively.

5. The antibody or the antigen-binding fragment thereof according to any one of claims 1-4, comprising:
(i) a heavy chain variable region comprising the amino acid sequence set forth in SEQ ID NO: 9, 10, 11, 12, 13, 22, 30, 38, 44, or 52, or an amino acid sequence having at least 80% identity to the amino acid sequence set forth in SEQ ID NO: 9, 10, 11, 12, 13, 22, 30, 38, 44, or 52; and/or
(ii) a light chain variable region comprising the amino acid sequence set forth in SEQ ID NO: 14, 15, 16, 17, 23, 31, 39, 45, or 53, or an amino acid sequence having at least 80% identity to the amino acid sequence set forth in SEQ ID NO: 14, 15, 16, 17, 23, 31, 39, 45, or 53.

6. The antibody or the antigen-binding fragment thereof according to any one of claims 1-5, comprising the heavy chain variable region and the light chain variable region, wherein,
(1) the heavy chain variable region and the light chain variable region comprise the amino acid sequences set forth in SEQ ID NOs: 9 and 14, or amino acid sequences having at least 80% identity to the amino acid sequences set forth in SEQ ID NOs: 9 and 14, respectively;
(2) the heavy chain variable region and the light chain variable region comprise the amino acid sequences set forth in SEQ ID NOs: 10 and 15, or amino acid sequences having at least 80% identity to the amino acid sequences set forth in SEQ ID NOs: 10 and 15, respectively;
(3) the heavy chain variable region and the light chain variable region comprise the amino acid sequences set forth in SEQ ID NOs: 11 and 15, or amino acid sequences having at least 80% identity to the amino acid sequences set forth in SEQ ID NOs: 11 and 15, respectively;
(4) the heavy chain variable region and the light chain variable region comprise the amino acid sequences set forth in SEQ ID NOs: 10 and 16, or amino acid sequences having at least 80% identity to the amino acid sequences set forth in SEQ ID NOs: 10 and 16, respectively;
(5) the heavy chain variable region and the light chain variable region comprise the amino acid sequences set forth in SEQ ID NOs: 11 and 16, or amino acid sequences having at least 80% identity to the amino acid sequences set forth in SEQ ID NOs: 11 and 16, respectively;
(6) the heavy chain variable region and the light chain variable region comprise the amino acid sequences set forth in SEQ ID NOs: 10 and 17, or amino acid sequences having at least 80% identity to the amino acid sequences set forth in SEQ ID NOs: 10 and 17, respectively;
(7) the heavy chain variable region and the light chain variable region comprise the amino acid sequences set forth in SEQ ID NOs: 12 and 17, or amino acid sequences having at least 80% identity to the amino acid sequences set forth in SEQ ID NOs: 12 and 17, respectively;
(8) the heavy chain variable region and the light chain variable region comprise the amino acid sequences set forth in SEQ ID NOs: 13 and 17, or amino acid sequences having at least 80% identity to the amino acid sequences set forth in SEQ ID NOs: 13 and 17, respectively;
(9) the heavy chain variable region and the light chain variable region comprise the amino acid sequences set forth in SEQ ID NOs: 22 and 23, or amino acid sequences having at least 80% identity to the amino acid sequences set forth in SEQ ID NOs: 22 and 23, respectively;
(10) the heavy chain variable region and the light chain variable region comprise the amino acid sequences set forth in SEQ ID NOs: 30 and 31, or amino acid sequences having at least 80% identity to the amino acid sequences set forth in SEQ ID NOs: 30 and 31, respectively;
(11) the heavy chain variable region and the light chain variable region comprise the amino acid sequences set forth in SEQ ID NOs: 38 and 39, or amino acid sequences having at least 80% identity to the amino acid sequences set forth in SEQ ID NOs: 38 and 39, respectively;
(12) the heavy chain variable region and the light chain variable region comprise the amino acid sequences set forth in SEQ ID NOs: 44 and 45, or amino acid sequences having at least 80% identity to the amino acid sequences set forth in SEQ ID NOs: 44 and 45, respectively; or
(13) the heavy chain variable region and the light chain variable region comprise the amino acid sequences set forth in SEQ ID NOs: 52 and 53, or amino acid sequences having at least 80% identity to the amino acid sequences set forth in SEQ ID NOs: 52 and 53, respectively.

7. The antibody or the antigen-binding fragment thereof according to any one of claims 1-6, further comprising a heavy chain constant region and a light chain constant region, wherein the heavy chain constant region comprises the amino acid sequence set forth in SEQ ID NO: 54, 55, or 56, or an amino acid sequence comprising 1, 2, 3, 4, or 5 amino acid substitutions, deletions, and additions compared to the amino acid sequence set forth in SEQ ID NO: 54, 55, or 56, and the light chain constant region comprises the amino acid sequence set forth in SEQ ID NO: 57, or an amino acid sequence comprising 1, 2, 3, 4, or 5 amino acid substitutions, deletions, and additions compared to the amino acid sequence set forth in SEQ ID NO: 57.

8. The antibody or the antigen-binding fragment thereof according to any one of claims 1-7, wherein the antibody or the antigen-binding fragment thereof (a) binds to human CD40; (b) binds to monkey CD40; (c) blocks the interaction between CD40 and CD40L; and/or (d) inhibits CD40 activity.

9. The antibody or the antigen-binding fragment thereof according to any one of claims 1-8, wherein the antibody or the antigen-binding fragment thereof is chimeric or humanized;
optionally, the antibody or the antigen-binding fragment thereof is of the IgG1, IgG2, or IgG4 isotype;
optionally, the antibody or the antigen-binding fragment thereof is selected from the group consisting of a monoclonal antibody, a monospecific antibody, a bispecific antibody, a trispecific antibody, a multispecific antibody, an Fab fragment, an F(ab')2 fragment, an Fd fragment, an Fv fragment, a dAb fragment, an isolated CDR region, a single-chain Fv molecule, or a combination thereof.

10. An isolated nucleic acid molecule encoding the antibody or the antigen-binding fragment thereof according to any one of claims 1-9.

11. An expression vector, comprising the nucleic acid molecule according to claim 10.

12. A host cell, comprising the nucleic acid molecule according to claim 10 or the expression vector according to claim 11.

13. A recombinant polypeptide or fusion protein, comprising the antibody or the antigen-binding fragment thereof according to any one of claims 1-9.

14. A pharmaceutical composition, comprising the antibody or the antigen-binding fragment thereof according to any one of claims 1-9, and one or more pharmaceutically acceptable carriers.

15. A method for treating or preventing an immune disease in a subject in need, comprising: administering to the subject a therapeutically effective amount of the antibody or the antigen-binding fragment thereof according to any one of claims 1-9, or the pharmaceutical composition according to claim 14;
optionally, the immune disease includes, but is not limited to, an inflammatory disease, an allergic reaction, an autoimmune disease, or a transplantation-related disease;
optionally, the immune disease includes, but is not limited to, an allergic reaction, Addison's disease, ankylosing spondylitis, spondyloarthritis, asthma, atherosclerosis, coronary heart disease, autoimmune hepatitis, autoimmune parotitis, type I diabetes, epididymitis, nephritis, Reiter's syndrome, thyroiditis, Graves' disease, Guillain-Barre syndrome, Hashimoto's disease, hemolytic anemia, idiopathic thrombocytopenia, systemic lupus erythematosus, subacute cutaneous lupus erythematosus, multiple sclerosis, myasthenia gravis, psoriasis, scleroderma, arthritis, sarcoidosis, Sjogren's syndrome, xerophthalmia, hidradenitis suppurativa, transplantation-related disease, vasculitis, and/or inflammatory bowel disease;
optionally, the method further comprises administering a therapeutically effective amount of a second therapeutic agent;
optionally, the second therapeutic agent includes a non-steroidal anti-inflammatory drug, a salicylate, hydroxychloroquine, sulfasalazine, a corticosteroid, a cytotoxic drug, or an immunosuppressive drug and/or antibody.
